(19) Europäisches Patentamt
European Patent Office
Office européen des brevets

(11) EP 2 514 436 B1

(12) **EUROPEAN PATENT SPECIFICATION**

(45) Date of publication and mention of the grant of the patent:
**20.12.2017 Bulletin 2017/51**

(51) Int Cl.:
***A61K 38/20*** *(2006.01)* ***A61K 38/17*** *(2006.01)*
***A61K 38/16*** *(2006.01)* ***A61P 31/00*** *(2006.01)*

(21) Application number: **12169572.0**

(22) Date of filing: **07.11.2008**

(54) **IL-22 FOR USE IN TREATING MICROBIAL DISORDERS**

IL-22 ZUR VERWENDUNG IN DER BEHANDLUNG VON MIKROBIELLEN ERKRANKUNGEN

IL-22 POUR L'UTILISATION DANS LE TRAITEMENT DE TROUBLES MICROBIENS

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **07.11.2007 US 986170 P**
**13.12.2007 US 13620 P**
**20.12.2007 US 15620 P**

(43) Date of publication of application:
**24.10.2012 Bulletin 2012/43**

(62) Document number(s) of the earlier application(s) in accordance with Art. 76 EPC:
**08848478.7 / 2 217 263**

(73) Proprietor: **Genentech, Inc.**
**South San Francisco, CA 94080 (US)**

(72) Inventors:
- **ABBAS, Alexander, R.**
  **San Carlos, CA California 94070 (US)**
- **DANILENKO, Dimitry, M.**
  **Millbrae, CA California 94030 (US)**
- **DE SAUVAGE, Frederic, J.**
  **Foster City, CA California 94404 (US)**
- **GHILARDI, Nico, P.**
  **Millbrae, CA California 94030 (US)**
- **MODRUSAN, Zora**
  **Fremont, CA California 94536 (US)**
- **OUYANG, Wenjun**
  **Foster City, CA California 94404 (US)**
- **VALDEZ, Patricia, A.**
  **Kensington. MD 20895 (US)**
- **ZHENG, Yan**
  **Ballwin, MO Missouri 63021 (US)**

(74) Representative: **Walton, Seán Malcolm et al**
**Mewburn Ellis LLP**
**City Tower**
**40 Basinghall Street**
**London EC2V 5DE (GB)**

(56) References cited:
**WO-A-02/29098** **WO-A-99/61617**
**US-A1- 2005 277 593**

- **WOLK K ET AL: "IL-22 regulates the expression of genes responsible for antimicrobial defense, cellular differentiation, and mobility in keratinocytes: a potential role in psoriasis", EUR J IMMUNOL, vol. 36, 2006, pages 1309-1323, XP002525091,**
- **CASH H L ET AL: "Symbiotic bacteria direct expression of an intestinal bactericidal lectin", SCIENCE, AMERICAN ASSOCIATION FOR THE ADVANCEMENT OF SCIENCE, WASHINGTON, DC; US, vol. 313, no. 5790, 25 August 2006 (2006-08-25), pages 1126-1130, XP002540223, ISSN: 0036-8075, DOI: 10.1126/SCIENCE.1127119**
- **OGAWA HITOSHI ET AL: "Increased expression of HIP/PAP and regenerating gene III in human inflammatory bowel disease and a murine bacterial reconstitution model.", INFLAMMATORY BOWEL DISEASES MAY 2003, vol. 9, no. 3, May 2003 (2003-05), pages 162-170, XP002688917, ISSN: 1078-0998**

EP 2 514 436 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KEILBAUGH S A ET AL: "Activation of RegIII beta/gamma and interferon gamma expression in the intestinal tract of SCID mice: an innate response to bacterial colonisation of the gut", GUT, BRITISH MEDICAL ASSOCIATION, LONDON, UK, vol. 54, 1 January 2005 (2005-01-01), pages 623-629, XP002540221, ISSN: 0017-5749, DOI: 10.1136/GUT.2004.056028

**Description**

## FIELD OF THE INVENTION

The present invention relates generally to the treatment of microbial disorders by modulation of the host immune response.

## BACKGROUND

Infection by microbial pathogens represents a major cause of death worldwide and continues to pose a serious threat to global health (WHO, The World Health Report (2004)). For example, Attaching and effacing (A/E) bacterial pathogens, such as enterohemorrhagic *Escherichia coli* (EHEC) and enteropathogenic *E. coli* (EPEC) are among the bacteria that cause diarrhea, morbidity and mortality, especially among infants and children in the developing world (2). *E. coli* O157:H7, one of the EHEC strains, caused many people to be hospitalized and 3 mortalities last year in the United States (MMWR Morb Mortal Wkly Rep 55, 1045 (Sep 29, 2006)). It is also believed that more than 90% of all cases of post-diarrhea hemolytic uremic syndrome (HUS) in industrialized countries were caused by *E. coli* O157:H7 infection (R. L. Siegler, Pediatr Clin North Am 42, 1505 (Dec, 1995)). Other EPEC strains such as *E. coli* O55:H7 also cause intestinal illness among infants world wide (T. S. Whittam et al., Infect Immun 61, 1619 (May, 1993)). Much of our knowledge on how hosts control the infection of A/E pathogens comes from the study of infection by *Citrobacter rodentium,* a natural pathogen in mice (L. Eckmann, Ann NY Acad Sci 1072, 28 (August 1, 2006)). Similar to the pathogenesis of EHEC or EHPC in human, intimately attaching of C. *rodentium* to murine colonic epithelial cells results in effacement of brush border microvilli, termed as attaching and effacing (A/E) lesion, and colonic hyperplasia (D. B. Schauer, S. Falkow, Infect Immun 61, 2486 (Jun, 1993)).

Both intestinal epithelial and immune cells play critical roles in host defense against A/E pathogens. The tight junctions of intestinal epithelial cells present the first barrier to prevent microbes leaving the intestinal lumen (T. T. MacDonald, G. Monteleone, Science 307, 1920 (March 25, 2005)). Additionally, epithelial cells secrete anti-microbial peptides to control pathogens in the gastrointestinal (GI) tract (A. Takahashi et al., FEBS Lett 508, 484 (Nov 23, 2001)). Studies with immune deficient mouse strains during *C. rodentium* infection established that $CD4^+$ T cells, B cells, and anti-*C. rodentium* specific antibody responses are all essential components of the adaptive immunity to contain and eradicate infection (L. Bry, M. B. Brenner, J Immunol 172, 433 (January 1, 2004)). Many cytokines produced by lymphocytes during infection can enhance the innate immune responses of epithelial cells. The specific functions of these cytokines, however, remain unclear during A/E pathogen infection.

IL-22, an IL-10 family cytokine, is produced by lymphocytes, particularly Th17 cells (Y. Zheng et al., Nature 445, 648 (Feb 8, 2007)). Th17 cells belong to a recently discovered $CD4^+$ T helper subset that also produces IL-17. IL-17 has important functions in the control of extracellular bacterial infections (K. I. Happel et al., J. Exp. Med. 202, 761 (September 19, 2005)). The role of IL-22, however, in host defense is still largely unknown. Tumor Necrosis Factor (TNF)-related proteins arc recognized in the art as a large family of proteins having a variety of activities ranging from host defense to immune regulation to apoptosis. TNF was first identified as a serum-derived factor that was cytotoxic for several transformed cell lines *in vitro* and caused necrosis of certain tumors *in vivo.* A similar factor in the superfamily was identified and referred to as lymphotoxin ("LT"). Due to observed similarities between TNF and LT in the early 1980's, it was proposed that TNF and LT be referred to as TNF-$\alpha$ and TNF-$\beta$, respectively. Scientific literature thus makes reference to both nomenclatures. As used in the present application, the term "TNF" refers to TNF-$\alpha$. Later research revealed two forms of lymphotoxin, referred to as LT$\alpha$ and LT$\beta$. US 2005-0129614 describes another polypeptide member of the TNF ligand super-family based on structural and biological similarities, designated TL-5. Members of the TNF family of proteins exist in membrane-bound forms that act locally through cell-cell contact, or as secreted proteins. A family of TNF-related receptors react with these proteins and trigger a variety of signalling pathways including cell death or apoptosis, cell proliferation, tissue differentiation, and proinflammatory responses. TNF-$\alpha$ by itself has been implicated in inflammatory diseases, autoimmune diseases, viral, bacterial, and parasitic infections, malignancies, and/or neurodegenerative diseases and is a useful target for specific biological therapy in diseases such as RA and Crohn's disease.

## SUMMARY OF THE INVENTION

The present invention is as defined in the claims. In particular, the invention provides a polypeptide comprising IL-22 for use in a method of treating a microbial disorder in a subject, comprising administering to said subject an effective amount of the polypeptide, wherein the microbial disorder is Inflammatory Bowel Disease (IBD). The invention further provides an IL-22 agonist which increases IL-22 activity for use in a method of treating a microbial disorder in a subject, comprising administering to said subject an effective amount of the IL-22 agonist, wherein the microbial disorder is IBD and wherein the IL-22 agonist is selected from the group:

(a) an IL-22-Fc fusion polypeptide
(b) an IL-22 nucleic acid;
(c) an IL-22 fusion polypeptide;
(d) an antibody or biologically active fragment thereof, wherein the antibody or fragment binds to IL-22, IL-22R or IL-22BP;
(e) a monoclonal antibody that binds to IL-22, IL-22R or IL-22BP; and
(f) a humanized antibody that binds to IL-22, IL-22R or IL-22BP.

**BRIEF DESCRIPTION OF THE DRAWINGS**

**Fig. 1** depicts data demonstrating host defense against *C. rodentium* infection. Fig. 1(A) depicts the results of a real-time RT-PCR analysis on receptor subunits for IL-22 in uninfected wildtype mouse GI track; Fig. 1(B-F) depicts a real-time RT-PCR analysis on various cytokine expressions in wildtype mouse colons upon *C. rodentium* infection; Fig. 1(G) depicts survival of C57B1/6 (n=5), IL-23p40$^{-/-}$ (n=5), and IL-6$^{-/-}$ (n=5) mice after *C. rodentium* infection; and Fig. 1(H) depicts a time course real-time RT-PCR analysis on IL-22 and IL-17 expressions in C57B1/6, IL-23p40$^{-/-}$, and IL-6$^{-/-}$ mouse colons upon *C. rodentium* infection. For *C. rodentium* infection, the mice were orally inoculated with 2x10$^9$ CFU of bacteria. All of the above data are representative of two independent experiments.

**Fig. 2** depicts data demonstrating that IL-22 deficiency renders mice susceptible to *C. rodentium* infection. 6-7 weeks old IL-22$^{-/-}$ (Fig. 2(A-C)), IL-17RC$^{-/-}$ (D), IL-20R$\beta^{-/-}$ mice (Fig. 2(F)) or wildtype mice (Fig. 2(A-C, E)) were orally inoculated with 2x10$^9$ CFU of *C. rodentium* and weighed at indicated time points. Histologic analysis of colons from IL-22$^{-/-}$ and wildtype mice 8 days post inoculation using hematoxylin-and-eosin (H&E) staining (Fig. 2(B and C)). Arrows indicate submucosal inflammation (Fig. 2(B)), and bacterial invasion into mucosal glands (Fig. 2(C)). Representative data are shown (bars = 100 $\mu$m for Fig. 2(B) and bars = 25 $\mu$m for Fig. 2(C)). Wildtype C57B1/6 mice received 150 $\mu$g of anti-IL-22 mAb or isotype control IgG1 mAb intraperitoneally, every other day, starting on day 0 or day 8 post inoculation (Fig. 2(E)). $*p < 0.05$, $**p < 0.01$, $***p < 0.001$. All data are representative of two independent experiments.

**Fig. 3** depicts data demonstrating the effect of IL-22 deficiency in mice during *C. rodentium* infection. C57B1/6 mice (Fig. 3(A, B, and F)), IL-22$^{-/-}$ and wildtype mice (Fig. 3(C-E, and G)) were orally inoculated with 2x10$^9$ CFU of *C. rodentium.* Mice also received 150 $\mu$g of anti-IL-22 mAb or isotype control IgG1 mAb intraperitoneally every other day starting from the same day as *C. rodentium* inoculation (Fig. 3(A and B)). On day 10, colons were photographed and individual colon length was measured (Fig. 3(A)). Histologic analysis of colons was performed using hematoxylin-and-eosin (H&E) staining (Fig. 3(B)). Histologic analysis of colons and livers (day 8) from infected IL-22$^{-/-}$ and wildtype mice was performed using H&E staining (Fig. 3(C and E)). Arrows in Fig. 3(C) indicate colonic transmural inflammation and ulceration. Fig. 3(E) depicts a hepatic septic microabscess in the IL-22$^{-/-}$ mouse. Representative data are shown in Fig. 3(C), where the bars = 500 $\mu$m for the upper panels and bars = 100 $\mu$m for the lower panels. In Fig.3(E), the bars = 25 $\mu$m. Fig. 3(D) depicts the $\log_{10}$ CFU of *C. rodentium* in colon, liver, spleen, and mesenteric lymph node. Fig. 3(F-G) depicts the serum anti-*C. rodentium* IgG levels by ELISA. $*p < 0.05$. All of the above data are representative of two independent experiments.

**Fig. 4** depicts data demonstrating that IL-22 induces anti-microbial RegIII family protein expression upon *C. rodentium* infection. *In vitro* culture of C57B1/6 mouse colons were treated with 10 $\mu$g of IL-22 for 24 hours, RNA were isolated and used for microarray analysis (Fig.4(A)) and real-time RT-PCR analysis (Fig. 4(B)). In Fig. 4(C), 1L-22$^{-/-}$ mice and wildtype littermates were orally inoculated with 2x10$^9$ CFU of *C. rodentium,* and real-time RT-PCR was performed on RNA isolated from individual mouse colon collected on indicated time points. All data are representative of two independent experiments.

**Fig. 5** depicts data demonstrating the targeted disruption of the murine *IL-17RC* gene. Fig. 5(A) depicts the strategy for generation of IL-17RC knockout mice. Exons 1-5 (open boxes) encompassing the *IL-17RC* coding sequence was replaced with a neomycin resistance cassette. Fig. 5(B) depicts the genotyping of offspring from wildtype (WT) and knockout (KO) mice using the indicated primer sets (PI, P2 and P3). Tail tip fibroblasts from WT and KO mice were generated and stimulated with various concentrations of IL-17A and IL-17F *in vitro* for 24 hours, and culture supernatant were collected for IL-6 ELISA (Fig. 5(C)).

**Fig. 6** depicts data of a real-time RT-PCR analysis on IL-19, IL-20 and IL-24 expression in wildtype mouse colons upon *C. rodentium* infection, over time. C57B1/6 mice were orally inoculated with 2x10$^9$ CFU of C. *rodentium.* Colons were collected at indicated time points and isolated RNAs were used for real-time RT-PCR analysis.

**Fig. 7** depicts data demonstrating IL-20R$\alpha$ and IL-20R$\beta$ expression in the GI tract. Real-time RT-PCR analysis on receptor subunits for IL-19, IL-20 and IL-24 in uninfected wildtype mouse GI tract.

**Fig. 8** depicts data demonstrating targeted disruption of the murine *IL-20Rβ* gene. Fig. 8(A) depicts the strategy for generation of IL-20Rβ knockout mice. Exon 1 (open boxes) was replaced with a neomycin resistance cassette. Fig. 8(B) depicts the phenotyping of offspring from wildtype (WT), heterozygous (HET) and knockout (KO) mice using the indicated primer sets (p1, p2 and p3). Fig. 8(C) WT and KO mouse ears were injected intradermally with 500 ng recombinant IL-20 in 20 μl PBS or with 20 μl PBS alone. 24 hours later, mouse ears were collected for RNA isolation. Isolated RNAs were used for real-time RT-PCR analysis for genes known to be upregulated upon IL-20 signaling.

**Fig. 9** depicts data of a histologic analysis of mouse colons from anti-IL-22 mAb treated wildtype mice inoculated with *C. rodentium.* C57B1/6 mice were orally inoculated with $2x10^9$ CFU of *C. rodentium.* Mice also received 150 μg of anti-IL-22 mAb or isotype control IgG1 mAb intraperitoneally every other day starting from the same day as *C. rodentium* inoculation. On day 10, routine histologic analysis of colons was performed using hematoxylin-and-eosin (H&E) staining. Arrows indicate mucosal ulceration with transmural inflammation. Representative images are shown, bars = 500 μm for the upper panels and bars = 250 μm for the lower panels.

**Fig. 10** depicts data demonstrating serum Ig levels in IL-22$^{-/-}$ mice and wildtype littermates during *C. rodentium* infection. IL-22$^{-/-}$ and wild type littermates mice were orally inoculated with $2x10^9$ CFU of *C. rodentium.* On indicated time points, mouse blood were collected. Levels of total serum IgM and IgG (Fig. 10(A)) and serum anti-*C. rodentium* IgG2a, IgG2b, IgG2c and IgG3 (Fig. 10(B)) were determined by ELISA. All data are representative of two independent experiments.

**Fig. 11** depicts data demonstrating an *ex vivo* colon culture ELISA of IL-22 (Fig. 11(A)) and IL-17 (Fig.11(B)) expression in C57B1/6, IL-23p19$^{-/-}$, and IL-6$^{-/-}$ mouse colons after *C. rodentium* infection. For *C. rodentium* infection, mice were orally inoculated with $2x10^9$ CFU of bacteria. All data are representative of at least two independent experiments.

**Fig. 12** depicts a FACS analysis of IL-22R expression on isolated mouse IEL, LPMCs and colonic epithelial cells (Fig. 12 (A)), and a FACS analysis of IL-22R expression on primary human colonic epithelial cells (Fig. 12 (B)). All data are representative of at least two independent experiments.

**Fig. 13** depicts data demonstrating that IL-22, produced by dendritic cells (DCs), is critical for innate immune responses against *C. rodentium* infection. In Fig. 13(A), Rag2$^{-/-}$ and wildtype Balb/c mice were orally inoculated with $2x10^9$ CFU of *C. rodentium.* In Fig. 13(B and C), the mice also received 150 μg of isotype control IgG1 mAb or anti-IL-22 mAb intraperitoneally every other day starting at the same day as bacteria inoculation and were weighed at the indicated time points. Fig. 13(B) depicts a time course real-time RT-PCR analysis, and Fig. (13(C) depicts *an ex vivo* colon culture ELISA of IL-22 and IL-17 expression in colons of wildtype Balb/c and Rag2$^{-/-}$ mice following *C. rodentium* infection. Fig. 13(D) depicts the immunohistochemical staining for IL-22, CD11c, and DAPI in day 4 colons from *C. rodentium* infected Rag2$^{-/-}$ miceMagnification: 400x. Fig. 13(E) depicts data demonstrating that IL-23 directly induces IL-22 production, as measured by ELISA, from isolated murine CD11c$^+$ DCs *in vitro.* All data are representative of two independent experiments.

**Fig. 14** depicts data demonstrating that IL-22 can induce STAT3 activation in human colon cells lines. In Fig. 14(A), IL-22$^{-/-}$ mice and wildtype littermates were orally inoculated with $2x10^9$ CFU of *C. rodentium.* One group of IL-22$^{-/-}$ mice also received mRegIIIγ-Ig fusion protein. Animals were weighed and monitored at the indicated time points. *$p$ < 0.05, **$p$ < 0.01. In Fig. 14(B), IL-23 directly induces IL-22 production from isolated human DCs, measured by ELISA. Fig. 14(C) depicts IL-22R expression by FACS on human colon cell lines. Fig. 14(D) depicts a Western blotting showing that IL-22 can induce STAT3 activation in human colon cell lines. Fig.14(E) depicts a real-time RT-PCR analysis for RegIIIβ and RegIIIγ expression in human colonic epithelial cell lines treated with IL-22. All data are representative of two independent experiments.

**Fig. 15** depicts the characterization of anti-IL-22 mAb for immunohistochemisty. Fig. 15(A) depicts colon sections from day4 *C. rodentium* infected IL-22$^{-/-}$ and wildtype mice or uninfected wildtype mice, stained with Alexa555 conjugated anti-IL-22 mAb (8E11) or isotype control. Fig 15(B) depicts cell pellets of IL-22-expressing 293 cells stained with Alexa555 conjugated anti-IL-22 mAb (8E11) or isotype control. The magnification is at 200x.

**Fig. 16** depicts a time-course analysis on RegIIIγ and RegIIIβ expression in C57B1/6 and IL-23p19-/- mouse colons following *C. rodentium* infection. C57B1/6 and IL-23p19-/- mice were orally inoculated with 2x109 CFU of *C. rodentium.* At the indicated time points, mouse colons were collected for RNA extraction and subsequently real-time RT-PCR analysis on mouse RegIIIγ and RegIIIβ expression.

**Fig. 17** depicts a time-course analysis on other Reg family members expressions in IL-22-/- and wildtype mouse colons following *C. rodentium* infection. IL-22-/- and wild type littermates mice were orally inoculated with 2x109 CFU of C. *rodentium.* At the indicated time points, mouse colons were collected for RNA extraction and subsequently real-time RT-PCR analysis.

**Fig. 18** depicts data demonstrating that recombinant human RegIII_fusion protein can partially protect IL-22-/- following *C. rodentium* infection. IL-22-/- mice and wildtype littermates were orally inoculated with 2x109 CFU of *C.*

*rodentium.* One group of IL-22-/- mice also received human RegIII_-cFlag fusion proteins. Animals were weighed and monitored at the indicated time points. *$p < 0.05$.

**Fig. 19** A-C depicts 161 genes differentially expressed in colon, from IL-22 treatment.

**Fig. 20** depicts the 2D hierarchical clustering of 161 genes differentially expressed in colon from IL-22 treatment, where selected genes were clustered by iterative agglomeration of vectors most highly linked by Pearson correlation coefficient, with data for agglomerated vectors summarized by average linkage.

**Fig. 21** depicts data demonstrating LTbRFc and anti-IL-22 mAb both lead to mortality after C. rodentium infection.

**Fig. 22** depicts data demonstrating LT pathway regulation of multiple upstream aspects involved in IL-22 production.

**Fig. 23** depicts data demonstrating IL-22 partially rescues the defects seen in LTbR treated mice.

**Fig. 24** depicts data demonstrating anti-IL-22 mAb treatment leads to reduced colon follicles, compromised B/T organization, and reduced DC, T cell and B cell numbers in the colon.

## DETAILLED DESCRIPTION OF THE INVENTION

The present inventors discovered a novel cytokine pathway that mediates immune response and resistance of mammals to infectious microbial pathogens. In particular, the present inventors discovered that IL--22 is one of the key cytokines that bridges adaptive immune response and innate epithelial defense during early infection of an attaching or effacing (A/E) bacterial pathogen.

As shown herein, cytokines such as IL-22 that are produced by immune cells during the early stages of infection are necessary for intestinal epithelial cells to elicit a full-anti-microbial response and wound-healing response in order to prevent systemic invasion of pathogenic microbes into the host. The studies herein show that IL-22 protects the integrity of the intestinal epithelial barrier and prevents bacterial invasion with systemic spread. Further, the studies herein indicate that IL-22 is involved in the elicitation of the early anti-bacterial IgG responses, and is indespensable for the induction of anti-microbial lectins, such as RegIII$\beta$ and RegIII$\gamma$, from colonic epithelial cells during bacterial infection. The lack of either or both of these mechanisms may contribute to the compromised host defense response with increased systemic spread and mortality in IL-22$^{-/-}$ mice during C. rodentium infection.

As shown herein, the induction of RegIII$\beta$ and RegIII$\gamma$ indicates that IL-22 may have broader functions in controlling various bacterial infections. The studies herein further support the role of $Th_{IL-17}$ cells and their effector cytokines in infectious disorders and autoimmune disorders. Further, the studies herein indicate that IL-22 and its downstream products, such as RegIII$\beta$ and RegIII$\gamma$, may be beneficial for the treatment of infectious disorders.

## GENERAL TECHNIQUES

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y.; Current Protocols in Molecular Biology (F. M. Ausubel, et al. eds., (2003)); the series Methods in Enzymology (Academic Press, Inc.): PCR 2: A Practical Approach (M. J. MacPherson, B. D. Hames and G. R. Taylor eds. (1995)), Harlow and Lane, eds. (1988) Antibodies, A Laboratory Manual*,* and Animal Cell Culture (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Handbook of Experimental Immunology (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J.B. Lippincott Company, 1993).

## I. DEFINITIONS

For purposes of interpreting this specification, the following definitions will apply and whenever appropriate, terms used in the singular will also include the plural and vice versa.

An "anti-microbial polypeptide" or "AMP" is a polypeptide that mediates, or otherwise effects, an anti-microbial immune response to a microbial pathogen, and encompasses encompasses a fragment, variant, analog, derivative or mimetic thereof that retains an AMP activity, e.g., an anti-microbial activity, or activity for modulating an anti-microbial immune response. These methods can be used to treat subjects that are infected with or at risk for infection with an infectious microbial pathogen, e.g., a virus or bacterium. The activity of the AMP can be modulated or differentially regulated (e.g., up or down regulated) relative to another AMP or the same AMP.

An AMP encompasses a native AMP and variant forms thereof (which are further defined herein), and may be isolated from a variety of sources, such as from human tissue or from another source, or prepared by recombinant or synthetic methods. A native AMP may be from any species, e.g., murine or human. AMPs include, but are not limited to, LT, IL-6, IL-18, IL-22, IL-23 (including e.g., IL-23 p19 or IL-23 p40), and Reg or Reg-related proteins encoded by the genes of the Reg super family. The Reg super family includes Reg and Reg-related genes from human, rat, and mouse and are grouped into four subclasses, types I, II, III, and IV. For example, type I includes human *REG Iα,* human *REG 1β,* rat *RegI,* and mouse *RegI;* type II includes mouse *RegII;* type III includes human REG III, human *HIP/PAP* (gene expressed in hepatocellular carcinoma-intestine-panercas/gene encoding pancreatitis-associated protein), rat *PAP/Peptide23,* rat *RegIII/PAPII,* rat *PAP III,* mouse *RegIIIα, RegIIIβ, RegIIIγ,* mouse *RegIIIδ,* and hamster *INGAP* (islet neogenesis-associated protein). Type IV contains human *REG IV.* Additionally, human Reg-related Sequence (RS) is reportedly a pseudogene. In one embodiment, the REG protein is encoded by a member of the human REG gene family which includes, but is not limited to, REG Iα, REG Iβ, HIP/PAP, REG III, REG IV, and Reg-related sequence (RS).

Lymphotoxin (LT) is a trimeric cytokine in the tumor necrosis family; expressed by activated T, B, and NK cells; and involved in inflammatory response signaling and secondary lymphoid organ architecture. "Lymphotoxin-" or "LT" is defined herein as a biologically active polypeptide having the amino acid sequence shown in FIG. 2A of US Pat. No. 5,824,509. "LT" is defined to specifically exclude human TNFα or its natural animal analogues (Pennica et al., Nature 312:20/27 : 724-729 (1984) and Aggarwal et al., J. Biol. Chem. 260: 2345-2354 (1985)). As used herein, "LT" refers to one or more LT subunits as described herein.

"Lymphotoxin-α" or "LTα" is defined to specifically exclude human LTβ as defined, for example, in US 5,661,004. "Lymphotoxinα-3 trimer" or "LTα3" refers to a homotrimer of LTa monomers. This homotrimer is anchored to the cell surface by the LTβ, transmembrane and cytoplasmic domains.

"Lymphotoxin-αβ" or "LTαβ" or "LTαβ complex" refers to a heterotrimer of LTα with LTβ. These heterotrimers contain either two subunits of LTα and one subunit of LTβ (LTα2β1), or one subunit of LTα and two of LTβ (LTα1β2). The term "LTαβ" or "LTαβ" as used herein refers to a heterotrimer made up of one subunit of LTα and two of LTα (LTα1β2).

"Tumor necrosis factor receptor-1" or "TNFRI" and "tumor necrosis factor receptor-II" or "TNFRII" refer to cell-surface TNF receptors for the LTα3 homotrimer, also known as p55 and p75, respectively.
"Lymphotoxin-β receptor" or "LTβ-R" refers to the receptor to which the LTαβ heterotrimers bind.

The amino acid sequence of an AMP may comprise an amino acid sequence selected from the following group: SEQ ID NO: 2 (human IL-6), SEQ ID NO: 4 (human IL-12B), SEQ ID NO: 6 (human IL-18), SEQ ID NO: 8 (human IL-22), SEQ ID NO: 10 (human IL-23 p19 or IL-23A), SEQ ID NO: 12 (human REG1A), SEQ ID NO: 14 (human REG1B), (SEQ ID NO: 16 (human REG3A, variant 1), SEQ ID NO: 18 (human REG3A, variant 2), SEQ ID NO: 20 (human REG3A, variant 3), SEQ ID NO: 22 (human REG3G, variant 2), SEQ ID NO: 24 (human REG3G, variant 1), SEQ ID NO: 26 (human REG4), SEQ ID NO: 28 (murine IL-6), SEQ ID NO: 30 (murine IL-12B), SEQ ID NO: 32 (murine IL-18), SEQ ID NO: 34 (murine IL-22), SEQ ID NO: 36 (murine IL-23 p19 or IL-23A), SEQ ID NO: 38 (murine PAP), SEQ ID NO: 40 (murine REG1), SEQ ID NO: 42 (murine REG2), SEQ ID NO: 44 (murine REG3A), SEQ ID NO: 46 (murine REG3D), SEQ ID NO: 48 (murine REG4), SEQ ID NO: 50 (human LTα), SEQ ID NO: 52 (human LTβ), SEQ ID NO: 54 (murine LTα), and SEQ ID NO: 56 (murine LTβ).

The nucleic acid sequence encoding an AMP may comprise a nucleic acid sequence selected from the following group: SEQ ID NO: 1 (human IL-6), SEQ ID NO: 3 (human IL-12B), SEQ ID NO: 5 (human IL-18), SEQ ID NO: 7 (human IL-22), SEQ ID NO: 9 (human IL-23 p19 or IL-23A), SEQ ID NO: 11 (human REG1A), SEQ ID NO: 13 (human REG1B), SEQ ID NO: 15 (human REG3A, variant 1), SEQ ID NO: 17 (human REG3A, variant 2), SEQ ID NO: 19 (human REG3A, variant 3), SEQ ID NO: 21 (human REG3G, variant 2), SEQ ID NO: 23 (human REG3G, variant 1), SEQ ID NO: 25 (human REG4), SEQ ID NO: 27 (murine IL-6), SEQ ID NO: 29 (murine IL-12B), SEQ ID NO: 31 (murine IL-18), SEQ ID NO: 33 (murine IL-22), SEQ ID NO: 35 (murine IL-23 p19 or IL-23A), SEQ ID NO: 37 (murine PAP), SEQ ID NO: 39 (murine REG1), SEQ ID NO: 41 (murine REG2), SEQ ID NO: 43 (murine REG3A), SEQ ID NO: 45 (murine REG3D), SEQ ID NO: 47 (murine REG4), SEQ ID NO: 49 (human LTα), SEQ ID NO: 51 (human LTβ), SEQ ID NO: 53 (murine LTα), and SEQ ID NO: 55 (murine LTβ).

A "native sequence AMP polypeptide" or a "native sequence AMP polypeptide" refers to a polypeptide comprising the same amino acid sequence as a corresponding AMP polypeptide derived from nature. Such native sequence AMP polypeptides can be isolated from nature or can be produced by recombinant or synthetic means. The terms specifically encompass naturally-occurring truncated or secreted forms of the specific AMP polypeptide (e.g., an IL-22 lacking its associated signal peptide), naturally-occurring variant forms (e.g., alternatively spliced forms), and naturally-occurring allelic variants of the polypeptide. The native sequence AMP polypeptides disclosed herein are mature or full-length native sequence polypeptides.

A "variant " polypeptide, refers to an active polypeptide having at least about 80% amino acid sequence identity with a full-length native polypeptide sequence. Ordinarily, a variant polypeptide will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity, and alternatively at least about 99% amino acid sequence identity to a full-length or mature native polypeptide sequence.

"Percent (%) amino acid sequence identity," is defined as the percentage of amino acid residues in a candidate sequence that are identical with the amino acid residues in a specific or reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 1 and 2 below demonstrate how to calculate the % amino acid sequence identity of the amino acid sequence designated "Reference Protein" to the amino acid sequence designated "IL-22", wherein "IL-22" represents the amino acid sequence of an IL-22 polypeptide of interest, "Reference Protein" represents the amino acid sequence of a polypeptide against which the "IL-22" polypeptide of interest is being compared, and "X, "Y" and "Z" each represent different amino acid residues.

**Table 1**

| | | |
|---|---|---|
| IL-22 | XXXXXXXXXXXXXXX | (Length = 15 amino acids) |
| Reference Protein | XXXXXYYYYYYY | (Length = 12 amino acids) |

% amino acid sequence identity = (the number of identically matching amino acid residues between the two polypeptide sequences) divided by (the total number of amino acid residues of the IL-22 polypeptide) = 5 divided by 15 = 33.3%

**Table 2**

| | | |
|---|---|---|
| IL-22 | XXXXXXXXXX | (Length = 10 amino acids) |
| Reference Protein | XXXXXYYYYYYZZYZ | (Length = 15 amino acids) |

% amino acid sequence identity = (the number of identically matching amino acid residues between the two polypeptide sequences) divided by (the total number of amino acid residues of the IL-22 polypeptide) = 5 divided by 10=50%

An "isolated" biological molecule, such as the various polypeptides, polynucleotides, and antibodiesdisclosed herein, refers to a biological molecule that has been identified and separated and/or recovered from at least one component of its natural environment.

"Active" or "activity," with reference to a polypeptide, refers to a biological and/or an immunological activity of a native polypeptide, wherein "biological" activity refers to a biological function of a native polypeptide other than the ability to induce the production of an antibody against an antigenic epitope possessed by the native polypeptide. An "immunological" activity refers to the ability to induce the production of an antibody against an antigenic epitope possessed by a native polypeptide.

The term "antagonist" is used in the broadest sense, and includes any molecule that partially or fully blocks, inhibits, or neutralizes a biological activity of a polypeptide. Also encompassed by "antagonist" are molecules that fully or partially inhibit the transcription or translation of mRNA encoding the polypeptide. Suitable antagonist molecules include, e.g., antagonist antibodies or antibody fragments; fragments or amino acid sequence variants of a native polypeptide; peptides; antisense oligonucleotides; small organic molecules; and nucleic acids that encode polypeptide antagonists or antagonist antibodies.. Reference to "an" antagonist encompasses a single antagonist or a combination of two or more different antagonists.

The term "agonist" is used in the broadest sense and includes any molecule that partially or fully mimics a biological activity of a polypeptide, e.g., a native AMP. Also encompassed by "agonist" are molecules that stimulate the transcription or translation of mRNA encoding the polypeptide. Suitable agonist molecules include, e.g., agonist antibodies or antibody fragments; a native polypeptide; fragments or amino acid sequence variants of a native polypeptide; peptides; antisense oligonucleotides; small organic molecules; and nucleic acids that encode polypeptides agonists or antibodies. Reference to "an" agonist encompasses a single agonist or a combination of two or more different agonists.

An "anti-microbial immune response" includes, but is not limited to, resistance or defense to infection by a microbial pathogen. Such resistance or defense can result in an inhibition or decrease in microbial infectivity, replication, proliferation or other activity of a microbial pathogen. In particular, treatment resulting in an anti-microbial immune response can result in the alleviation of a microbial disorder or symptom of a microbial disorder.

"Alleviation", "alleviating" or equivalents thereof, refers to both therapeutic treatment and prophylactic or preventative measures, wherein the object is to ameliorate, prevent, slow down (lessen), decrease or inhibit the targeted microbial disorder or symptom thereof. Those in need of treatment include those already with the disorder as well as those prone to having the disorder or those in whom the disorder is to be prevented.

With reference to treating a microbial disorder, "treatment", "treating", or equivalents thereof, refers to alleviating a microbial disorder or a symptom of a microbial disorder, in a subject having the disorder.

"Chronic" administration refers to administration of an agent(s) in a continuous mode as opposed to an acute mode, so as to maintain the initial therapeutic effect for an extended period of time. "Intermittent" administration is treatment that is not consecutively done without interruption, but rather is cyclic in nature.

"Mammal" for purposes of treatment refers to any animal classified as a mammal, including humans, rodents (e.g., mice and rats), and monkeys; domestic and farm animals; and zoo, sports, laboratory, or pet animals, such as dogs, cats, cattle, horses, sheep, pigs, goats, rabbits, etc. In some embodiments, the mammal is selected from a human, rodent, or monkey. Similarly, "subject" for the purposes of treatment, refers to a mammalian subject, and includes both human and veterinary subjects.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibodies" (Abs) and "immunoglobulins" (Igs) are glycoproteins having similar structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (e.g., full length or intact monoclonal antibodies), polyclonal antibodies, monovalent antibodies, multivalent antibodies, multispecific antibodies (e.g., bispecific antibodies so long as they exhibit the desired biological activity) and may also include certain antibody fragments (as described in greater detail herein). An antibody can be chimeric, human, humanized and/or affinity matured.

An antibody that specifically binds to a particular antigen refers to an antibody that is capable of binding the antigen with sufficient affinity such that the antibody is useful as a diagnostic and/or therapeutic agent in targeting the antigen. Preferably, the extent of binding of such an antibody to a non-target polypeptide is less than about 10% of the binding of the antibody to the target antigen as measured, e.g., by a radioimmunoassay (RIA). In certain embodiments, an antibody that binds to a target antigen has a dissociation constant (Kd) of ≤ 1μM, ≤ 100 nM, ≤ 10 nM, ≤ 1 nM, or ≤ 0.1 nM.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Heath, Bethesda, MD (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins; IgA, IgD, IgE, IgG and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called α, δ, ε, γ, and μ, respectively. The subunit structures and three-dimensional configurations of different classes of immunoglobulins are well known and described generally in, for example, Abbas et al. Cellular and Mol. Immunology, 4th ed. (2000). An antibody may be part of a larger fusion molecule, formed by covalent or non-covalent association of the antibody with one or more other proteins or peptides.

The terms "full length antibody," "intact antibody" and "whole antibody" are used herein interchangeably to refer to an antibody in its substantially intact form, not antibody fragments as defined below. The terms particularly refer to an antibody with heavy chains that contain the Fc region.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an $F(ab')_2$ fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO93/1161; Hudson et al. (2003) Nat. Med. 9:129-134; and Hollinger et al., Proc. Natl. Acad. Sci. USA 90: 6444-6448 (1993). Triabodies and tetrabodies are also described in Hudson et al. (2003) Nat. Med 9:129-134.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody. In contrast to polyclonal antibody preparations which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage display technologies (see, e.g., Clackson et al., Nature, 352: 624-628 (1991); Marks et al., J. Mol. Biol 222: 581-597 (1992); Sidhu et al., J Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; 5,661,016; Marks et al., Bio. Technology 10: 779-783 (1992); Lonberg et al., Nature 368: 856-859 (1994); Morrison, Nature 368: 812-813 (1994); Fishwild et al., Nature Biolechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison etal., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit, or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies may be produced by procedures known in the art. Marks et al. Bio/Technology 10:779-783 (1992) describes affinity maturation by VII and VL domain shuffling. Random mutagenesis of HVR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody, "neutralizing" antibody, or "antagonist" antibody is one which inhibits or reduces a biological activity of the antigen it binds. Such antibodies may substantially or completely inhibit the biological activity of the antigen.

An "agonist antibody," as used herein, is an antibody which partially or fully mimics a biological activity of a polypeptide of interest.

Antibody "effector functions" refer to those biological activities attributable to the Fc region (a native sequence Fc region or amino acid sequence variant Fc region) of an antibody, and vary with the antibody isotype. Examples of antibody effector functions include: Clq binding and complement dependent cytotoxicity; Fc receptor binding; antibody-dependent cell-mediated cytotoxicity (ADCC); phagocytosis; down regulation of cell surface receptors (*e.g.* B cell receptor); and B cell activation.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used. Specific illustrative approaches are described in the following.

In one approach, the "Kd" or "Kd value" is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (Chen, et al., (1999) J. Mol. Biol. 293:865-881). To establish conditions for the assay, microtiter plates (Dynex) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., (1997) Cancer Res. 57:4593-4599). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 μl/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays.

According to another approach, the Kd or Kd value is measured by surface plasmon resonance assays using a BIAcore™-2000 or a BIAcore™-3000 (BIAcore, Inc., Piscataway, NJ) at 25°C with immobilized antigen CM5 chips at ~10 response units (RU). Briefly, carboxymethylated dextran biosensor chips (CM5, BIAcore Inc.) are activated with *N*-

ethyl-*N'*- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (~0.2 μM) before injection at a flow rate of 5 μl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% Tween 20 (PBST) at 25°C at a flow rate of approximately 25 μl/min. Association rates ($k_{on}$) and dissociation rates ($k_{off}$) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio $k_{off}/k_{on}$. See, e.g., Chen, Y., et al., (1999) J. Mol. Biol. 293:865-881. If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

An "on-rate," "rate of association," "association rate," or "$k_{on}$" can also be determined as described above using a BIAcore™-2000 or a BIAcore™-3000 system (BIAcore, Inc., Piscataway, NJ).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to a molecule (such as a nucleic acid, polypeptide, or antibody) so as to generate a "labeled" molecule. The label may be detectable by itself(e.g. radioisotope labels or fluorescent labels) or, in the case of an enzymatic label, may catalyze chemical alteration of a substrate compound or composition, resulting in a detectable product.

By "solid phase" is meant a non-aqueous matrix to which a molecule (such as a nucleic acid, polypeptide, or antibody) can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a nucleic acid, polypeptide, antibody, agonist or antagonist) to a mammal. The components of the liposome are commonly arranged in a bilayer formation, similar to the lipid arrangement of biological membranes.

A "small molecule" or "small organic molecule" is defined herein as an organic molecule having a molecular weight below about 500 Daltons.

An "oligopeptide" that binds to a target polypeptide is an oligopeptide that is capable of binding the target polypeptide with sufficient affinity such that the oligopeptide is useful as a diagnostic and/or therapeutic agent in targeting the polypeptide. In certain embodiments, the extent of binding of an oligopeptide to an unrelated, non-target polypeptide is less than about 10% of the binding of the oligopeptide to the target polypeptide as measured, e.g., by a surface plasmon resonance assay. In certain embodiments, an oligopeptide bnds to a target polypeptide with a dissociation constant (Kd) of ≤ 1μM, ≤ 100 nM, ≤ 10 nM, ≤ 1 nM, or ≤ 0.1 nM.

An "organic molecule" that binds to a target polypeptide is an organic molecule other than an oligopeptide or antibody as defined herein that is capable of binding a target polypeptide with sufficient affinity such that the organic molecule is useful as a diagnostic and/or therapeutic agent in targeting the polypeptide. In certain embodiments, the extent of binding of an organic molecule to an unrelated, non-target polypeptide is less than about 10% of the binding of the organic molecule to the target polypeptide as measured, e.g., by a surface plasmon resonance assay. In certain embodiments, an organic molecule binds to a target polypeptide with a dissociation constant (Kd) of ≤ 1μM, ≤ 100 nM, ≤ 10 nM, ≤ 1 nM, or ≤ 0.1 nM.

A "biological system" is an in vitro, ex vivo, or in vivo system comprising mammalian cells that share a common signaling pathway.

"Microbial disorder'" refers to a disease or condition wherein a microbial pathogen causes, mediates, or otherwise contributes to a morbidity of the disease or condition. Also included are diseases in which stimulation or intervention of an anti-microbial response has an ameliorative effect on progression of the disease. Included within this term are infectious diseases or conditions, and opportunistic diseases resulting from primary infection by a microbial pathogen. Examples of such infectious disease, include, but are not limited to, EHEC- and EPEC-caused diarrhea, Inflammatory Bowel Disease (IBD) and, more particularly, Ulcerative Colitis (UC) and Crohn's Disease (CD).

The term "T cell mediated disease" means a disease in which T cells directly or indirectly mediate or otherwise contribute to a morbidity in a mammal. The T cell mediated disease may be associated with cell mediated effects, lymphokine mediated effects, etc., and even effects associated with B cells if the B cells are stimulated, for example, by the lymphokines secreted by T cells.

An "autoimmune disorder" or "autoimmunity" refers to any condition in which a humoral or cell-mediated immune response is mounted against a body's own tissue. An "IL-23 mediated autoimmune disorder" is any autoimmune disorder that is caused by, maintained, or exacerbated by IL-23 activity.

"Inflammation" refers to the accumulation of leukocytes and the dilation of blood vessels at a site of injury or infection, typically causing pain, swelling, and redness,

"Chronic inflammation" refers to inflammation in which the cause of the inflammation persists and is difficult or impossible to remove.

"Autoimmune inflammation" refers to inflammation associated with an autoimmune disorder.

"Arthritic inflammation" refers to inflammation associated with arthritis.

"Inflammatory bowel disease" or "IBD" refers to a chronic disorder characterized by inflammation of the gastrointestinal tract. IBD encompasses ulcerative colitis, which affects the large intestine and/or rectum, and Crohn's disease, which may affect the entire gastrointestinal system but more commonly affects the small intestine (ileum) and possibly the large intestine.

The term "effective amount" is a concentration or amount of a molecule (e.g., a nucleic acid, polypeptide, agonist, or antagonist) that results in achieving a particular stated purpose. An "effective amount" may be determined empirically. A "therapeutically effective amount" is a concentration or amount of molecule which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e.g.*, $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "growth inhibitory agent" when used herein refers to a compound or composition which inhibits growth of a cell, especially a cell overexpressing any of a gene, either *in vitro* or *in vivo.* Thus, a growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell population as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; lymphotoxin-$\alpha$ and -$\beta$, mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGA-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1$\alpha$, IL-2, IL-3, IL-4, IL-5, IL-6, IL-7, IL-8, IL-9, IL-10, IL-11, IL-12, IL-6, IL-17, IL-18, IL-22, 1L-23; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

As used herein, the term "inflammatory cells" designates cells that enhance the inflammatory response such as mononuclear cells, eosinophils, macrophages, and polymorphonuclear neutrophils (PMN).

## II. Compositions and Methods

A. Anti-Microbial Polypeptides (AMP) and Modulators Thereof

Anti-microbial polypeptides (AMPs) are polypeptides that mediate, or otherwise effect, an anti-microbial immune response to a microbial pathogen. AMPs include, but are not limited to, LT, IL-6, IL-22, IL-23 (including e.g., IL-23 p19 or IL-23 p40), and Reg or Reg-related proteins encoded by the genes of the Reg super family. The Reg super family includes Reg and Reg-related genes from human, rat, and mouse and are grouped into four subclasses, types I, II, III, and IV. For example, type I includes human *REG I*$\alpha$, human *REG I*$\beta$*,* rat *RegI,* and mouse *RegI*; type II includes mouse *RegII-,* type III includes human REG III, human *HIP*/*PAP* (gene expressed in hepatocellular carcinoma-intestine-pancreas/gene encoding pancreatitis-associated protein), rat *PAP*/*Peptide23,* rat *RegIII*/*PAPII,* rat *PAP III,* mouse *RegIII*$\alpha$*, RegIII*$\beta$*, RegIII*$\gamma$*,* mouse *RegIII*$\delta$*,* and hamster *INGAP* (islet neogenesis-associated protein). Type IV contains human *REG IV.* Additionally, human Reg-related Sequence (RS) is reportedly a pseudogene. In one example the REG protein is encoded by a member of the human REG gene family which includes, but is not limited to, REG I$\alpha$, REG I$\beta$, HIP/PAP, REG III, REG IV, and Reg-related sequence (RS).

In some aspects, the amino acid sequence of an AMP comprises an amino acid sequence selected from the following group: SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, and SEQ ID NO: 56.

In other aspects, the nucleic acid sequence encoding an AMP comprises a nucleic acid sequence selected from the following group: SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ I D NO: 41, SEQ ID NO: 43, SEQ ID NO: 45, SEQ ID NO: 47, SEQ ID NO: 49, SEQ ID NO: 51, SEQ ID NO: 53, and SEQ ID NO: 55.

An activity of an AMP can be increased or decreased and/or differentially regulated relative to the activity of another AMP or the same AMP. Examples of an activity of an AMP includes, but is not limited to, AMP expression, signal transduction, binding to a binding partner, anti-microbial response, or other biological or immunological activity thereof.

An increase in the activity of one or more AMPs results in an enhanced or induced anti-microbial immune response in a subject.

AMPs include, but are not limited to, polypeptides that directly or indirectly interact with IL-22, e.g., polypeptides that are upstream or downstream of an IL-22 signal transduction pathway that mediates host resistance to infection by a microbial pathogen (e.g., a bacteria or virus). Examples of such AMPs include, but are not limited to, LT, IL-6, IL-18, and IL-23 (including e.g., IL-23 p19 or IL-23 p40).

Modulators include, but are not limited to, polypeptides and nucleic acid molecules (e.g., a DNA molecule or RNA molecule) that directly or indirectly modulate an activity of an AMP. Examples of such modulation include, but are not limited to, an increase, decrease, induction or activation, inhibition, or regulation (e.g., up or down regulation) of an activity of an AMP.

In a particular embodiment, the modulator indirectly modulates IL-22 activity by decreasing or inhibiting IL-22 Binding Protein (BP) activity and thereby, increasing IL-22 activity. In a further embodiment, the modulator decreases or inhibits binding of IL-22 BP to IL-22 and thereby, increases IL-22 activity.

The modulator may be a polypeptide e.g., a polypeptide that binds to or otherwise interacts with an AMP to increase, induce, or regulate an activity of an AMP. In one approach, the modulator is a fusion polypeptide that modulates an activity of an AMP.

In one approach, the modulator is an antibody that binds to an AMP. In a particular approach, the antibody is a monoclonal antibody. In another approach, the antibody is an antibody fragment selected from a Fab, Fab'-SH, Fv, scFv, or $(Fab')_2$ fragment. In another approach, the antibody is a fusion polypeptide (e.g., an Fc fusion polypeptide). In another approach, the antibody is a chimeric antibody. In a particular approach, the antibody is humanized. In another embodiment, the antibody is a human antibody. In another approach, the antibody binds to the same epitope as an antibody selected from a human, non-human primate, or other mammal (e.g., pig, sheep, rabbit, marmot, rat, or mouse.)

In a particular approach, the modulator is a recombinant AMP or nucleic acid molecule encoding an AMP (e.g., a DNA or RNA molecule).

In another particular approach, the modulator is a recombinant AMP or nucleic acid molecule encoding an AMP (e.g., a DNA or RNA molecule) that can be expressed in a cell.

AMPs encompass native full-length or mature AMPs as well as variants thereof. AMP variants can be prepared by introducing appropriate nucleotide changes into the DNA encoding an AMP, and/or by synthesis of the desired anti-microbial polypeptide. Those skilled in the art will appreciate that amino acid changes may alter post-translational processing of a polypeptide, such as changing the number or position of glycosylation sites or altering the membrane anchoring characteristics.

Variations in native AMP or in various domains of the AMP, as described herein, can be made, for example, using any of the techniques and guidelines for conservative and non-conservative mutations set forth, for instance, in U.S. Patent No. 5,364,934. Variations may be a substitution, deletion or insertion of one or more codons encoding the AMP that results in a change in the amino acid sequence of the AMP as compared with a native sequence AMP. Optionally, the variation is by substitution of at least one amino acid with any other amino acid in one or more domains of the AMP. Guidance in determining which amino acid residue may be inserted, substituted or deleted without adversely affecting the desired activity may be found by comparing the sequence of the AMP with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

In particular embodiments, conservative substitutions of interest are shown in Table 3 under the heading of preferred substitutions. If such substitutions result in a change in biological activity, then more substantial changes, denominated exemplary substitutions in Table 6, or as further described below in reference to amino acid classes, are introduced and the products screened.

Table 3

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantival modifications in function or immunological identity of the AMP polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and

(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on cloned DNA to produce a DNA encoding a variant AMP.

Fragments of an AMP or other polypeptides are also provided herein. Such fragments may be truncated at the N-terminus or C-terminus, or may lack internal residues, for example, when compared with a full length native protein. Certain fragments lack amino acid residues that are not essential for a desired biological activity of an AMP or polypeptide. Accordingly, in certain embodiments, a fragment of an AMP or other polypeptide , is biologically active. In certain embodiments, a fragment of full length AMP lacks the N-terminal signal peptide sequence. In certain embodiments, a fragment of full-length AMP is a soluble form of a membrane-bound AMP. For example, a soluble form of AMP may lack all or a substantial portion of the transmembrane domain.

Covalent modifications of AMPs or other polypeptides are included within the scope of this invention. One type of covalent modification includes reacting targeted amino acid residues of a polypeptide with an organic derivatizing agent that is capable of reacting with selected side chains or the N- or C-terminal residues of the polypeptide. Derivatization with bifunctional agents is useful, for instance, for crosslinking the polypeptide to a water-insoluble support matrix or surface for use in the method for purifying antibodies to the polypeptide, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., San Francisco, pp. 79-86 (1983)], acetylation of the N-tenninal amine, and amidation of any C-terminal carboxyl group.

Another type of covalent modification of a polypeptide comprises altering the native glycosylation pattern of the polypeptide. "Altering the native glycosylation pattern" is intended for purposes herein to mean deleting one or more carbohydrate moieties found in the native sequence of a polypeptide (either by removing the underlying glycosylation site or by deleting the glycosylation by chemical and/or enzymatic means), and/or adding one or more glycosylation sites that arc not present in the native sequence of the polypeptide. In addition, the phrase includes qualitative changes in the glycosylation of the native proteins, involving a change in the nature and proportions of the various carbohydrate moieties present.

A polypeptide may also be modified in a way to form a chimeric molecules comprising the polypeptide fused to another, heterologous polypeptide or amino acid sequence. In one embodiment, a chimeric molecule comprises a fusion of the polypeptide with a tag polypeptide which provides an epitope to which an anti-tag antibody can selectively bind. The epitope tag is generally placed at the amino- or carboxyl- terminus of the polypeptide. The presence of such epitope-tagged forms of the polypeptide can be detected using an antibody against the tagged polypeptide. Also, provision of the epitope tag enables the AMP to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In another embodiment, a chimeric molecule may comprise a fusion of a polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble form of a polypeptide (e.g., an AMP or polypeptide modulator thereof) in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### *1. Preparation of Polypeptides*

Polypeptides for use in present invention may be prepared by routine recombinant methods, e.g., culturing cells transformed or transfected with a vector containing a nucleic acid encoding an AMP or polypeptide modulator thereof. Host cells comprising any such vector are also provided. By way of example, host cells may be CHO cells, *E. coli,* or yeast. A process for producing any of the herein described polypeptides is further provided and comprises culturing host cells under conditions suitable for expression of the desired polypeptide and recovering the desired polypeptide from the cell culture.

Alternative methods, which are well known in the art, may be employed to prepare a polypeptide. For example, a sequence encoding a polypeptide or portion thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of a polypeptide or portion thereof may be chemically synthesized separately and combined using chemical or enzymatic methods to produce the full-length polypeptide or portion thereof.

Recombinantly expressed polypeptides may be recovered from culture medium or from host cell lysates. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metalchelating columns to bind epitope-tagged forms of a polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular polypeptide produced. LT polypeptides may be purified by expressing a tagged LT polypeptide such as, for example, an LT$\alpha$-tagged polypeptide (SEQ ID NO:61).

### *2. Detection of Gene Expression*

Expression of a gene encoding a polypeptide can be detected by various methods in the art, e.g, by detecting expression of mRNA encoding the polypeptide. As used herein, the term "detecting" encompasses quantitative or qualitative detection. By detecting gene expression of a polypeptide, one can identify, e.g., those tissues that express this gene. Gene expression may be measured using certain methods known to those skilled in the art, e.g., Northem blotting, (Thomas, Proc. Natl. Acad. Sci. USA, 77:5201-5205 [1980]); quantitative PCR; or *in situ* hybridization, using an appropriately labeled probe, based on the sequences provided herein. Alternatively, gene expression may be measured by immunological methods, such as immunohistochemical staining of tissue sections and assay of cell culture or body fluids, to quantitate directly the expression of gene product. Antibodies useful for immunohistochemical staining and/or assay of sample fluids encompass any of the antibodies provided herein. Conveniently, the antibodies may be prepared against a native sequence encoding e.g., an AMP against a synthetic peptide comprising a fragment of the AMP sequence; or against an exogenous sequence fused to AMP polypeptide or fragment thereof (including a synthetic peptide).

## B. Antibodies

Antibodies that bind to any of the above- or below- described polypeptides are described. In one embodiment, an isolated antibody that binds to an AMP and thereby modulates AMP activity, e.g., increasing an activity of the AMP. Exemplary antibodies include polyclonal, monoclonal, humanized, human, bispecific, and heteroconjugate antibodies. An antibody may be an antibody fragment, e.g., a Fab, Fab'-SH, Fv, scFv, or (Fab')2 fragment. In one embodiment, an isolated antibody that binds to an IL-22 is employed.

Exemplary monoclonal antibodies that bind an AMP are described herein. These antibodies include the anti-IL-22 antibodies designated 3F11.3 ("3F11"), 11H4.4 ("11H4"), and 8E11.9 ("8E11"), and the anti-IL-22R antibodies designated 7E9.10.8 ("7E9"), 8A12.32 ("8A12"), 8H11.32.28 ("8H11"), and 12H5, also monoclonal antibodies that compete with 3F11.3, 11H4.4, or 8E11.9 for binding to IL-22, monoclonal antibodies that bind to the same epitope as 3F11.3, 11H4.4, or 8E11.9, monoclonal antibodies that compete with 7E9, 8A12, 8H11, or 12H5 for binding to IL-22R and, monoclonal antibodies that bind to the same epitope as 7E9, 8A12, 8H11, or 12H5. Various embodiments of antibodies are provided below:

*1. Polyclonal Antibodies*

Antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the polypeptide of interest or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

*2. Monoclonal Antibodies*

Antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the polypeptide of interest or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies that bind to the polypeptide of interest. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

Monoclonal antibodies can be made by using combinatorial libraries to screen for antibodies with the desired activity or activities. For example, a variety of methods are known in the art for generating phage display libraries and screening such libraries for antibodies possessing-the desired binding characteristics. Such methods are described generally in Hoogenboom et al. (2001) in Methods in Molecular Biology 178:1-37 (O'Brien et al., ed., Human Press, Totowa, NJ), and in certain embodiments, in Lee et al. (2004) J. Mol. Biol. 340:1073-1093.

In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the antibodies can be obtained by designing a suitable antigen screening procedure to select for the phage clone of interest followed by construction of a full length antibody clone using the Fv sequences from the phage clone of interest and suitable constant region (Fc) sequences described in Kabat et al., Sequences of Proteins of Immunological Interest, Fifth Edition, NIH Publication 91-3242, Bethesda MD(1991), vols. 1-3.

The monoclonal antibodies may also be made by recombinant DNA methods, such as those described in U.S. Patent No. 4,816,567. DNA encoding the monoclonal antibodies can be readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody, or can be substituted for the variable domains of one antigen-combining site of an antibody to create a chimeric bivalent antibody.

### *3. Monovalent Antibodies*

Monovalent antibodies are also provided. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### *4. Antibody Fragments*

Antibody fragments are also provided. Antibody fragments may be generated by traditional means, such as enzymatic digestion, or by recombinant techniques. In certain circumstances there are advantages of using antibody fragments, rather than whole antibodies. The smaller size of the fragments allows for rapid clearance, and may lead to improved access to solid tumors. For a review of certain antibody fragments, see Hudson et al. (2003) Nat. Med. 9:129-134.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form $F(ab')_2$ fragments (Carter et al., Bio/Technology 10:163-167 (1992)). According to another approach, $F(ab')_2$ fragments can be isolated directly from recombinant host cell culture. Fab and $F(ab')_2$ fragment with increased in vivo half-life comprising salvage receptor binding epitope residues are described in U.S. Pat. No. 5,869,046. Other techniques for the production of antibody fragments will be apparent to the skilled practitioner. In certain embodiments, an antibody is a single chain Fv fragment (scFv). See WO 93/16185; U.S. Pat. Nos. 5,571,894; and 5,587,458. Fv and scFv are the only species with intact combining sites that are devoid of constant regions; thus, they may be suitable for reduced nonspecific binding during in vivo use. scFv fusion proteins may be constructed to yield fusion of an effector protein at either the amino or the carboxy terminus of an scFv. See Antibody Engineering, ed. Borrebaeck, supra. The antibody fragment may also be a "linear antibody", e.g., as described in U.S. Pat. No. 5,641,870, for example. Such linear antibodies may be monospecific or bispecific.

### *5. Humanized Antibodies*

Humanized antibodies are also provided. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigcn(s), is achieved. In general, the hypervariable region residues are directly and most substantially involved in influencing antigen binding.

### *6. Human Antibodies*

Human antibodies are also provided. Human antibodies can be constructed by combining Fv clone variable domain sequence(s) selected from human-derived phage display libraries with known human constant domain sequences(s) as described above. Alternatively, human monoclonal antibodies can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described herein is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published April 1, 1993). Unlike traditional humanization of non-human antibodies by CDR grafting, this technique provides completely human antibodies, which have no FR or CDR residues of non-human origin.

### *7. Bispecific Antibodies*

Bispecific antibodies are also provided. Bispecific antibodies are monoclonal antibodies that have binding specificities for at least two different antigens. In certain embodiments, bispecific antibodies are human or humanized antibodies. In certain embodiments, one of the binding specificities is for a polypeptide of interest and the other is for any other antigen. In certain embodiments, bispecific antibodies may bind to two different epitopes of a polypeptide of interest. Bispecific antibodies may also be used to localize cytotoxic agents to cells which express a polypeptide of interest, such a cell surface polypeptide. These antibodies possess a TAT226-binding arm and an arm which binds a cytotoxic agent, such as, e.g., saporin, anti-interferon-$\alpha$, vinca alkaloid, ricin A chain, methotrexate or radioactive isotope hapten. Bispecific antibodies can be prepared as full length antibodies or antibody fragments (*e.g.* $F(ab')_2$ bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different approach, antibody variable domains with the desired binding specificities (antibody-antigen combining sites) are fused to immunoglobulin constant domain sequences. The fusion, for example, is with an immunoglobulin heavy chain constant domain, comprising at least part of the hinge, CH2, and CH3 regions. In certain embodiments, the first heavy-chain constant region (CH1), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the $C_H3$ domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/00373, and EP 03089). Heteroconjugate antibodies may be made using any convenient cross-linking method. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al, Science, 229: 81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med., 175: 217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al.,J. Immunol., 148(5):1547-1553 (1992). The leucine zipper peptides from the Fos and Jun proteins were linked to the Fab' portions of two different antibodies by gene fusion. The antibody homodimcrs were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### *8. Multivalent Antibodies*

Multivalent antibodies are also provided. A multivalent antibody may be internalized (and/or catabolized) faster than a bivalent antibody by a cell expressing an antigen to which the antibodies bind. The antibodies can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. In certain embodiments, the dimerization domain comprises (or consists of) an Fc region or a hinge region. In this scenario, the antibody will comprise an Fc region and three or more antigen binding sites amino-terminal to the Fc region. In certain embodiments, a multivalent antibody comprises (or consists of) three to about eight antigen binding sites. In one such embodiment, a multivalent antibody comprises (or consists of) four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n -VD2-(X2)n -Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### *9. Single-Domain Antibodies*

Single-domain antibodies are also provided. A single-domain antibody is a single polyeptide chain comprising all or a portion of the heavy chain variable domain or all or a portion of the light chain variable domain of an antibody. In certain embodiments, a single-domain antibody is a human single-domain antibody (Domantis, Inc., Waltham, MA; *see, e.g.,* U.S. Patent No. 6,248,516 B1). In one embodiment, a single-domain antibody consists of all or a portion of the heavy chain variable domain of an antibody.

### *10. Antibody* ***Variants***

In some embodiments, amino acid sequence modification(s) of the antibodies described herein are contemplated. For example, it may be desirable to improve the binding affinity and/or other biological properties of the antibody. Amino acid sequence variants of the antibody may be prepared by introducing appropriate changes into the nucleotide sequence encoding the antibody, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (*e.g.*, charged residues such as arg, asp, his, lys, and glu) and replaced by a neutral or negatively charged amino acid (e.g., alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrascquence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.* for ADEPT) or a polypeptide which increases the serum half-life of the antibody.

In certain embodiments, an antibody is altered to increase or decrease the extent to which the antibody is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition or deletion of glycosylation sites to the antibody is conveniently accomplished by altering the amino acid sequence such that one or more of the above-described tripeptide sequences (for N-linked glycosylation sites) is created or removed. The alteration may also be made by the addition, deletion, or substitution of one or more serine or threonine residues to the sequence of the original antibody (for O-linked glycosylation sites).

Where the antibody comprises an Fc region, the carbohydrate attached thereto may be altered. For example, antibodies with a mature carbohydrate structure that lacks fucose attached to an Fc region of the antibody are described in US Pat Appl No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/011878, Jean-Mairet et al. and US Patent No. 6,602,684, Umana et al. Antibodies with at least one galactose residue in the oligosaccharide attached to an Fc region of the antibody are reported in WO 1997/30087, Patel et al. See, also, WO 1998/58964 (Raju, S.)and WO 1999/22764 (Raju, S.) concerning antibodies with altered carbohydrate attached to the Fc region thereof. See also US 2005/0123546 (Umana et al.) on antigen-binding molecules with modified glycosylation.

In certain embodiments, a glycosylation variant comprises an Fc region, wherein a carbohydrate structure attached to the Fc region lacks fucose. Such variants have improved ADCC function. Optionally, the Fc region further comprises one or more amino acid substitutions therein which further improve ADCC, for example, substitutions at positions 298, 333, and/or 334 of the Fc region (Eu numbering of residues). Examples of publications related to "defucosylated" or "fucose-deficient" antibodies include: US 2003/0157108; WO 2000/61739; WO 2001/29246; US 2003/0115614; US 2002/0164328; US 2004/0093621; US 2004/0132140; US 2004/0110704; US 2004/01 10282; US 2004/0109865; WO 2003/085119; WO 2003/084570; WO 2005/035586; WO 2005/035778; WO2005/053742; Okazaki et al. J. Mol. Biol. 336:1239-1249 (2004); Yamane-Ohnuki et at. Biotech. Bioeng 87: 614 (2004). Examples of cell lines producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such as alpha-1,6-fucosyltransferase gene, *FUT8,* knockout CHO cells (Yamane-Ohnuki et al. Biotech. Bioeng. 87: 614 (2004)).

Another type of variant is an amino acid substitution variant. These variants have at least one amino acid residue in the antibody molecule replaced by a different residue. Sites of interest for substitutional mutagenesis include the hypervariable regions, but FR alterations are also contemplated. Conservative substitutions are shown in Table 3 above under the heading of "preferred substitutions." If such substitutions result in a desirable change in biological activity, then more substantial changes, denominated "exemplary substitutions" in Table 3, or as further described above in reference to amino acid classes, may be introduced and the resulting antibodies screened for the desired binding propeties.

One type of substitutional variant involves substituting one or more hypervariable region residues of a parent antibody (*e.g.* a humanized or human antibody). Generally, the resulting variant(s) selected for further development will have modified (e.g., improved) biological properties relative to the parent antibody from which they are generated. A convenient way for generating such substitutional variants involves affinity maturation using phage display. Briefly, several hypervariable region sites (*e.g.* 6-7 sites) are mutated to generate all possible amino acid substitutions at each site. The antibodies thus generated are displayed from filamentous phage particles as fusions to at least part of a phage coat protein (e.g., the gene III product of M13) packaged within each particle. The phage-displayed variants are then screened for their biological activity (*e.g.* binding affinity). In order to identify candidate hypervariable region sites for modification, scanning mutagenesis (e.g., alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such variants are generated, the panel of variants is subjected to screening using techniques known in the art, including those described herein, and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of antibodies thereby generating an Fc region variant. The Fc region variant may comprise a human Fc region sequence (*e.g.,* a human IgG1, IgG2, IgG3 or IgG4 Fc region) comprising an amino acid modification (*e.g.* a substitution) at one or more amino acid positions including that of a hinge cysteine.

In accordance with this description and the teachings of the art, it is contemplated that in some embodiments, an antibody may comprise one or more alterations as compared to the wild type counterpart antibody, e.g. in the Fc region. These antibodies would nonetheless retain substantially the same characteristics required for therapeutic utility as compared to their wild type counterpart. For example, it is thought that certain alterations can be made in the Fc region that would result in altered (*i.e.,* either improved or diminished) C1q binding and/or Complement Dependent Cytotoxicity (CDC), e.g., as described in WO99/51642. See also Duncan & Winter Nature 322:738-40 (1988); U.S. Patent No. 5,648,260; U.S. Patent No. 5,624,821; and WO94/29351 concerning other examples of Fc region variants. WO00/42072 (Presta) and WO 2004/056312 (Lowman) describe antibody variants with improved or diminished binding to FcRs. See, also, Shields et al. J. Biol. Chem. 9(2): 6591-6604 (2001). Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). These antibodies comprise an Fc region with one or more substitutions therein which improve binding of the Fc region to FcRn. Polypeptide variants with altered Fc region amino acid sequences and increased or decreased C1q binding capability are described in US patent No. 6,194,551B1, WO99/51642. See, also, Idusogie et al. J. Immunol. 164: 4178-4184 (2000).

In one embodiment, the invention employs antibodies comprising modifications in the interface of Fc polypeptides comprising the Fc region, wherein the modifications facilitate and/or promote heterodimerization. These modifications comprise introduction of a protuberance into a first Fe polypeptide and a cavity into a second Fc polypeptide, wherein the protuberance is positionable in the cavity so as to promote complexing of the first and second Fc polypeptides. Methods of generating antibodies with these modifications are known in the art, e.g., as described in U.S. Pat. No. 5,731,168.

### *11. Antibody Derivatives*

Antibodies can be further modified to contain additional nonproteinaceous moieties that are known in the art and readily available. Preferably, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

In certain embodiments, an antibody may be labeled and/or may be immobilized on a solid support.

### *12. Heteroconjugate Antibodies*

Heteroconjugate antibodies are also provided. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### *13. Effector Function Engineering*

It may be desirable to modify an antibody with respect to effector function, so as to enhance, e.g., the effectiveness of the antibody in treating a microbial disorder. For example, cysteine residue(s) may be introduced into the Fc region, thereby allowing interchain disulfide bond formation in this region. Homodimeric antibodies with enhanced anti-anti-microbial activity may also be prepared using heterobifunctional cross-linkers. Alternatively, an antibody can be engineered that has dual Fc regions and may thereby have enhanced activity.

### *14. Vectors, Host Cells, and Recombinant Methods*

For recombinant production of an antibody, in one approach the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Generally, host cells are of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

a)Generating antibodies using prokaryotic host cells:

*(1) Vector Construction*

Polynucleotide sequences encoding polypeptide components of an antibody can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell may be used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as λGEM.TM.-11 may be utilized in making a recombinant vector which can be used to transform susceptible host cells such as E. coli LE392.

An expression vector may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Sicbenlist et al. (1980) Cell 20: 269) using linkers or adaptors to supply any required restriction sites.

In one approach, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. In one approach, the signal sequences used in both cistrons of the expression system are STII signal sequences or variants thereof.

In another approach, the production of the immunoglobulins can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby, permitting proper folding and assembly of expressed protein subunits. Proba and Pluckthun Gene, 159:203 (1995).

Antibodies can also be produced by using an expression system in which the quantitative ratio of expressed polypeptide components can be modulated in order to maximize the yield of secreted and properly assembled antibodies. Such modulation is accomplished at least in part by simultaneously modulating translational strengths for the polypeptide components.

One technique for modulating translational strength is disclosed in Simmons et al., U.S. Pat. No. 5,840,523. It utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence. In certain embodiments, changes in the nucleotide sequence are silent. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:15 1-158.

In one embodiment, a set of vectors is generated with a range of TIR strengths for each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5, 840,523. Based on the translational strength comparison, the desired individual TIRs are selected to be combined in the expression vector constructs.

Prokaryotic host cells suitable for expressing antibodies include Archacbacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. In one embodiment, gram-negative cells are used.

In one embodiment, E. coli cells are used as hosts. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coliλ 1776 (ATCC 31,537) and E. coli RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

*(2) Antibody Production*

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides are grown in media known in the art and suitable for culture of the selected host cells. Examples of suitable media include luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. In certain embodiments, for E. coli growth, growth temperatures range from about 20°C to about 39°C; from about 25°C to about 37°C; or about 30°C. The pH of the medium may be any pH ranging from about 5 to about 9, depending mainly on the host organism. In certain embodiments, for E. coli, the pH is from about 6.8 to about 7.4, or about 7.0.

If an inducible promoter is used in the expression vector protein expression is induced under conditions suitable for the activation of the promoter. In one approach, PhoA promoters are used for controlling transcription of the polypeptides. Accordingly, the transformed host cells are cultured in a phosphate-limiting medium for induction. In certain embodiments, the phosphate-limiting medium is the C.R.A.P. medium (see, e.g., Simmons et al., J. Immunol. Methods (2002), 263:133-147). A variety of other inducers may be used, according to the vector construct employed, as is known in the art.

In one approach, the expressed polypeptides are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one approach, antibody production is conducted in large quantity by a fermentation process. Various large-scale fed-batch fermentation procedures are available for production of recombinant proteins. Large-scale fermentations have at least 1000 liters of capacity, and in certain embodiments, about 1,000 to 100,000 liters of capacity. These fermentors use agitator impellers to distribute oxygen and nutrients, especially glucose (the preferred carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J. Biol. Chem. 274:19601-19605; Georgiou et al., U.S. Patent No. 6,083,715; Georgiou et al., U.S. Patent No. 6,027,888; Bothmann and Pluckthun (2000). J. Biol. Chem. 275:17100-17105; Ramm and Pluckthun (2000) J. Biol. Chem. 275:17106-17113; Arie et al. (2001) Mol. Microbiol. 39:199-210.

To minimize proteolysis of expressed heterologous proteins (especially those that are proteolytically sensitive), certain host strains deficient for proteolytic enzymes can be used. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli protease-deficient strains are available and described in, for example, Joly et al. (1998), *supra;* Georgiou et al., U.S. Patent No. 5,264,365; Georgiou et al., U.S. Patent No. 5,508,192; Hara et al., Microbial Drug Resistance, 2:63-72 (1996).

In one approach E. coli strains deficient for proteolytic enzymes and transformed with plasmids overexpressing one or more chaperone proteins are used as host cells in the expression system.

*(3) Antibody Purification*

In one approach an antibody produced herein is further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

In one approach, Protein A immobilized on a solid phase is used for immunoaffinity purification of the antibody products. Protein A is a 41kD cell wall protein from *Staphylococcus aureas* which binds with a high affinity to the Fc region of antibodies. Lindmark et al (1983) J. Immunol. Meth. 62:1-13. The solid phase to which Protein A is immobilized can be a column comprising a glass or silica surface, or a controlled pore glass column or a silicic acid column. In some applications, the column is coated with a reagents, such as glycerol, to possibly prevent nonspecific adherence of contaminants.

As the first step of purification, a preparation derived from the cell culture as described above can be applied onto a Protein A immobilized solid phase to allow specific binding of the antibody of interest to Protein A. The solid phase would then be washed to remove contaminants non-specifically bound to the solid phase. Finally the antibody of interest is recovered from the solid phase by elution.

b) <u>Generating antibodies using eukaryotic host cells:</u>

A vector for use in a eukaryotic host cell generally includes one or more of the following non-limiting components: a signal sequence, an origin of replication, one or more marker genes, an enhancer element, a promoter, and a transcription termination sequence.

*(1) Signal sequences component*

A vector for use in a eukaryotic host cell may also contain a signal sequence or other polypeptide having a specific cleavage site at the N-terminus of the mature protein or polypeptide of interest. The heterologous signal sequence selected may be one that is recognized and processed (i.e., cleaved by a signal peptidase) by the host cell. In mammalian cell expression, mammalian signal sequences as well as viral secretory leaders, for example, the herpes simplex gD signal, are available. The DNA for such a precursor region is ligated in reading frame to DNA encoding the antibody.

*(2) Origin of replication*

Generally, an origin of replication component is not needed for mammalian expression vectors. For example, the SV40 origin may typically be used only because it contains the early promoter.

*(3) Selection gene component*

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, etc.

For example, in some embodiments, cells transformed with the DHFR selection gene are first identified by culturing all of the transformants in a culture medium that contains methotrexate (Mtx), a competitive antagonist of DHFR. In some embodiments, an appropriate host cell when wild-type DHFR is employed is the Chinese hamster ovary (CHO) cell line deficient in DHFR activity (e.g., ACC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Patent No. 4,965,199.

*(4) Promoter component*

Expression and cloning vectors usually contain a promoter that is recognized by the host organism and is operably linked to nucleic acid encoding a polypeptide of interest (e.g., an antibody). Promoter sequences are known for eukaryotes. For example, virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. In certain embodiments, any or all of these sequences may be suitably inserted into eukaryotic expression vectors.

Transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes el al., Nature 297:598-601 (1982), describing expression of human β-interferon cDNA in mouse cells under the control of a thymidine kinase promoter from herpes simplex virus. Alternatively, the Rous Sarcoma Virus long terminal repeat can be used as the promoter.

*(5) Enhancer element component*

Transcription of DNA encoding an antibody by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) describing enhancer elements for activation of eukaryotic promoters. The enhancer may be spliced into the vector at a position 5' or 3' to the antibody polypeptide-encoding sequence, but is generally located at a site 5' from the promoter.

*(6) Transcription termination component*

Expression vectors used in eukaryotic host cells may also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/11026 and the expression vector disclosed therein.

*(7) Selection and transformation of host cells*

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

*(8) Culturing the host cells*

The host cells used to produce an antibody may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other supplements may also be included at appropriate concentrations that would be known to those skilled in the art. The culture conditions, such as temperature, pH, and the like, are those previously used with the host cell selected for expression, and will be apparent to the ordinarily skilled artisan.

*(9) Purification of antibody*

When using recombinant techniques, the antibody can be produced intracellularly, or directly secreted into the medium. If the antibody is produced intracellularly, as a first step, the particulate debris, either host cells or lysed fragments, may be removed, for examples, by centrifugation or ultrafiltration. Where the antibody is secreted into the medium, supernatants from such expression systems may be first concentrated using a commercially available protein concentration filter, for example, an Amicon or Millipore Pellicon ultrafiltration unit. A protease inhibitor such as PMSF may be included in any of the foregoing steps to inhibit proteolysis, and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being a convenient technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Methods 62:1-13 (1983)). Protein G is recommended for all mouse isotypes and for human γ3 (Guss et al., EMBO J. 5:15671575 (1986)). The matrix to which the affinity ligand is attached may be agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to further purification, for example, by low pH hydrophobic interaction chromatography using an elution buffer at a pH between about 2.5-4.5, preferably performed at low salt concentrations (e.g., from about 0-0.25M salt).

In general, various methodologies for preparing antibodies for use in research, testing, and clinical use are well-established in the art, consistent with the above-described methodologies and/or as deemed appropriate by one skilled in the art for a particular antibody of interest.

### C. Agonists and Antagonists

A polypeptide comprising IL-22 or an IL-22 agonist which increases IL-22 activity may be employed in the present invention as claimed. Reference to an agonist of an AMP should be construed accordingly.

In one embodiment, an agonist of an AMP is an antibody, e.g., and IL-22 antibody or an anti-IL-22R antibody. In certain embodiments, an anti-IL-22 antibody is an agonistic antibody that promotes the interaction of IL-22 with IL-22R. In certain embodiments, an anti-IL-22R antibody binds to the extracellular ligand binding domain of an IL-22R. For example, an anti-IL-22R antibody may bind to the extracellular ligand binding domain of human IL-22R, which is found in SEQ ID NO:3 from about amino acids 18-228.

In a particular embodiment, an IL-22 agonist is an antibody that binds IL-22BP and blocks or inhibits binding of IL-22BP to IL-22, and thereby induces or increases an IL-22 activity (e.g., binding to IL-22R).

In another embodiment, an agonist of an AMP is an oligopeptide that binds to the AMP. In one embodiment, an oligopeptide binds to the extracellular ligand binding domain of IL-22R. Oligopeptides may be chemically synthesized using known oligopeptide synthesis methodology or may be prepared and purified using recombinant technology. Such oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length. Such oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. USA, 82:178-182 (1985); Geysen et al., in Synthetic Peptides as Antigen, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In another described approach, an agonist of an AMP is an organic molecule that binds to the AMP, other than an oligopeptide or antibody as described herein. An organic molecule may be, for example, a small molecule. In one embodiment, an organic molecule binds to the extracellular domain of an IL-22R. An organic molecule that binds to an AMP may be identified and chemically synthesized using known methodology (see, e.g., PCT Publication Nos. WO00/00823 and WO00/39585). Such organic molecules are usually less than about 2000 daltons in size, alternatively less than about 1500, 750, 500, 250 or 200 daltons in size, wherein such organic molecules that are capable of binding to an AMP may be identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585).

An IL-22 agonist may be an organic molecule that binds IL-22BP and blocks or inhibits binding of IL-22BP to IL-22, and thereby induces or increases an IL-22 activity (e.g., binding to IL-22R).

### D. Pharmaceutical Formulations

Pharmaceutical formulations are prepared for storage by mixing an agent having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; he:camethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

Lipofcctions or liposomes can also be used to deliver an agent into a cell. Where the agent is an antibody fragment, the smallest inhibitory fragment which specifically binds to the target protein is preferred. For example, based upon the variable region sequences of an antibody, peptide molecules can be designed which retain the ability to bind the target protein sequence. Such peptides can be synthesized chemically and/or produced by recombinant DNA technology (see, e.g., Marasco et al., Proc. Natl. Acad. Sci. USA 90, 7889-7893 [1993]). Antibodies disclosed herein may also be formulated as immunoliposomes. Liposomes containing an antibody are prepared by methods known in the art, such as described in Epstein et al., Proc. Natl. Acad. Sci. USA, 82: 3688 (1985); Hwang et al.. Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556. Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of an antibody can be conjugated to liposomes as described in Martin et al., J. Biol. Chem., 257: 286-288 (1982) via a disulfide-interchange reaction. A chemotherapeutic agent (such as doxorubicin) is optionally contained within the liposome. See Gabizon et al., J. National Cancer Inst., 81(19): 1484 (1989).

An agent may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustaincd-release preparations of an agent may be prepared. Suitable examples of sustained-release preparations include semipermeable matrices of solid hydrophobic polymers containing the agent, which matrices are in the form of shaped articles, e.g., films, or microcapsules. Examples of sustained-release matrices include polyesters, hydrogens (for example, poly(2-hydroxyethylmethacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOTTM (injectable microspheres composed of lactic acid-glycolic acid copolymer and leuprolide acetate), and poly-D-(-)-3-hydroxyhutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying suifhydryl residues, lyophilizing from acidic solutions, controlling moisture content, using appropriate additives, and developing specific polymers matrix compositions.

A pharmaceutical formulation herein may also contain more than one active compound as necessary for the particular indication being treated. For example, in one embodiment, a Pharmaceutical formulation containing more than one active compound comprises 1) at least one agonist of IL-22, e.g., an antibody that binds to IL-22 and/or an antibody that binds to IL-22R; and 2) at least one antibody that binds to IL-6 or IL-23 (wherein any number of the antibodies listed in 2) may be selected in any combination). In another embodiment, a pharmaceutical formulation contains two or more active compounds having complementary activities.

### E. Methods of Treatment

The disclosure relates to methods of treating a microbial disorder.

The invention provides the claimed medical uses and kit.

Examples of a microbial pathogen include, but are not limited to, a bacteria or virus. In one embodiment, the microbial pathogen is a bacteria e.g., a gram-negative or gram-positive bacteria. In a particular embodiment, the bacteria is a gram-negative bacteria. In another embodiment, the bacteria is an attaching or effacing (A/E) bacteria and, more particularly, an enterohemorrhagic Escherichia coli (EHEC) or enteropathogenic E. Coli (EPEC). In one embodiment, the bacteria is enteropathogenic E. coli (EHEC) is E. coli 0157:1-7 or E. coli 055:H7.

The therapeutic methods comprise one or more compositions or pharmaceutical formulations. Such methods include in vitro, ex vivo, and in vivo therapeutic methods, unless otherwise indicated.

In various embodiments, the present disclosure provides methods of modulating an anti-microbial immune response by stimulating or inhibiting an AMP-mediated signaling pathway and/or $Th_{IL-17}$ cell function. Such methods are useful for treatment of microbial disorders. For example, in one embodiment, the present disclosure provides a method of enhancing an anti-microbial immune response by stimulating an AMP-mediated signaling pathway, e.g., and IL-22 and/or IL-23 mediated signaling pathway. In another embodiment, the present disclosure provides methods of modulating an anti-microbial immune response by stimulating or inhibiting a cytokine-mediated signaling pathway. For example, in one embodiment, the present disclosure provides methods of enhancing an anti-microbial immune response by stimulating a cytokine-mediated signaling pathway, e.g., an IL-22 and/or IL-23 signaling pathway. Moreover, the present disclosure provides methods of modulating an anti-microbial immune response by stimulating or inhibiting a $Th_{IL-17}$ cell function.

In one embodiment, the present disclosure provides a method of stimulating an AMP-mediated signaling pathway in a biological system, the method comprising providing an AMP agonist to the biological system. Examples of such a biological system include, but are not limited to, mammalian cells in an in vitro cell culture system or in an organism in vivo. In another embodiment, the present disclosure provides a method of inhibiting an AMP-mediated signaling pathway in a biological system, the method comprising providing an AMP antagonist to the biological system.

In a particular embodiment, the present disclosure provides a method of enhancing an anti-microbial immune response in a biological system by stimulating an IL-23 and/or IL-22 mediated signaling pathway in a biological system, the method comprising providing an IL-22 or IL-22 agonist to the biological system. In one embodiment, an IL-22 agonist is IL-22. In another embodiment, the IL-22 agonist is an antibody that binds to IL-22.

In another embodiment, a method of inhibiting an IL-23-mediated signaling pathway in a biological system is provided, the method comprising providing an IL-22 antagonist to the biological system. In one embodiment, the antagonist of IL-22 is an antibody, e.g., a neutralizing anti-IL-22 antibody and/or a neutralizing anti-IL-22R antibody.

In another embodiment, the present disclosure provides a method of stimulating a $Th_{IL-17}$ cell function, the method comprising exposing a $Th_{IL-17}$ cell to an agonist of an AMP that mediates the IL-23 mediated signaling pathway (e.g., IL-23, IL-6, or IL-22). Such methods are useful for treating a microbial disorder. In one embodiment, an IL-22 agonist is IL-22. In another embodiment, the IL-22 agonist is an antibody that binds to IL-22.

In another embodiment, a method of inhibiting a $Th_{IL-17}$ cell function is provided, the method comprising exposing a $Th_{IL-17}$ cell to an antagonist of an AMP that mediates the IL-23 mediated signaling pathway (e.g., IL-23, IL-6, or IL-22). In one embodiment the antagonist is an anti-IL-22 antibody, e.g., a neutralizing anti-IL-22 antibody.

Exemplary $Th_{IL-17}$ cell functions include, but are not limited to, stimulation of cell-mediated immunity (delayed-type hypersensitivity); recruitment of innate immune cells, such as myeloid cells (e.g., monocytes and neutrophils) to sites of inflammation; and stimulation of inflammatory cell infiltration into tissues. In one embodiment, a $Th_{IL-17}$ cell function is mediated by IL-23 and/or IL-22.

Compositions are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

For the treatment or reduction in the severity of a microbial disorder, the appropriate dosage of a composition will depend on the type of disorder to be treated, as defined above, the severity and course of the disorder, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of a disorder, about 1 μg/kg to 15 mg/kg (*e.g.,* 0.1-20 mg/kg) of a polypeptide or antibody is an initial candidate dosage for administration to a patient, whether, for example, by one or more separate administrations, or by continuous infusion. A typical daily dosage might range from about I μg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

### F. Diagnostic Methods and Methods of Detection

The disclosure also provides a method of detecting the presence of an AMP in a biological sample, comprising contacting the biological sample with an antibody to the AMP, under conditions permissive for binding of the antibody to the AMP, and detecting whether a complex is formed between the antibody and AMP.

In one embodiment, the disclosure provides a method of monitoring treatment of a microbial disorder in a subject, wherein the method comprises detecting the level of expression of a gene encoding an AMP in a test sample of tissue cells obtained from the subject in need of treatment, and the expression level in the test sample is detected.

The detection may be qualitative or quantitative. In one embodiment, the test sample comprises blood or serum. In one embodiment, detecting the level of expression of a gene encoding an AMP comprises (a) contacting an anti-AMP antibody with a test sample obtained from the mammal, and (b) detecting the formation of a complex between the antibody and an AMP in the test sample. The antibody may be linked to a detectable label. Complex formation can be monitored, for example, by light microscopy, flow cytometry, fluorimetry, or other techniques known in the art. The test sample may be obtained from an individual suspected of having a microbial disorder.

In one embodiment, detecting the level of expression of a gene encoding an AMP polypeptide comprises detecting the level of mRNA transcription from the gene. Levels of mRNA transcription may be detected, either quantitatively or qualitatively, by various methods known to those skilled in the art. Levels of mRNA transcription may also be detected directly or indirectly by detecting levels of cDNA generated from the mRNA. Exemplary methods for detecting levels of mRNA transcription include, but are not limited to, real-time quantitative RT-PCR and hybridization-based assays, including microarray-based assays and filter-based assays such as Northern blots.

In another embodiment, the disclosure provides a method of detecting the presence of an AMP in a biological sample, comprising contacting the biological sample with an antibody to the AMP, under conditions permissive for binding of the antibody to the AMP, and detecting whether a complex is formed between the antibody and AMP.

In another embodiment, the disclosure concerns a diagnostic kit containing an anti-AMP in suitable packaging. The kit preferably contains instructions for using the antibody to detect an AMP. In one embodiment, the diagnostic kit is for diagnosing a microbial disorder. In one embodiment, the diagnostic kit is for diagnosing a microbial infection.

In another embodiment, the disclosure provides a kit comprising one or more AMPs and/or modulators thereof. In another embodiment, the disclosure provides a kit comprising one or more one or more pharmaceutical compositions each comprising an AMP or modulator thereof.

## G. Assays

### *1. Cell-Based Assays and Animal Morlels*

Cell-based assays and animal models for immune diseases are useful in practicing certain embodiments. Certain cell-based assays provided in the Examples below are useful, e.g., for testing the efficacy of IL-22 antagonists or agonists.

*In vivo* animal models are also useful. Exemplary animal models are also described in the Examples below. The *in vivo* nature of such models makes them predictive of responses in human patients. Animal models of immune related diseases include both non-recombinant and recombinant (transgenic) animals. Non-recombinant animal models include, for example, rodent, *e.g.*, murine models. Such models can be generated by introducing cells into syngeneic mice using standard techniques, *e.g.*, subcutaneous injection, tail vein injection, spleen implantation, intraperitoneal implantation, implantation under the renal capsule, *etc.*

Graft-versus-host disease models provide a means of assessing T cell reactivity against MHC antigens and minor transplant antigens. Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. A suitable procedure for assessing graft-versus-host disease is described in detail in Current Protocols in Immunology, above, unit 4.3.

An animal model for skin allograft rejection is a means of testing the ability of T cells to mediate *in vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss, H. Jr. and Sachs, D. H., Fundamental Immunology, 2nd ed., W. E. Paul ed., Raven Press, NY, 1989, 889-992. A suitable procedure is described in detail in *Current Protocols in Immunology,* above, unit 4.4. Other transplant rejection models which can be used to test compounds are the allogeneic heart transplant models described by Tanabe, M. et al, Transplantation (1994) 58:23 and Tinubu, S. A. et al, J. Immunol. (1994) 4330-4338.

Contact hypersensitivity is a simple *in vivo* assay for cell mediated immune function (delayed type hypersensitivity). In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19(1): 37-44 (1998).

Additionally, compositions can be tested on animal models for psoriasis-like diseases. For example, compositions can be tested in the scid/scid mouse model described by Schon, M. P. et al, Nat. Med. (1997) 3: 183, in which the mice demonstrate histopathologic skin lesions resembling psoriasis. Another suitable model is the human skin/scid mouse chimera prepared as described by Nickoloff, B. J. et al, Am. J. Path. (1995) 146:580. Another suitable model is described in Boyman et al., J Exp Med. (2004) 199(5):731-6, in which human prepsoriatic skin is grafted onto AGR129 mice, leading to the development of psoriatic skin lesions.

Knock out animals can be constructed which have a defective or altered gene encoding a polypeptide identified herein, as a result of homologous recombination between the endogenous gene encoding the polypeptide and a DNA molecule in which that gene has been altered. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (*e.g.,* by electroporation) and cells in which the introduced DNA has homologously recombined with the endogenous DNA are selected [see *e.g.*, Li et al., Cell, 69:915 (1992)]. The selected cells are then injected into a blastocyst of an animal (*e.g.,* a mouse or rat) to form aggregation chimeras [see *e.g..* Bradley, in Teratocarcirroma,s and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### *2. Screening Assays for Drug Candidates*

Screening assays for drug candidates are designed to identify compounds that bind to or complex with a polypeptide identified herein or a biologically active fragment thereof, or otherwise interfere with the interaction of a polypeptide with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting a test compound with a polypeptide identified herein under conditions and for a time sufficient to allow the polypeptide to interact with the test compound.

In binding assays, the interaction is binding and the complex formed can be isolated or detected in the reaction mixture. In a particular embodiment, a polypeptide or the test compound is immobilized on a solid phase, *e.g.,* on a microtiter plate, by covalent or non-covalent attachments. Non-covalent attachment generally is accomplished by coating the solid surface with a solution of the polypeptide or test compound and drying. Alternatively, an immobilized antibody, *e.g.*, a monoclonal antibody specific for a polypeptide to be immobilized, can be used to anchor the polypeptide to a solid surface. The assay is performed by adding the non-immobilized component, which may be labeled by a detectable label, to the immobilized component, *e.g.*, the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the test compound interacts with but does not bind to a particular polypeptide identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad Sci. USA 88, 9578-9582 (1991)] as disclosed by Chevray and Nathans, Proc. Natl, Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

To identify compounds that interfere with the interaction of a polypeptide identified herein and other intra- or extracellular component(s), a reaction mixture may be prepared containing the polypeptide and the component under conditions allowing for the interaction of the polypeptide with the component. To test the ability of a test compound to inhibit the interaction, the reaction mixture is prepared in the absence and in the presence of the test compound. If there is a decrease in the interaction of the polypeptide with the component in the presence of the test compound, then the test compound is said to inhibit the interaction of the polypeptide with the component.

In certain embodiments, methods for identifying agonists or antagonists of an AMP comprise contacting an AMP with a candidate agonist or antagonist molecule and measuring a detectable change in one or more biological activities normally associated with the AMP. Such activities include, but are not limited to, those described in the Examples below.

In one embodiment, the disclosure provides methods for identifying agonists of an IL-22 polypeptide comprise contacting an IL-22 polypeptide with a candidate agonist molecule and measuring a detectable change in one or more biological activities normally associated with the IL-22 polypeptide. Such activities include, but are not limited to, those described in the Examples below.

### *3.Antibody binding assays*

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.*, US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

Immunhistochemistry may also be used to determine the cellular location of an antigen to which an antibody binds. For immunohistochemistry, the tissue sample may be fresh or frozen or may be embedded in paraffin and fixed with a preservative such as formalin, for example. Articles of Manufacture disclosure

In another embodiment, the disclosure provides an article of manufacture comprising compositions useful for the diagnosis or treatment of the microbial disorders described herein. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide, an antibody, an agonist, or an antagonist. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

In one embodiment, the disclosure provides an article of manufacture, comprising:

(a) a composition of matter comprising an AMP or modulator thereof (e.g., an IL-22 agonist);

(b) a container containing said composition; and
(c) a label affixed to said container, or a package insert included in said container, referring to the use of said agonist in the treatment of an microbial disorder. The composition may comprise an effective amount of the agonist.

## EXAMPLES

The following are examples of methods and compositions of the invention, and are provided herein for illustrative purposes, and are not intended to limit the scope of the present invention. It is understood that various other embodiments may be practiced, given the general description provided herein.

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

The data presented herein demonstrate for the first time that IL-22 is one of the key cytokines that bridges adaptive immune response and innate epithelial defense during early infection of an A/E bacterial pathogen. As shown herein, the induction of RegIIIβ and RegaIIIγ also indicates that IL-22 may have broader functions in controlling various bacterial infections. The data further supports the role of Th17 cells and their effector cytokines in infectious diseases and autoimmune diseases. Finally, the present studies indicate that IL-22 and its downstream products, such as RegIIIβ and RegIIIγ, may be beneficial for the treatment of certain infectious diseases.

### EXAMPLE 1: IL-23 is essential for IL-22 regulation during an infectious disease process.

The data herein demonstrate that IL-23 is essential for IL-22 regulation during an infectious disease process.

Both IL-22 receptor pairs, IL-22R and IL-10Rβ chains, are expressed in the GI tract of wildtype C57B1/6 mice (Fig. 1A). Their expression in the duodenum, jejunum, ileum, and colon are higher than they are in the skin, a tissue where IL-22 has been shown to induce hyperplasia. Consistently both colonic epithelial cells and subepithelial myofibroblasts have been reported to respond to IL-22. During C. *rodentium* infection, IL-22 was induced in the colon of wildtype mice (Fig. 1B), as were cytokines that promote Th17 cell differentiation, including the p19 and p40 subunit of IL-23 (Fig. 1C-D), and IL-6 (Fig. 1E). All of these cytokines were rapidly induced, with peak expression around day 4 post inoculation. In contrast, IL-17 induction had slower kinetics and reached its maximum level at day 12 post inoculation (Fig. 1F).

Since either IL-23 or IL-6 promotes IL-22 production from T cells *in vitro,* the present inventors sought to first define their role in regulation of IL-22 production during C. *rodentium* infection. When comparing the survival rate of wildtype, $p19^{-/-}$, $p40^{-/-}$, and $1L\text{-}6^{-/-}$ mice after *C. rodentiurn* infection, we consistently found all the mice from either the $p40^{-/-}$ group (Fig. 1G) or the $p19^{-/-}$ group (data not shown) died 10 days post inoculation. Interestingly, 60% mortality was also observed in the $IL\text{-}6^{-/-}$ group around day 12 (Fig. 1G), indicating that IL-6 is required, to a certain extent, for a total control of *C. rodentium* infection. Next we examined IL-22 and IL-17 expression in both $p19^{-/-}$ and $IL\text{-}6^{-/-}$ mice (Fig. 1H). While, IL-17 expression was not altered in $p19^{-/-}$ mice (*15*), induction of IL-22 was diminished in $p19^{-/-}$ mice compared to wildtype mice. In $IL\text{-}6^{-/-}$ mice, however, while the peak level of IL-22 was comparable to that of wildtype mice, its induction was significantly delayed (Fig 1H). Furthermore, in $IL\text{-}6^{-/-}$ mice, the induction of IL-17 was significantly reduced, consistent with an essential role of IL-6 for IL-17 production.

Directly measuring IL-22/IL-17 proteins by ELISA in *ex vivo* culture of colons from infected mice confirmed the kinetics of IL-22/IL-17 production and the absence of IL-22 induction from $p19^{-/-}$mice during *C. rodentium* infection (Fig. 11). These data for the first time demonstrate that IL-23 is essential for IL-22 regulation during an infectious disease process.

These data for the first time demonstrate that IL-23 is essential for IL-22 regulation during an infectious disease process.

### Example 2: IL-22 is a key downstream effector cytokine that contributes to the biology of IL-23 in controlling microbial infection.

The altered regulation of IL-22 in both IL-23 deficient and $IL\text{-}6^{-/-}$ mice indicated that IL-22 may play a critical role in the host defense against *C. rodentium* infection. To further examine the role of IL-22, $IL\text{-}22^{-/-}$ mice were inoculated with *C. rodentium.* While wildtype littermates transiently lost weight but were able to fully recover after day 6, $IL\text{-}22^{-/-}$ mice continued losing weight following *C. rodentium* infection (Fig. 2A). About 80% of $IL\text{-}22^{-/-}$ mice became moribund or died 12 days post *C. rodentium* inoculation (Fig. 2A). Histologic analysis of the colons from day 8 infected $IL\text{-}22^{-/-}$ mice demonstrated increased mucosal thickness when compared with that of WT mice (Fig. 2B). Coincidently, there was also increased submucosal inflammation (Arrow, Fig. 2B). Furthermore, while in control mice, *C. rodentium* infection was predominantly superficial, large numbers of bacteria penetrated deeply into colonic crypts in $IL\text{-}22^{-/-}$ mice (Arrows,

Fig. 2C). FACS analysis with an anti-IL-22R antibody (Fig. 12) revealed that IL-22R was expressed by E-cadherin positive primary murine colonic epithelial cells, but not by $CD45^+$ intra-epithelial lymphocytes (IEL) or lamina propria mononuclear cells (LPMCs) (Fig. 12A). Similarly, primary human colonic epithelial cells also expressed IL-22R (Fig. 12B). These data suggest that colonic epithelial cells were directly targeted by IL-22.

These data support the importance of IL-22 in host defense against *C. rodentium* infection, and indicate that IL-22 may be one of the key downstream effector cytokines that contribute to the biology of IL-23 in controlling microbial infections.

**Example 3: IL-17A and IL,-17F pathways are not required for host defense against *C. rodentium* infection.**

The partial impairment of host defense in IL-$6^{-/-}$ mice against *C. rodentium* could also be explained by the delayed induction of IL-22 in these mice (Fig. 1H, left panel). However, it is also possible that lethality in *C. rodentium* infected IL-$6^{-/-}$ mice may have been due to their inability to upregulate IL-17 (Fig. 1H, right panel). The IL-17 pathway is crucial for the control of many extracellular bacterial infections, such as *Klebsiella prreumoniae.* IL-17 signals through IL-17R and IL-17RC (D. Toy et al., J Immunol 177, 36 (July 1, 2006)), and induces prointlammatory responses from many cell types, including epithelial cells (J. Witowski, K. Ksiazek, A. Jorres, Cell Mol Life Sci 61, 567 (Mar, 2004)). To analyze the role of the IL-17 pathway during *C. rodentium* infection, IL-$17RC^{-/-}$ mice were generated (Fig. 5). Compared to wildtype littermates, there was no obvious defect in IL-$17RC^{-/-}$ mice in terms of development or composition of T cells, B cells and other immune cells (data not shown). However, fibroblasts generated from the tail tip (Fig. 5C) or lung tissue (data not shown) of IL-$17RC^{-/-}$ mice were completely incapable of producing IL-6 when stimulated with either IL-17A or 1L-17F, indicating that IL-17RC is an essential receptor for both IL-17A and IL,-17F mediated functions. Following *C. rodentium* inoculation, both IL-$17RC^{-/-}$ mice and wildtype littennates survived the course of infection without any significant loss of weight (Fig. 2D) or any histologic differences in the colon (data not shown).

These results indicate that IL-17A and IL,-17F pathways were not required for host defense against *C. rodentium* infection, directly excluding the possibility that defective 1L-17 production is the major cause of observed mortality in IL-$6^{-/-}$ mice. Thus, the delayed induction of IL-22 observed in IL-$6^{-/-}$ mice might be the reason that these mice were incapable of surviving the infection. Other factors downstream of IL-6, however, may also be important. The results from IL-$6^{-/-}$ mice imply that the early induction of IL-22 might be critical for the host to mount a sufficient response against *C. rodentium* infection in order to prevent lethality.

**Example 3: IL-22 plays a critical role in the early stage of bacterial infection.**

To determine whether early induction of IL-22 is critical for the host to mount a sufficient response against *C. rodentium* infection in order to prevent lethality, anti-IL-22 neutralizing antibody was administrated every other day starting either at day 0 or at day 8 post inoculation of *C. rodentium.* As expected, mice that received anti-IL-22 mAb at the same time as the bacterial inoculation continued to lose weight, and all became moribund or died 12 days post inoculation. In contrast, all isotype control antibody treated animals survived (Fig. 2E). Mice that received anti-IL-22 mAb starting 8 days post inoculation had a similar outcome as did isotype mAb treated mice, with full recovery from infection.

Therefore, these data indicate that IL-22 plays a critical role in the early stage of *C. rodentium* infection, but plays no role during the later phase of host defense when bacteria are being eradicated.

**Example 4: IL-19, IL-20, and IL-24 are dispensable for host defense against bacterial infection.**

Other IL-10 family cytokines, IL-19, IL-20, and IL-24, all induce similar biological functions as those induced by IL-22 in human epidermal keratinocytes (S. M. Sa et al., J Immunol 178, 2229 (February 15, 2007).). IL-19, IL-20, and IL-24 were all upregulated in wildtype mouse colon during *C. rodentium* infection (Fig. 6). They may, therefore, play similar role as does IL-22 during *C. rodentium* infection. IL-19 signals through IL-20Rα and 1L-20Rβ chains. IL-20 and IL-24 can signal through two different receptor pairs, IL-20Rα/IL-20Rβ and IL-22R/IL-20Rβ (J.-C. Renauld, Nature Reviews Immunology 3, 667 (2003)). Therefore, IL-20Rβ is the common receptor chain for these three cytokines. In the GI tract, expression of IL-20Rα and IL-20Rβ chains was significantly lower than the expression of these chains in skin (Fig. 7).

To critically address the role of these three cytokines during *C. rodentium* infection, IL-$20R\beta^{-/-}$mice were generated (Fig. 8). These mice exhibited normal development with similar lymphocyte composition and development in all major lymphoid organs when compared to wildtype mice (data not shown). The ear skin from these mice failed to upregulate S100 family proteins when treated with recombinant IL-20, indicating a defect in IL-20 signaling *in vivo* (Fig. 8C).

IL-$20R\beta^{-/-}$ mice survived *C. rodentium* infection as well as wildtype mice did (Fig. 2F), demonstrating that IL-19, IL-20, and IL-24 are dispensable for host defense against *C. rodentium.*

**Example 5: IL-22 deficiency may compromise epithelial integrity during the early stage of *C. rodentium* infection.**

The present inventors examined the downstream mechanisms of IL-22 during *C. rodentium* infection. Both 1L-$22^{-/-}$ mice and wildtype mice treated with anti-IL-22 mAb on day 0 developed more severe bloody diarrhea and an increased incidence of rectal prolapse compared to control mice 8 days post inoculation of *C. rodentitm* (data not shown). Colons from IL-$22^{-/-}$ mice (data not shown) or day 0 anti-IL-22 mAb treated mice were thickened and shortened 10 days post inoculation (Fig. 3A), as well as having a smaller cecum, compared to control mice. Histologic analysis further revealed increased inflammation in colons lacking IL-22 signaling (Fig. 3B). There were also marked multifocal mucosal ulceration and multiple foci of transmural inflammation in both IL-$22^{-/-}$ and anti-IL-22 mAb treated mice (Fig. 3C, and Fig. 9). Furthermore, the bacterial burdens in mesenteric lymph node, spleen, and liver of IL-$22^{-/-}$ mice were significantly increased compared to those of wildtype mice. Interestingly, the difference in bacterial burdens in colons of wildtype mice and IL-$22^{-/-}$ mice was negligible (Fig. 3D). Consistent with these results, there was also evidence of systemic bacterial spread, particularly in the livers of IL-$22^{-/-}$ mice, where multifocal hepatocellular necrosis with embolic microabscessation was evident (Fig. 3E).

In conclusion, these data indicate that the epithelial integrity is compromised in IL-$22^{-/-}$ mice during the early stage of *C. rodentium* infection.

**Example 6: IL-22 deficiency leads to a reduction in anti-bacterial IgG titers**

Previous studies established the essential role of anti-*C. rodentium* antibodies in the clearance of bacteria. Transferring serum from wildtype mice post-infection fully rescued CD$4^{-/-}$ mice from death following *C. rodentium* challenge *(10).* In our studies, IL-22 deficient mice became moribund or died starting around day 8, when antibody responses were not fully developed in wildtype mice (Fig. 3F). On day 8, anti-*C. rodentium* antibody titers were 50 fold less than those on day 16 in wildtype mice. However, when we compared the titers of anti-*C. rodentium* antibodies on day 8 from wildtype and IL-$22^{-/-}$ mice, there was an unexpected significant reduction in the anti-C. *rodentium* IgG titer in IL-$22^{-/-}$ mice compared to that in wildtype mice (Fig. 3G). In contrast, there was no decrease in total IgG, IgM, IgA or anti-*C. rodentium* IgM and IgA titers in IL-$22^{-/-}$ mice (Fig. 10A and data not shown). Further, IgG subtype analysis revealed that while there was no anti-*C. rodentium* IgG1 in either wildtype or IL-$22^{-/-}$ mice 8 days post inoculation, other anti-*C. rodentium* IgG subtypes, including IgG2a, IgG2b, IgG2c and IgG3, were all significantly reduced in It-$22^{-/-}$mice (Fig. 10B). It was unlikely that differences in anti-bacterial specific IgG contributed to clearance of *C. rodentium* from the colon at this time point, especially since IgG is not targeted to the colonic mucosal lumen, and colonic bacterial burdens in both wildtype and IL-$22^{-/-}$ mice were similar (Fig. 3D). It is possible, though that circulating anti-*C. rodentium* IgG may be important in controlling penetration of *C. rodentium* through the intestinal epithelial barrier, and preventing systemic spread, since a recent study demonstrated that circulating IgG, but not secretory IgA or IgM, was required for systemic clearance of *C. rodentium* (C. Maaser et al., Infect. Immun. 72, 3315 (June 1, 2004)). How IL-22 deficiency leads to a reduction in anti-bacterial IgG titers is unclear. It is unlikely that IL-22 directly acts on B cells, since the expression of IL-22R is not detectable on B cells (S. Lecart et al., Int. Immunol. 14, 1351 (November 1, 2002)). Nonetheless, reduced anti-*C. rodentium* IgG might be one of the factors that contribute to the defective host defense response in IL-$22^{-/-}$ mice during *C. rodentium* infection.

**Example 7: IL-22 was indispensable for the induction of anti-microbial lectins, such as RegIIIβ and RegIIIγ, from colonic epithelial cells during bacterial infection.**

IL-22 treatment of colon tissues from uninfected wildtype mice *ex vivo* upregulated many anti-microbial proteins, including S100A8, S100A9, RegIIIβ, RegIIIγ, haptoglobin, SAA3, and lactotransferrin by microarray analysis (Fig.s 4A, 19, and 20). The induction of these proteins was confirmed by real-time RT-PCR (Fig. 4B and data not shown). During *C. rodentium* infection, however, only S100A8, S100A9, RegIIIβ and RegIIIγ were differentially expressed in IL-$22^{-/-}$ mice compared to wildtype mice (Fig. 4C). All other genes were either not induced or were not different in colons of wildtype vs. IL-$22^{-/-}$ mice (data not shown). Expression of both S100A8 and S100A9 was slightly higher in the colons of IL-$22^{-/-}$ mice than it was in wildtype colon on day 4 and day 6, suggesting that differential expression of these proteins was most likely not responsible for the increased mortality observed in IL-$22^{-/-}$ mice during *C. rodentium* infection. Differences were not found in the expression of defensins, proteins that are important in host defense of infected epithelium (T. Ganz, Science 286, 420 (October 15, 1999)), between wildtype and iL-$22^{-/-}$ mice (data not shown). Interestingly, the upregulation of RegIIIβ and RegIIIγ observed in wild type mice was completely abolished in IL-$22^{-/-}$ mice post *C. rodentium* inoculation (Fig. 4C), indicating that these two proteins had potential functions in controlling *C. rodentium* infection. Both RegIIIβ and RegIIIγ belong to a family of secreted C-type lectin proteins (H. L. Cash, C. V. Whitham, L. V. Hooper, Protein Expression and Purification 48, 151 (2006)). RegIIIβ and RegIIIγ expression levels increase dramatically in response to bacterial colonization as well as following other inflammatory stimuli in mice (S. A. Keilbaugh et al., Gut 54, 623 (May 1, 2005) H. Ogawa et al., Inflammatory Bowel Diseases 9, 162 (2003) H. Ogawa, K. Fukushima, I. Sasaki, S. Matsuno, Am JPhysiol Gastrointest Liver Physiol 279, G492 (Sep, 2000)).

RegIIIβ or RegIIIγ may prevent the invasion of *C. rodentium* deep into the colonic crypts, as we saw no differences in bacterial burdens from the colons of IL-22$^{-/-}$ vs. wildtype mice (Fig. 3D). Alternatively, RegIIIβ or RegIIIγ proteins may act as autocrine growth factors that play a role in epithelial repair and/or protection in the setting of intestinal inflammation (H. Ogawa et al., Inflammatory Bowel Diseases 9, 162 (2003); S. L. Pull, J. M. Doherty, J. C. Mills, J. I. Gordon, T. S. Stappenbeck, PNAS 102, 99 (January 4, 2005); V. Moucadel et al., Eur J Cell Biol 80, 156 (Feb, 2001)).

**Example 8: Adaptive immunity is not essential for IL-22 mediated early host defense against *C. rodentium* infection**

The above data suggested roles of IL-22 in both innate immunity and adaptive immunity. Therefore, we used recombination activating gene 2 deficient (Rag2$^{-/-}$) mice to critically examine the function of IL-22 in innate vs. adaptive immunity during *C. rodentium* infection. Rag2$^{-/-}$ mice gradually lost weight and eventually became moribund or died around day 30, due to their lack of B and T cells, and their consequent inability to mount anti-*C. rodentium* antibody responses (Fig. 13A). In contrast to p19$^{-/-}$ or IL-22$^{-/-}$ mice, none of the Rag2$^{-/-}$ mice lost more than 10% of their body weight or died during the first two weeks of infection. Furthermore, Rag$^{-/-}$ mice treated with anti-IL-22 mAb lost weight very rapidly (Fig. 13A), similar to WT mice treated with anti-IL-22 mAb (Fig. 2E). All Rag$^{-/-}$ mice treated with anti-IL-22 mAb became moribund or died around day 10 (Fig. 13). These data suggest that the IL-22 pathway is still active in Rag2$^{-/-}$ mice, and that IL-22 is essential to protect mice from death during the early phase of *C. rodentium* infection in the absence of adaptive immunity. These data also indicate that reduction in anti-*C. rodentium* IgG titers was insufficient cause for the morbidity and mortality observed in IL-2$^{-/-}$ mice following *C. rodentium* infection, as lack of antibody production in Rag2$^{-/-}$ mice alone did not cause rapid weight loss and early death following infection.

IL-22 production in Rag2$^{-/-}$ mice was comparable with that of WT mice following *C. rodentium* infection (Fig. 13B). In contrast, induction of IL-17A was significantly reduced in Rag2$^{-/-}$mice (Fig. 13B and C). T cells and B cells, therefore, were not the sources of IL-22 in this model. Immunohistochemical staining with an anti-IL-22 mAb (Fig. 15) detected IL-22 positive cells in the colon of WT mice infected with *C. rodentium*, but not in uninfected colon or colon from infected IL-22$^{-/-}$ mice. IL-22 positive cells primarily co-localized with CD11c$^+$ cell clusters in the colon of Rag2$^{-/-}$ mice (Fig. 13D), but not with F4/80, Gr-1, or DX5 positive cells (data not shown). In addition, IL-23 induced IL-22 production directly from CD11c$^+$ DCs *in vitro* (Fig. 13E). Taken together, our data demonstrate that DCs are one of the major sources of IL-22 production during *C. rodentium* infection, and that IL-23 can directly promote IL-22 production from DCs.

**Example 9: RegIII plays an important role during bacterial infection.**

Interestingly, the upregulation of RegIIIβ and RegIIIγ observed in wild type mice was completely abolished in IL-22$^{-/-}$ mice (Fig. 4C), as well as in p19$^{-/-}$ mice, (Fig. 16) post *C. rodentium* inoculation. RegIIIβ and RegIIIγ belong to a family of secreted C-type lectin proteins. We found that other family members, including RegI, RegII, RegIIIα, and RegIIIδ (Fig. 17), but not RegIV (data not shown), were also upregulated in *C. rodentium* infected colons, and that their induction was completely abolished in IL-22$^{-/-}$ mice. Exogenous mouse RegIIIγ fusion protein (rmRegIIIγ significantly protected IL-22$^{-/-}$ mice from the weight loss induced by the *C. rodentium* infection, and approximately 50% of rmRegIIIγ fusion protein treated animals survived the infection, whereas 100% of control treated IL-22$^{-/-}$ mice became moribund or died (Fig. 14A). These data support the hypothesis that Reg family proteins, such as RegIIIγ, mediate essential functions in controlling *C. rodentium* infection downstream of IL-22.

Finally, the presence of the IL-23/IL-22/Reg axis was also validated in a human system. Human IL-23 induces hIL-22 production from human DCs (Fig. 14B). Primary human colonic epithelial cells (Fig. 12B) and the human colonic epithelial cell lines, HT29 and HCT15, express IL-22R (Fig. 14C). *In vitro,* primary human colonic epithelial cells grew slowly, and gradually lost their expression of IL-22R during expansion (data not shown). Therefore, we used colonic epithelial cell lines to test their response to human IL-22. IL-22 induced STAT3 activation in these colonic epithelial cell lines (Fig. 14D), and both RegIIIβ and RegIIIγ were significantly induced by IL-22 (Fig. 14E). Importantly, human RegIIIγ fusion protein (rhRegIIIγ), like rmRegIIIγ fusion protein, also reduced the mortality of IL-22$^{-/-}$ mice, to 40% following *C. rodentium* infection, versus 100% mortality in control treated IL-22$^{-/-}$ mice (Fig. 18). In conclusion, our data imply that the IL-22 pathway may play an essential role in controlling bacterial infections, particularly A/E bacterial infections, in the human GI tract.

**Summary**

The present inventors demonstrate herein that IL-22 plays an indispensable role in early host defense against attaching and effacing (A/E) bacterial pathogens.

The data herein indicate that IL-22 protects the integrity of the intestinal epithelial barrier and prevents bacterial invasion with systemic spread through two mechanisms. First, IL-22 is involved in the elicitation of the early anti-bacterial IgG responses. Second, IL-22 is indispensable for the induction of anti-microbial lectins, such as RegIIIβ and RegIIIγ, from colonic epithelial cells during bacterial infection. The lack of either or both of these mechanisms may contribute to the compromised host defense response with increased systemic spread and mortality in IL-22$^{-/-}$ mice during *C. rodentium* infection.

While adaptive immune responses are essential for clearance of these pathogens (L. Bry, M. B. Brenner, J Immunol 172, 433 (January 1, 2004)), cytokines such as IL-22 that are produced by immune cells during the early stages of infection are also necessary for intestinal epithelial cells to elicit a full anti-microbial response and wound healing response in order to prevent systemic invasion of' pathogenic bacteria into the host. As shown herein, the induction of RegIIIβ and RegIIIγ also indicates that IL-22 may have broader functions in controlling various bacterial infections. The data further supports the role of Th17 cells and their effector cytokines in infectious diseases and autoimmune diseases. Finally, the present studies indicate that IL-22 and its downstream products, such as RegIIIβ and RegIIIγ, may be beneficial for the treatment of certain infectious diseases.

## Materials and Methods

### *Mice*

C57B1/6, IL-12p40$^{-/-}$, and IL-6$^{-/-}$ mice were purchased from the Jackson Laboratory. IL-22$^{-/-}$mice and IL-12p19$^{-/-}$ were generated as described before *(11, Fig.5*). IL-17RC$^{-/-}$ and IL-20Rβ$^{-/-}$mice were generated by Lexicon Pharmaceuticals (The Woodlands, TX) by using strategies as described (Fig.5 and Fig.8). Briefly, knockout mice were made by standard homologous recombination using depicted targeting vectors. Targeting vectors are electroporated into 129 strain ES cells and targeted clones are identified. Targeted clones are microinjected into host blastocysts to produce chimeras. Chimeras are bred with C57B1/6 animals to produce F1 heterozygotes. Heterozygotes are intercrossed to produce F2 wild type, heterozygote and homozygote cohorts. Mice used in these studies were genotyped by tail DNA via PCR using a pool of three primers. The primers used for wild-type allele amplification of IL-20Rβ$^{-/-}$ mice were 5'-GTG GAA GCT ACT TGA TGA GTA GGG-3' (p1) and 5'-AGA TGC GAA AAT GGA GAT TAA AAG-3' (p2), which yielded a 595 bp product. The primers used for mutant allele amplification of IL-20Rβ$^{-/-}$ mice were 5'-CTA CCC GTG ATA TTG CTG AAG AG-3' (p3) and p2, which yielded a 351 bp product. The primers used for wild-type allele amplification of IL-17RC$^{-/-}$ mice were 5'-GAG CCT GAA GAA GCT GGA AA-3' (P3) and 5'-CAA GTG TTG GCA GAG ATG GA-3' (P2), which yielded a 534 bp product. The primers used for mutant allele amplification of IL-17RC$^{-/-}$ mice were 5'-TCG CCT TCT TGA CGA GTT CT-3' (P1) and P2, which yielded a 404 bp product.

### *Bacteria strains and infection of mice*

6-8 weeks old mice were fasted for 8h before oral inoculation with 2x10$^{9}$ *C. rodentium* strain DBS100 (ATCC 51459; American Type Culture Collection) in a total volume of 200 μl per mouse. While fasting, animals had access to water. Inoculation and all subsequent manipulations were conducted in BL-2 biosafety cabinets. Animals were allowed access to food after inoculation. Bacteria were prepared by incubation with shaking at 37°C overnight in LB broth. The relative concentration of bacteria was assessed by measuring absorbance at OD600 and each inoculation culture was serially diluted and plated to confirm CFU administered.

## Tissue collection, histology and CFU counts

Control or infected mice were inoculated as described. Samples of whole blood, spleen, liver, mesenteric lymph node, and colon were removed under aseptic conditions. The colon was dissected to the anal canal, and the terminal 0.5-cm piece was used for CFU analysis. Proximal segments were fixed in 10% neutral buffered formalin. Sections were stained with H&E to evaluate tissue pathology. Spleen, liver, mesenteric lymph node, and colon were weighed and homogenized. Homogenates were serially diluted and plated in triplicates to MacConkey agar (Remel). *C. rodentium* colonies were identified as pink colonies. Colonies were counted after 24 h of incubation at 37°C to determine the $log_{10}$ CFU per gram of tissues.

## RNA isolation and real-time RT-PCR

Cell and tissue RNA were isolated by RNeasy Mini Kit (Qiagen) according to the manufacture's directions. Real-time RT-PCR was conducted on an ABI 7500 Real-Time PCR system (Applied Biosystems) with primers and probes using TaqMan one-step RT-PCR master mix reagents (Applied Biosystems). The sequences for primers and probes were as follows: mIL-22, forward, 5'-TCC GAG GAG TCA GTG CTA AA-3', reverse, 5'-AGA ACG TCT TCC AGG GTG AA-3', and probe, 5'-TGA GCA CCT GCT TCA TCA GGT AGC A-3' (FAM, TAMRA); mIL-17A, forward, 5'-GCT CCA GAA GGC CCT CAG A-3', reverse, 5'-CTT TCC CTC CGC ATT GAC A-3', and probe, 5'- ACC TCA ACC GTT CCA CGT CAC-3' (FAM, TAMRA); mouse ribosomal housekeeping gene RPL-19, forward, 5'-GCA TCC TCA TGG AGC ACA T-3', reverse, 5'-CTG GTC AGC CAG GAG CTT-3', and probe, 5'-CTT GCG GGC CTT GTC TGC CTT-3' (FAM, TAMRA); mIL-19, forward, 5'-AGC CTG GAT TGA CAG GAA TC-3', reverse, 5'-GAT AAT CAG ACG AGG CGT TTC-3', and probe, 5'-TCT GGA AAC TCC TGC AGC CTG ACA C-3' (FAM, TAMRA); mIL-20, forward, 5'-TTT GGG AGA ACT AGG CAT TCT T-3', reverse, 5'-TCT TGG ACA GGA GTG TTC TCA-3', and probe, 5'-CAG CCT CTC CAC TTT CAT CTA TAG CAT CTC C-3' (FAM,TAMRA); mIL-24, forward, 5'-GCT CTC CAT GCC ATT TCA A-3', reverse, 5'-TGG CCA AGG GTC TGA AGT-3', and probe, 5'-TGT ACA TCC CTG CTG TCC TCA AGG C-3' (FAM, TAMRA); mIL-6, forward, 5'-TCC AAT GCT CTC CTA ACA GAT AAG-3', reverse, 5'-CAA GAT GAA TTG GAT GGT CTT G-3', and probe, 5'-TCC TTA GCC ACT CCT TCT GTG ACT CCA-3' (FAM, TAMRA); mS100A8, forward, 5'-TGT CCT CAG TTT GTG CAG AAT ATA AA-3', reverse, 5'-TCA CCA TCG CAA GGA ACT CC-3', and probe 5'-CGA AAA CTT GTT CAG AGA ATT GGA CAT CAA TAG TGA-3' (FAM, TAMRA); mS100A9, forward, 5'-GGT GGA AGC ACA GTT GGC A-3', reverse, 5'-GTG TCC AGG TCC TCC ATG ATC-3', and probe, 5'-TGA AGA AAG AGA AGA GAA ATG AAG CCC TCA TAA ATG-3' (FAM, TAMRA); mRegIIIγ, forward, 5'-ATG GCT CCT ATT GCT ATG CC-3', reverse, 5'-GAT GTC CTG AGG GCC TCT T-3', and probe, 5'-TGG CAG GCC ATA TCT GCA TCA TAC C-3' (FAM, TAMRA); mPAP/HIP/RegIIIβ, forward, 5'- ATG GCT CCT ACT GCT ATG CC-3', reverse, 5'- GTG TCC TCC AGG CCT CTT T-3', and probe, 5'-TGA TGC AGA ACT GGC CTG CCA-3' (FAM, TAMRA); mIL-12p40, forward, 5'-ACA TCT ACC GAA GTC CAA TGC A-3', reverse, 5'-GGA ATT GTA ATA GCG ATC CTG AGC-3', and probe, 5'-TGC ACG CAG ACA TTC CCG CCT-3' (FAM, TAMRA); mIL-23p19, forward, 5'-GGT GGC TCA GGG AAA TGT-3', reverse, 5'-GAC AGA GCA GGC AGG TAC AG-3', and probe, 5'-CAG ATG CAC AGT ACT CCA GAC AGC AGC-3' (FAM, TAMRA); mIL-20Rβ, forward, 5'-CAG GTG CTT CCA GTC CGTCT-3', reverse, 5'-CTC TCC TGG AAT CCC CAA AGT-3', and probe, 5'-CAG CAC AGA TGC CAA CGG CCT CAT-3' (FAM, TAMRA); mIL-20Rα, forward, 5'-CTG GCC GCT TCG GGA CGC-3', reverse, 5'-AAC CAC AGA AGA CAC AAG GAA CTG-3', and probe, 5'-TCT GCT GCT GGC CGC TTC GG-3' (FAM, TAMRA); mIL-22R, forward, 5'-GCT GGA CTC CCT TGT GTG T-3', reverse, 5'-CAC ATG GCC TCA GTC TCA A-3', and probe, 5'-CGC GGG ACC CTC ATC CTT TAG-3' (FAM, TAMRA); mIL-10Rβ, forward, 5'-TCC ACA GCA CCT GAA GGA GTT-3', reverse, 5'-GGA GGG AAG GAG AAC AGC AGA-3', and probe, 5'-TGG GCC ACC CCC ATC ACA GC-3' (FAM, TAMRA). Reactions were run in duplicates and samples were normalized to the control housekeeping gene RPL-19 and reported according to the $\Delta\Delta Ct$ method: $\Delta\Delta Ct = \Delta Ct_{sample}$ - $\Delta Ct_{reference}$.

### *Ig ELISA*

Analyses were performed on serum from collected whole blood as previously described (*10*). Briefly, ELISA plates (Nunc) were coated with heat-killed *C. rodentium* or with a goat anti-mouse Ig capture Ab diluted 1/1000 in PBS (SouthernBiotech). Coated plates were washed in PBS plus 0.05% Tween 20, blocked for 1 h with 300 μl of blocking buffer (PBS + 0.5 % BSA + 10 PPM Proclin), and washed before addition of serially diluted standards (mouse monoclonal IgA, IgG, IgG3, and IgM from SouthernBiotech; IgG1, IgG2a, and IgG2b isotypes from Sigma-Aldrich; mouse IgG2c obtained from Bethyl laboratories) or unknowns. Samples were incubated for 4 hours at room temperature. Plates were washed five times and the Ig isotypes were detected with goat anti-mouse IgA, IgM, IgG, IgG1, IgG2a, IgG2b, IgG2c, and IgG3 (SouthernBiotech) conjugated to horseradish peroxidase (HRP), diluted 1/4,000 in assay diluent (PBS + 0.5 % BSA + 0.05 % Tween 20 + 10 PPM Proclin, pH 7.4), and incubated for 1 hour at room temperature. After washing, TMB peroxidase substrate was added to each well and allowed to develop for 15 minutes, then stop solution (1 M Phosphoric acid) were added to each well. Absorbance was read at 450 nm in a Molecular Devices (Sunnyvale, CA) plate reader at $OD_{450}$.

### *In vitro colon culture*

Colons were removed from C57Bl/6 mice. After cleaning with cold PBS, colons were cut longitudinally. Colons were placed in a 100mm Petri dish with 10ml HBSS (Mediatech) buffer containing 2.5 μg/ml of Fungizone-Amphotericin B, 10 μg/ml Gentamicin, 100 U/ml Penicillin and 100 μg/ml Streptomycin (all from GIBCO, Invitrogen). Colons were gently scraped to remove mucus at the edge of the Petri dish and were transferred to a new Petri dish with fresh HBSS buffer. Colons were cut into 1-2 mm pieces and transferred to a 24-well plate with 50mg colons/1ml/well in RPMI buffer containing 10% heat inactivated FCS (HyClone), 2.5 μg/ml of Fungizone-Amphotericin B, 10 μg/ml Gentamicin, 2 mM L-Glutamine, 100 U/ml Penicillin and 100 μg/ml Streptomycin. 10 μg/ml of IL-22 (R & D systems) were added to the culture and incubated in 37 °C for 24 hours.

***Microarray analysis***

Quantity and quality of total RNA samples was determined using an ND-1000 spectrophotometer (Nanodrop Technologies) and Bioanalyzer 2100 (Agilent Technologies), respectively. The method for preparation of Cy-dye labeled cRNA and array hybridization was provided by Agilent Technologies. Briefly, total RNA sample was converted to double-stranded cDNA and then to Cy-dye labeled cRNA using Agilent's Low RNA Input Fluorescent Linear Amplification Kit. The labeled cRNA was purified using RNeasy mini kit (Qiagen). cRNA yield and Cy-dye incorporation was determined using ND-1000 spectrophotometer. 750 ng of the labeled cRNA was fragmented and hybridized to the Agilent's Whole Mouse Genome array as described in manufacturer's In situ Hybridization kit-plus. All samples were labeled with Cy5 and hybridized against Cy3 labeled universal mouse reference (Stratagene). Following hybridization, the arrays were washed, dried and scanned on Agilent's DNA microarray scanner. Agilent's Feature Extraction software 8.5 was used to analyze acquired array images. For microarray data clustering (Fig. 20), expression data was processed to Agilent log-ratio data by standard methods. Selected genes were clustered by iterative agglomeration of vectors most highly linked by Pearson correlation coefficient, with data for agglomerated vectors summarized by average linkage.

***In vitro mouse tail tip fibroblast culture and stimulation***

To establish tail tip fibroblasts (TTFs), the tails from IL-17RC$^{-/-}$ adult mice and wild type littermates were peeled, minced into 1 cm pieces, placed on culture dishes, and incubated in high glucose DMEM (containing 10% FCS, 2 nm glutamine, 100 U/ml Penicillin and 100 μg/ml Streptomycin) for 5 days. Cells that migrated out of the graft pieces were transferred to new plates (passage 2) and maintained in the same media. We used TTFs at passage 3 for stimulation experiments. TTFs were seeded into 24-well plate at a density of 1.2x10$^5$ per well. Twenty four hours after seeding, recombinant murine IL-17A and IL-17F (R&D Systems) were added to the culture medium at various concentrations. Cell culture supernatant was harvested 24 hours after addition of cytokines and levels of murine IL-6 was measured by enzyme linked immunosorbent assay (ELISA) by mouse IL-6 ELISA set (BD Biosciences) following manufacturer's instructions.

***Blockage of murine IL-22 in vivo***

Blocking anti-mouse IL-22 (Clone 8E11, isotype mouse IgG1) mAb (*11*) was intraperitoneally injected before (Day 0) or 8 days after (Day 8) *C. rodentium* infection at a dose of 150 μg/mouse every other day. Certain control group also received isotype control IgG1 mAb.

***Statistics***

Statistical significance was calculated by one -way or two-way *ANOVA* using Prism software (GraphPad). All *p* values ≤ 0.05 are considered significant, and are indicated in the text. Unless otherwise specified, all studies for which data are presented are representative of at least two independent experiments.

Although the foregoing invention has been described in some detail by way of illustration and example for purposes of clarity of understanding, the descriptions and examples should not be construed as limiting the scope of the invention.

**Example 10: The LT pathway is mediated by IL-22 during *Citrobacer rodentium* infection.**

To help determine if IL-22 is important for the mortality caused by LT blockade, we performed a rescue experiment in which we expressed IL-22 in the mouse at the same time of LTbR-Fc treatment. The method we used for IL-22 expression was hydrodynamic tail vein delivery of plasmid DNA encoding mouse IL-22. Human LTbR-Ig was constructed as follows: human LTbR encompassing the extracellular domain (position 1 through position 224; SEQ ID NO:57) was cloned into a modified pRK5 expression vector encoding the human IgG1 Fc region (SEQ ID NO:58) downstream of the LTbR sequence. Proteins were overexpressed in CHO cells and purified by protein A affinity chromatography. Murine LTbR.1g was constructed as follows: murine LTbR encompassing the extracellular domain (position 1 through position 222; SEQ ID NO:59) was cloned into a modified pRK5 expression vector encoding the murine IgG2a Fc region (SEQ ID NO:60) downstream of the LTbR sequence.

In Figure 21, we find that LTbR-Fc produces a similar weight loss curve (Figure 21 right panel) and death curve (Figure 21 left panel) to IL-22 blockade which led us to examine the relationship between LT and IL-22. *C. rodentium* infection leads to early expression of IL-22 in the colon.

Fig. 21 shows the percent survival of mice inoculated with *Citrobacter rodentium.* 6-8 week old Balb/c mice were fasted for 8h before oral inoculation with 2x109 *C. rodentium* strain DBS100 (ATCC 51459; American Type Culture Collection) in a total volume of 200 μl per mouse. While fasting, animals had access to water. Inoculation and all subsequent manipulations were conducted in BL-2 biosafety cabinets. Animals were allowed access to food after inoculation. Bacteria were prepared by incubation with shaking at 37°C overnight in LB broth. The relative concentration of bacteria was assessed by measuring absorbance at OD600 and each inoculation culture was serially diluted and plated to confirm CFU administered. On the day of inoculation, mice were also injected with 150ug of anti-gp120 mAb, anti-IL-22 8E11 mAb, or LTbR-Fc 3 times per week.

**LT pathway regulate multiple upstream aspects that important for IL-22 production.** Figure 22 provides data on the LT pathway after infection with *C. rodentium.* A, C, E. Colons were harvested at different timepoints after infection with *C. rodentium.* Mice were injected with 150ug anti-gp120 or LTbR-Fc every other day. RNA was extracted using Qiagen RNeasy Kit. Taqman analysis was performed to determine expression of IL-22, RegIIg, p19, or p40 relative to the day 0 timepoint. B. On day 4 after infection, colons were collected. After cleaning with cold PBS, colons were cut longitudinally. Colons were placed in a 100 mm Petri dish with 10 ml HBSS (Mediatech) buffer containing 2.5 μg/ml of Fungizone-Amphotericin B, 10 μg/ml Gentamicin, 100 U/ml Penicillin and 100 μg/ml Streptomycin (all from GIBCO, Invitrogen). Colons were gently scraped to remove mucus at the edge of the Petri dish and were transferred to a new Petri dish with fresh HBSS buffer. Colons were cut into 1-2 mm pieces and transferred to a 24-well plate with 50 mg colons/1 ml/well in RPMI buffer containing 10 % heat inactivated FCS (HyClone), 2.5 μg/ml of Fungizone-Amphotericin B, 10 μg/ml Gentamicin, 2 mM L-Glutamine, 100 U/ml Penicillin and 100 μg/ml Streptomycin. 10 ng/ml of rmIL-22 (R & D systems) were added to the culture and incubated in 37°C for 24 hours. Supernatants were collected for an IL-22 ELISA. D. Day 6 colon lamina propria cells. DC determined by CD11c+ and MHC II+. Colons were harvested and flushed with HBSS without calcium and magnesium (Invitrogen) with 2% FBS and 10mM HEPES. Colons were cut longitudinally, and then sectioned into 2-4 cm pieces, and the pieces were transferred to a 10 cm dish with HBSS without calcium and magnesium, 2% FBS, 1mM EDTA, 10mM HEPES, and 1mM DTT (Sigma-Aldrich). IEL fractions were collected and discarded after a 45 minute incubation at 37°C while shaking at 200 rpm. For LPMCs isolation, the remaining epithelial layer was peeled off and the colon pieces were diced and placed into RPMI containing 10 % FCS, 20mM HEPES, and 0.5 mg/ml collagenase/dispase (Roche Diagnostics). Colon pieces were incubated for one hour at 37°C while shaking. Isolated epithelial cells were washed and used for FACS analysis.

In Figure 22, we find that LTbR-Fc blocked the induction of IL-22 as well as RegIIIg which has been shown to be induced by IL-22 (Figure 22A-C). Dendritic cells have previously been shown to produce IL-22 and we find a slight reduction of DC numbers in the lamina propria of the colon 6 days after infection (Figure 22D). The decrease in IL-22 caused by LTbR-Fc is most likely due the loss of IL-23, since both p19 and p40 expression is inhibited after LTbR-Fc treatment during infection (Figure 22E).

**IL-22 partially rescues the defects seen in LTbR treated mice.** Figure 23 provides data concerning the effect of IL-22 on LTbR treated mice. A. Test of expression of IL-22 in serum and RegIIIg in colon after tail vein injection of IL-22 plasmid. B. Rescue of LTbRFc effects with IL-22 plasmid.

On day-1, animals were weighed and grouped, extra mice were euthanized. After weighing, all animals were fasted 14 h. The next morning (day 0), all mice were orally inoculated with 2-4 X 10e9 CFU of C. rodentium in 200 ul PBS. 150ug control mAb or Fc fusion protein was injected i.p. in 200ul PBS three times per week for two weeks starting on the same day as bacteria inoculation. Food was replaced back by investigators after inoculation. Six hours later plasmid DNA was injected by tail vein. Tail vein injection experiments: 1) DNA construct (pRK vector or pRK-mIL-22) was diluted in Ringer's to a concentration to yield a final dose of 10 micrograms/mouse/injection. 2) Each mouse was injected intravenously in the tail vein with approximately 1.6ml of the solution containing DNA in Ringer's. 3) Doses were administered as a bolus intravenous injection (tail vein) over a period of 4-5 seconds (8 seconds maximum) for maximum DNA uptake. Mice were restrained without anesthesia in a conical acrylic restrainer with a heating element to increase body temperature and dilate blood vessels. 4) Disposable sterile syringes were used for each animal. Animals were continuously monitored until they are clinically normal. 5) Animals were observed for any adverse clinical signs for at least 20 minutes post dose. If animals were not clinically normal by 1 hour post dose, they were euthanized or they were monitored until they were clinically normal. Moribund animals were euthanized. All manipulations were performed in BL-2 biosafety cabinets. During infection, moribund animals or those showing unalleviated distress or rectal prolapse were euthanized.

The mice were monitored for 4 weeks everyday. Between day 5 to day 17 when LTbR-Fc treated mice might become moribund, the mice were monitored twice per day including weekends. Fecal pellets were collected every week to measure CFU of C. rodentium. Mice were weighed once per week during the study. If mice exhibited a weight loss of 15% or more, they were weighed daily. If the weight loss exceeds 20%, the mice were euthanized. At the end of the study, all mice were euthanized and spleen, and colon were collected for histology, RNA or FACS analysis.

As shown in Figure 23A, we can detect expression of IL-22 in the mouse serum beginning at 2 hours post-injection, with expression declining at 72 hours. We can also detect expression of RegIIIg in the colon, suggesting that active IL-22 can act on the colon when expressed in this manner. IL-22 could partially rescue mortality and weight loss induced by LTb-Fc treatment during infection (Figure 23B).

**Treatment of mice with IL-22 mAb (8E11).** Figure 24 shows data demonstrating that treatment with IL-22 mAb 8E11 leads to reduced colon follicles, compromised B/T cell organization, and reduced DC, T cell, and B cell numbers in the colon. A. Six days after infection, colons were harvested and cut longitudinally. After a 30 minute incubation in HBSS without calcium and magnesium, 2% FBS, 1mM EDTA, 10mM HEPES, and 1mM DTT, colons were gently scraped to remove epithelial cells. Follicles were identified as white, round masses. There were five mice per group and each colon was counted and plotted as total follicles found or total follicles greater than 1mm found. B. Six days after infection, colons were flushed with cold PBS and quick frozen in OCT. Six micron sections were cut, dried, then fixed in acetone. Sections were blocked with 10% serum, the incubated with anti-CD5 FITC and anti-B220 APC at 10ug/ml each. Images were capture on a NIKON BX61 microscope. C. Six days after infection colon lamina propria cells were isolated at described above. FAC analysis was performed to determine the number of dendritic cells, CD3 T cells, and B cells after 8E11 treatment.

As shown in Figure 24, we treated mice with either IL-22 blocking antibody or LTbR-Fc and counted lymphoid follicles in the colon after six days post infection in order to determine if IL-22 could have a role in formation of colon lymphoid structures. We found a decrease in follicles greater than 1mm, suggesting IL-22 and LT could be important for the increase in follicle size after infection (Figure 24A). Histological analysis shows that blocking IL-22 disrupted the T and B cell zones of the follicle while LT blockade had a similar effect (Figure 24B). We next determined whether blockade of IL-22 leads to a change in cell numbers in the colon lamina propria. We found that IL-22 blockade led to decreases in DC, T cell, and B cell numbers during infection. In conclusion, IL-22 appears to be important for lymphoid follicle formation and may be an important downstream component of the lymphotoxin pathway in the colon.

Sequence Listing

<110> Genentech, Inc.

<120> Compositions and Methods for Treatment of Microbial Disorders

<130> SMW/FP6829519

<140> EP
<141> 2008-11-07

<150> EP08848478.7
<151> 2008-11-07

<150> PCT/US2008/082890
<151> 2008-11-07

<150> 60/986,170
<151> 2007-11-07

<150> 61/013,620
<151> 2007-12-13

<150> 61/015,620
<151> 2007-12-20

<160> 61

<210> 1
<211> 1131
<212> DNA
<213> Homo sapiens

<400> 1

cattctgccc tcgagcccac cgggaacgaa agagaagctc tatctcccct 50
ccaggagccc agctatgaac tccttctcca caagcgcctt cggtccagtt 100
gccttctccc tggggctgct cctggtgttg cctgctgcct tccctgcccc 150
agtaccccca ggagaagatt ccaaagatgt agccgcccca cacagacagc 200
cactcacctc ttcagaacga attgacaaac aaattcggta catcctcgac 250
ggcatctcag ccctgagaaa ggagacatgt aacaagagta acatgtgtga 300
aagcagcaaa gaggcactgg cagaaaacaa cctgaacctt ccaaagatgg 350
ctgaaaaaga tggatgcttc caatctggat tcaatgagga gacttgcctg 400
gtgaaaatca tcactggtct tttggagttt gaggtatacc tagagtacct 450
ccagaacaga tttgagagta gtgaggaaca agccagagct gtgcagatga 500
gtacaaaagt cctgatccag ttcctgcaga aaaaggcaaa gaatctagat 550
gcaataacca cccctgaccc aaccacaaat gccagcctgc tgacgaagct 600
gcaggcacag aaccagtggc tgcaggacat gacaactcat ctcattctgc 650
gcagctttaa ggagttcctg cagtccagcc tgagggctct tcggcaaatg 700
tagcatgggc acctcagatt gttgttgtta atgggcattc cttcttctgg 750
tcagaaacct gtccactggg cacagaactt atgttgttct ctatggagaa 800

ctaaaagtat gagcgttagg acactatttt aattattttt aatttattaa 850
tatttaaata tgtgaagctg agttaattta tgtaagtcat atttatattt 900
ttaagaagta ccacttgaaa cattttatgt attagttttg aaataataat 950
ggaaagtggc tatgcagttt gaatatcctt tgtttcagag ccagatcatt 1000
tcttggaaag tgtaggctta cctcaaataa atggctaact tatacatatt 1050
tttaaagaaa tatttatatt gtatttatat aatgtataaa tggtttttat 1100
accaataaat ggcattttaa aaaattcagc a 1131

<210> 2
<211> 212
<212> PRT
<213> Homo sapiens

<400> 2

```
Met Asn Ser Phe Ser Thr Ser Ala Phe Gly Pro Val Ala Phe Ser
1               5                   10                  15

Leu Gly Leu Leu Leu Val Leu Pro Ala Ala Phe Pro Ala Pro Val
                20                  25                  30

Pro Pro Gly Glu Asp Ser Lys Asp Val Ala Ala Pro His Arg Gln
                35                  40                  45

Pro Leu Thr Ser Ser Glu Arg Ile Asp Lys Gln Ile Arg Tyr Ile
                50                  55                  60

Leu Asp Gly Ile Ser Ala Leu Arg Lys Glu Thr Cys Asn Lys Ser
                65                  70                  75

Asn Met Cys Glu Ser Ser Lys Glu Ala Leu Ala Glu Asn Asn Leu
                80                  85                  90

Asn Leu Pro Lys Met Ala Glu Lys Asp Gly Cys Phe Gln Ser Gly
                95                  100                 105

Phe Asn Glu Glu Thr Cys Leu Val Lys Ile Ile Thr Gly Leu Leu
                110                 115                 120

Glu Phe Glu Val Tyr Leu Glu Tyr Leu Gln Asn Arg Phe Glu Ser
                125                 130                 135

Ser Glu Glu Gln Ala Arg Ala Val Gln Met Ser Thr Lys Val Leu
                140                 145                 150

Ile Gln Phe Leu Gln Lys Lys Ala Lys Asn Leu Asp Ala Ile Thr
                155                 160                 165

Thr Pro Asp Pro Thr Thr Asn Ala Ser Leu Leu Thr Lys Leu Gln
                170                 175                 180

Ala Gln Asn Gln Trp Leu Gln Asp Met Thr Thr His Leu Ile Leu
                185                 190                 195

Arg Ser Phe Lys Glu Phe Leu Gln Ser Ser Leu Arg Ala Leu Arg
                200                 205                 210

Gln Met
```

<210> 3
<211> 2347
<212> DNA
<213> Homo sapiens

<400> 3

ctgtttcagg gccattggac tctccgtcct gcccagagca agatgtgtca 50
ccagcagttg gtcatctctt ggttttccct ggtttttctg gcatctcccc 100
tcgtggccat atgggaactg aagaaagatg tttatgtcgt agaattggat 150
tggtatccgg atgcccctgg agaaatggtg gtcctcacct gtgacacccc 200
tgaagaagat ggtatcacct ggaccttgga ccagagcagt gaggtcttag 250
gctctggcaa aaccctgacc atccaagtca aagagtttgg agatgctggc 300
cagtacacct gtcacaaagg aggcgaggtt ctaagccatt cgctcctgct 350
gcttcacaaa aaggaagatg gaatttggtc cactgatatt ttaaaggacc 400
agaaagaacc caaaaataag acctttctaa gatgcgaggc caagaattat 450
tctggacgtt tcacctgctg gtggctgacg acaatcagta ctgatttgac 500
attcagtgtc aaaagcagca gaggctcttc tgacccccaa ggggtgacgt 550
gcggagctgc tacactctct gcagagagag tcagagggga caacaaggag 600
tatgagtact cagtggagtg ccaggaggac agtgcctgcc cagctgctga 650
ggagagtctg cccattgagg tcatggtgga tgccgttcac aagctcaagt 700
atgaaaacta caccagcagc ttcttcatca gggacatcat caaacctgac 750
ccacccaaga acttgcagct gaagccatta aagaattctc ggcaggtgga 800
ggtcagctgg gagtaccctg acacctggag tactccacat tcctacttct 850
ccctgacatt ctgcgttcag gtccagggca agagcaagag agaaaagaaa 900
gatagagtct tcacggacaa gacctcagcc acggtcatct gccgcaaaaa 950
tgccagcatt agcgtgcggg cccaggaccg ctactatagc tcatcttgga 1000
gcgaatgggc atctgtgccc tgcagttagg ttctgatcca ggatgaaaat 1050
ttggaggaaa agtggaagat attaagcaaa atgtttaaag acacaacgga 1100
atagacccaa aaagataatt tctatctgat ttgctttaaa acgttttttt 1150
aggatcacaa tgatatcttt gctgtatttg tatagttaga tgctaaatgc 1200
tcattgaaac aatcagctaa tttatgtata gattttccag ctctcaagtt 1250
gccatgggcc ttcatgctat ttaaatattt aagtaattta tgtatttatt 1300
agtatattac tgttatttaa cgtttgtctg ccaggatgta tggaatgttt 1350
catactctta tgacctgatc catcaggatc agtccctatt atgcaaaatg 1400
tgaatttaat tttatttgta ctgacaactt ttcaagcaag gctgcaagta 1450

```
catcagtttt atgacaatca ggaagaatgc agtgttctga taccagtgcc 1500
atcatacact tgtgatggat gggaacgcaa gagatactta catggaaacc 1550
tgacaatgca aacctgttga gaagatccag gagaacaaga tgctagttcc 1600
catgtctgtg aagacttcct ggagatggtg ttgataaagc aatttagggc 1650
cacttacact tctaagcaag tttaatcttt ggatgcctga attttaaaag 1700
ggctagaaaa aaatgattga ccagcctggg aaacataaca agaccccgtc 1750
tctacaaaaa aaatttaaaa ttagccaggc gtggtggctc atgcttgtgg 1800
tcccagctgt tcaggaggat gaggcaggag gatctcttga gcccaggagg 1850
tcaaggctat ggtgagccgt gattgtgcca ctgcatacca gcctaggtga 1900
cagaatgaga ccctgtctca aaaaaaaaaa tgattgaaat taaaattcag 1950
ctttagcttc catggcagtc ctcaccccca cctctctaaa agacacagga 2000
ggatgacaca gaaacaccgt aagtgtctgg aaggcaaaaa gatcttaaga 2050
ttcaagagag aggacaagta gttatggcta aggacatgaa attgtcagaa 2100
tggcaggtgg cttcttaaca gccctgtgag aagcagacag atgcaaagaa 2150
aatctggaat ccctttctca ttagcatgaa tgaacctgat acacaattat 2200
gaccagaaaa tatggctcca tgaaggtgct acttttaagt aatgtatgtg 2250
cgctctgtaa agtgattaca tttgtttcct gtttgtttat ttatttattt 2300
atttttgcat tctgaggctg aactaataaa aactcttctt  tgtaatc 2347
```

<210> 4
<211> 328
<212> PRT
<213> Homo sapiens

<400> 4

```
Met Cys His Gln Gln Leu Val Ile Ser Trp Phe Ser Leu Val Phe
 1               5                  10                  15

Leu Ala Ser Pro Leu Val Ala Ile Trp Glu Leu Lys Lys Asp Val
                20                  25                  30

Tyr Val Val Glu Leu Asp Trp Tyr Pro Asp Ala Pro Gly Glu Met
                35                  40                  45

Val Val Leu Thr Cys Asp Thr Pro Glu Glu Asp Gly Ile Thr Trp
                50                  55                  60

Thr Leu Asp Gln Ser Ser Glu Val Leu Gly Ser Gly Lys Thr Leu
                65                  70                  75

Thr Ile Gln Val Lys Glu Phe Gly Asp Ala Gly Gln Tyr Thr Cys
                80                  85                  90

His Lys Gly Gly Glu Val Leu Ser His Ser Leu Leu Leu Leu His
                95                  100                 105

Lys Lys Glu Asp Gly Ile Trp Ser Thr Asp Ile Leu Lys Asp Gln
                110                 115                 120
```

```
Lys Glu Pro Lys Asn Lys Thr Phe Leu Arg Cys Glu Ala Lys Asn
                125                 130                 135

Tyr Ser Gly Arg Phe Thr Cys Trp Trp Leu Thr Thr Ile Ser Thr
                140                 145                 150

Asp Leu Thr Phe Ser Val Lys Ser Ser Arg Gly Ser Ser Asp Pro
                155                 160                 165

Gln Gly Val Thr Cys Gly Ala Ala Thr Leu Ser Ala Glu Arg Val
                170                 175                 180

Arg Gly Asp Asn Lys Glu Tyr Glu Tyr Ser Val Glu Cys Gln Glu
                185                 190                 195

Asp Ser Ala Cys Pro Ala Ala Glu Glu Ser Leu Pro Ile Glu Val
                200                 205                 210

Met Val Asp Ala Val His Lys Leu Lys Tyr Glu Asn Tyr Thr Ser
                215                 220                 225

Ser Phe Phe Ile Arg Asp Ile Ile Lys Pro Asp Pro Pro Lys Asn
                230                 235                 240

Leu Gln Leu Lys Pro Leu Lys Asn Ser Arg Gln Val Glu Val Ser
                245                 250                 255

Trp Glu Tyr Pro Asp Thr Trp Ser Thr Pro His Ser Tyr Phe Ser
                260                 265                 270

Leu Thr Phe Cys Val Gln Val Gln Gly Lys Ser Lys Arg Glu Lys
                275                 280                 285

Lys Asp Arg Val Phe Thr Asp Lys Thr Ser Ala Thr Val Ile Cys
                290                 295                 300

Arg Lys Asn Ala Ser Ile Ser Val Arg Ala Gln Asp Arg Tyr Tyr
                305                 310                 315

Ser Ser Ser Trp Ser Glu Trp Ala Ser Val Pro Cys Ser
                320                 325
```

<210> 5
<211> 1145
<212> DNA
<213> Homo sapiens

<400> 5

attctctccc cagcttgctg agccctttgc tcccctggcg actgcctgga 50
cagtcagcaa ggaattgtct cccagtgcat tttgccctcc tggctgccaa 100
ctctggctgc taaagcggct gccacctgct gcagtctaca cagcttcggg 150
aagaggaaag gaacctcaga ccttccagat cgcttcctct cgcaacaaac 200
tatttgtcgc aggaataaag atggctgctg aaccagtaga agacaattgc 250
atcaactttg tggcaatgaa atttattgac aatacgcttt actttatagc 300
tgaagatgat gaaaacctgg aatcagatta ctttggcaag cttgaatcta 350
aattatcagt cataagaaat ttgaatgacc aagttctctt cattgaccaa 400
ggaaatcggc ctctatttga agatatgact gattctgact gtagagataa 450
tgcaccccgg accatattta ttataagtat gtataaagat agccagccta 500
gaggtatggc tgtaactatc tctgtgaagt gtgagaaaat ttcaactctc 550
tcctgtgaga acaaaattat ttcctttaag gaaatgaatc ctcctgataa 600
catcaaggat acaaaaagtg acatcatatt ctttcagaga agtgtcccag 650
gacatgataa taagatgcaa tttgaatctt catcatacga aggatacttt 700
ctagcttgtg aaaaagagag agaccttttt aaactcattt tgaaaaaaga 750
ggatgaattg gggatagat ctataatgtt cactgttcaa aacgaagact 800
agctattaaa atttcatgcc gggcgcagtg gctcacgcct gtaatcccag 850
ccctttggga ggctgaggcg ggcagatcac cagaggtcag gtgttcaaga 900
ccagcctgac caacatggtg aaacctcatc tctactaaaa atacaaaaaa 950
ttagctgagt gtagtgacgc atgccctcaa tcccagctac tcaagaggct 1000
gaggcaggag aatcacttgc actccggagg tagaggttgt ggtgagccga 1050
gattgcacca ttgcgctcta gcctgggcaa caacagcaaa actccatctc 1100
aaaaaataaa ataaataaat aaacaaataa aaaattcata atgtg 1145

<210> 6
<211> 193
<212> PRT
<213> Homo sapiens

<400> 6

```
Met Ala Ala Glu Pro Val Glu Asp Asn Cys Ile Asn Phe Val Ala
1               5                   10                  15

Met Lys Phe Ile Asp Asn Thr Leu Tyr Phe Ile Ala Glu Asp Asp
                20                  25                  30

Glu Asn Leu Glu Ser Asp Tyr Phe Gly Lys Leu Glu Ser Lys Leu
                35                  40                  45

Ser Val Ile Arg Asn Leu Asn Asp Gln Val Leu Phe Ile Asp Gln
                50                  55                  60

Gly Asn Arg Pro Leu Phe Glu Asp Met Thr Asp Ser Asp Cys Arg
                65                  70                  75

Asp Asn Ala Pro Arg Thr Ile Phe Ile Ile Ser Met Tyr Lys Asp
                80                  85                  90

Ser Gln Pro Arg Gly Met Ala Val Thr Ile Ser Val Lys Cys Glu
                95                  100                 105

Lys Ile Ser Thr Leu Ser Cys Glu Asn Lys Ile Ile Ser Phe Lys
                110                 115                 120

Glu Met Asn Pro Pro Asp Asn Ile Lys Asp Thr Lys Ser Asp Ile
                125                 130                 135

Ile Phe Phe Gln Arg Ser Val Pro Gly His Asp Asn Lys Met Gln
                140                 145                 150

Phe Glu Ser Ser Ser Tyr Glu Gly Tyr Phe Leu Ala Cys Glu Lys
                155                 160                 165

Glu Arg Asp Leu Phe Lys Leu Ile Leu Lys Lys Glu Asp Glu Leu
                170                 175                 180

Gly Asp Arg Ser Ile Met Phe Thr Val Gln Asn Glu Asp
                185                 190
```

<210> 7
<211> 1147
<212> DNA
<213> Homo sapiens

<400> 7

cgaccaggtt ctccttcccc agtcaccagt tgctcgagtt agaattgtct 50
gcaatggccg ccctgcagaa atctgtgagc tctttcctta tggggaccct 100
ggccaccagc tgcctccttc tcttggccct cttggtacag ggaggagcag 150
ctgcgcccat cagctcccac tgcaggcttg acaagtccaa cttccagcag 200
ccctatatca ccaaccgcac cttcatgctg gctaaggagg ctagcttggc 250
tgataacaac acagacgttc gtctcattgg ggagaaactg ttccacggag 300
tcagtatgag tgagcgctgc tatctgatga agcaggtgct gaacttcacc 350
cttgaagaag tgctgttccc tcaatctgat aggttccagc cttatatgca 400
ggaggtggtg cccttcctgg ccaggctcag caacaggcta agcacatgtc 450
atattgaagg tgatgacctg catatccaga ggaatgtgca aaagctgaag 500
gacacagtga aaaagcttgg agagagtgga gagatcaaag caattggaga 550
actggatttg ctgtttatgt ctctgagaaa tgcctgcatt tgaccagagc 600
aaagctgaaa aatgaataac taacccctt tccctgctag aaataacaat 650
tagatgcccc aaagcgattt tttttaacca aaaggaagat gggaagccaa 700
actccatcat gatgggtgga ttccaaatga acccctgcgt tagttacaaa 750
ggaaaccaat gccacttttg tttataagac cagaaggtag actttctaag 800
catagatatt tattgataac atttcattgt aactggtgtt ctatacacag 850
aaaacaattt atttttaaa taattgtctt tttccataaa aaagattact 900
ttccattcct ttaggggaaa aaacccctaa atagcttcat gtttccataa 950
tcagtacttt atatttataa atgtatttat tattattata agactgcatt 1000
ttatttatat cattttatta atatggattt atttatagaa acatcattcg 1050
atattgctac ttgagtgtaa ggctaatatt gatatttatg acaataatta 1100
tagagctata acatgtttat ttgacctcaa taaacacttg gatatcc 1147

<210> 8
<211> 179
<212> PRT
<213> Homo sapiens

<400> 8

Met Ala Ala Leu Gln Lys Ser Val Ser Ser Phe Leu Met Gly Thr
1               5                   10                  15

Leu Ala Thr Ser Cys Leu Leu Leu Leu Ala Leu Leu Val Gln Gly
            20                  25                  30

Gly Ala Ala Ala Pro Ile Ser Ser His Cys Arg Leu Asp Lys Ser
            35                  40                  45

Asn Phe Gln Gln Pro Tyr Ile Thr Asn Arg Thr Phe Met Leu Ala
            50                  55                  60

Lys Glu Ala Ser Leu Ala Asp Asn Asn Thr Asp Val Arg Leu Ile
            65                  70                  75

Gly Glu Lys Leu Phe His Gly Val Ser Met Ser Glu Arg Cys Tyr
            80                  85                  90

Leu Met Lys Gln Val Leu Asn Phe Thr Leu Glu Glu Val Leu Phe
            95                  100                 105

Pro Gln Ser Asp Arg Phe Gln Pro Tyr Met Gln Glu Val Val Pro
            110                 115                 120

Phe Leu Ala Arg Leu Ser Asn Arg Leu Ser Thr Cys His Ile Glu
            125                 130                 135

Gly Asp Asp Leu His Ile Gln Arg Asn Val Gln Lys Leu Lys Asp
            140                 145                 150

Thr Val Lys Lys Leu Gly Glu Ser Gly Glu Ile Lys Ala Ile Gly
            155                 160                 165

Glu Leu Asp Leu Leu Phe Met Ser Leu Arg Asn Ala Cys Ile
            170                 175

<210> 9
<211> 1049
<212> DNA
<213> Homo sapiens

<400> 9

aaaacaacag gaagcagctt acaaactcgg tgaacaactg agggaaccaa 50

accagagacg cgctgaacag agagaatcag gctcaaagca agtggaagtg 100

ggcagagatt ccaccaggac tggtgcaagg cgcagagcca gccagatttg 150

agaagaaggc aaaaagatgc tggggagcag agctgtaatg ctgctgttgc 200

tgctgccctg gacagctcag ggcagagctg tgcctggggg cagcagccct 250

gcctggactc agtgccagca gctttcacag aagctctgca cactggcctg 300

gagtgcacat ccactagtgg gacacatgga tctaagagaa gagggagatg 350

aagagactac aaatgatgtt ccccatatcc agtgtggaga tggctgtgac 400

ccccaaggac tcagggacaa cagtcagttc tgcttgcaaa ggatccacca 450

gggtctgatt ttttatgaga agctgctagg atcggatatt ttcacagggg 500

agccttctct gctccctgat agccctgtgg gccagcttca tgcctcccta 550
ctgggcctca gccaactcct gcagcctgag ggtcaccact gggagactca 600
gcagattcca agcctcagtc ccagccagcc atggcagcgt ctccttctcc 650
gcttcaaaat ccttcgcagc ctccaggcct ttgtggctgt agccgcccgg 700
gtctttgccc atggagcagc aaccctgagt ccctaaaggc agcagctcaa 750
ggatggcact cagatctcca tggcccagca aggccaagat aaatctacca 800
ccccaggcac ctgtgagcca acaggttaat tagtccatta attttagtgg 850
gacctgcata tgttgaaaat taccaatact gactgacatg tgatgctgac 900
ctatgataag gttgagtatt tattagatgg gaagggaaat ttggggatta 950
tttatcctcc tggggacagt ttggggagga ttatttattg tatttatatt 1000
gaattatgta ctttttttcaa taaagtctta tttttgtggc taaaaaaaa 1049

<210> 10
<211> 189
<212> PRT
<213> Homo sapiens

<400> 10

```
Met Leu Gly Ser Arg Ala Val Met Leu Leu Leu Leu Leu Pro Trp
1               5                   10                  15

Thr Ala Gln Gly Arg Ala Val Pro Gly Gly Ser Ser Pro Ala Trp
                20                  25                  30

Thr Gln Cys Gln Gln Leu Ser Gln Lys Leu Cys Thr Leu Ala Trp
                35                  40                  45

Ser Ala His Pro Leu Val Gly His Met Asp Leu Arg Glu Glu Gly
                50                  55                  60

Asp Glu Glu Thr Thr Asn Asp Val Pro His Ile Gln Cys Gly Asp
                65                  70                  75

Gly Cys Asp Pro Gln Gly Leu Arg Asp Asn Ser Gln Phe Cys Leu
                80                  85                  90

Gln Arg Ile His Gln Gly Leu Ile Phe Tyr Glu Lys Leu Leu Gly
                95                  100                 105

Ser Asp Ile Phe Thr Gly Glu Pro Ser Leu Leu Pro Asp Ser Pro
                110                 115                 120

Val Gly Gln Leu His Ala Ser Leu Leu Gly Leu Ser Gln Leu Leu
                125                 130                 135

Gln Pro Glu Gly His His Trp Glu Thr Gln Gln Ile Pro Ser Leu
                140                 145                 150

Ser Pro Ser Gln Pro Trp Gln Arg Leu Leu Leu Arg Phe Lys Ile
                155                 160                 165

Leu Arg Ser Leu Gln Ala Phe Val Ala Val Ala Ala Arg Val Phe
                170                 175                 180

Ala His Gly Ala Ala Thr Leu Ser Pro

                        185
```

<210> 11
<211> 808
<212> DNA
<213> Homo sapiens

<400> 11

gatataaagc tcctacagct acctggcctg agaagccaac tcagactcag 50

ccaacagaga ttgttgattt gcctcttaag caagagattc attgcagctc 100

agcatggctc agaccagctc atacttcatg ctgatctcct gcctgatgtt 150

tctgtctcag agccaaggcc aagaggccca gacagagttg ccccaggccc 200

ggatcagctg cccagaaggc accaatgcct atcgctccta ctgctactac 250

tttaatgaag accgtgagac ctgggttgat gcagatctct attgccagaa 300

catgaattcg ggcaacctgg tgtctgtgct cacccaggcc gagggtgcct 350

ttgtggcctc actgattaag gagagtggca ctgatgactt caatgtctgg 400

attggcctcc atgaccccaa aaagaaccgc cgctggcact ggagcagtgg 450

gtccctggtc tcctacaagt cctggggcat tggagcccca agcagtgtta 500

atcctggcta ctgtgtgagc ctgacctcaa gcacaggatt ccagaaatgg 550

aaggatgtgc cttgtgaaga caagttctcc tttgtctgca agttcaaaaa 600

ctagaggcaa ctggaaaata catgtctaga actgatccag caattacaac 650

ggagtcaaaa attaaaccgg accatctctc caactcaact caacctggac 700

actctcttct ctgctgagtt tgccttgtta atcttcaata gttttaccta 750

ccccagtctt tggaacccta aataataaaa ataaacatgt ttccactatt 800

gtgctgtc 808

<210> 12
<211> 166
<212> PRT
<213> Homo sapiens

<400> 12

Met Ala Gln Thr Ser Ser Tyr Phe Met Leu Ile Ser Cys Leu Met
1 5 10 15

Phe Leu Ser Gln Ser Gln Gly Gln Glu Ala Gln Thr Glu Leu Pro
20 25 30

Gln Ala Arg Ile Ser Cys Pro Glu Gly Thr Asn Ala Tyr Arg Ser
35 40 45

Tyr Cys Tyr Tyr Phe Asn Glu Asp Arg Glu Thr Trp Val Asp Ala
50 55 60

Asp Leu Tyr Cys Gln Asn Met Asn Ser Gly Asn Leu Val Ser Val
65 70 75

Leu Thr Gln Ala Glu Gly Ala Phe Val Ala Ser Leu Ile Lys Glu
80 85 90

```
Ser Gly Thr Asp Asp Phe Asn Val Trp Ile Gly Leu His Asp Pro
                95                  100                 105

Lys Lys Asn Arg Arg Trp His Trp Ser Ser Gly Ser Leu Val Ser
                110                 115                 120

Tyr Lys Ser Trp Gly Ile Gly Ala Pro Ser Ser Val Asn Pro Gly
                125                 130                 135

Tyr Cys Val Ser Leu Thr Ser Ser Thr Gly Phe Gln Lys Trp Lys
                140                 145                 150

Asp Val Pro Cys Glu Asp Lys Phe Ser Phe Val Cys Lys Phe Lys
                155                 160                 165

Asn
```

<210> 13
<211> 773
<212> DNA
<213> Homo sapiens

<400> 13

```
aagccacctc aagtggacaa ggcacttacc aacagagatt gctgatttgc 50
tccttaagca agagattcac tgccgctaag catggctcag accaactcgt 100
tcttcatgct gatctcctcc ctgatgttcc tgtctctgag ccaaggccag 150
gagtcccaga cagagctgcc taatccccga atcagctgcc cagaaggcac 200
caatgcctat cgctcctact gctactactt taatgaagac cctgagacct 250
gggttgatgc agatctctat tgccagaaca tgaattcagg caacctggtg 300
tctgtgctca cccaggcgga gggtgccttc gtggcctcac tgattaagga 350
gagtagcact gatgacagca atgtctggat tggcctccat gacccaaaaa 400
agaaccgccg ctggcactgg agtagtgggt ccctggtctc ctacaagtcc 450
tgggacactg gatccccgag cagtgctaat gctggctact gtgcaagcct 500
gacttcatgc tcaggattca agaaatggaa ggatgaatct tgtgagaaga 550
agttctcctt tgtttgcaag ttcaaaaact agaggaagct gaaaaatgga 600
tgtctagaac tggtcctgca attactatga agtcaaaaat taaactagac 650
tatgtctcca actcagttca gaccatctcc tccctaatga gtttgcatcg 700
ctgatcttca gtaccttcac ctgtctcagt ctctagagcc ctgaaaaata 750
aaaacaaact tatttttatc cag 773
```

<210> 14
<211> 166
<212> PRT
<213> Homo sapiens

<400> 14

```
Met Ala Gln Thr Asn Ser Phe Phe Met Leu Ile Ser Ser Leu Met
1               5                   10                  15

Phe Leu Ser Leu Ser Gln Gly Gln Glu Ser Gln Thr Glu Leu Pro
                20                  25                  30

Asn Pro Arg Ile Ser Cys Pro Glu Gly Thr Asn Ala Tyr Arg Ser
                35                  40                  45

Tyr Cys Tyr Tyr Phe Asn Glu Asp Pro Glu Thr Trp Val Asp Ala
                50                  55                  60

Asp Leu Tyr Cys Gln Asn Met Asn Ser Gly Asn Leu Val Ser Val
                65                  70                  75

Leu Thr Gln Ala Glu Gly Ala Phe Val Ala Ser Leu Ile Lys Glu
                80                  85                  90

Ser Ser Thr Asp Asp Ser Asn Val Trp Ile Gly Leu His Asp Pro
                95                  100                 105

Lys Lys Asn Arg Arg Trp His Trp Ser Ser Gly Ser Leu Val Ser
                110                 115                 120

Tyr Lys Ser Trp Asp Thr Gly Ser Pro Ser Ser Ala Asn Ala Gly
                125                 130                 135

Tyr Cys Ala Ser Leu Thr Ser Cys Ser Gly Phe Lys Lys Trp Lys
                140                 145                 150

Asp Glu Ser Cys Glu Lys Lys Phe Ser Phe Val Cys Lys Phe Lys
                155                 160                 165

Asn
```

<210> 15
<211> 807
<212> DNA
<213> Homo sapiens

<400> 15

aaaccatacc atatcccacc agagagtgac tcctgattgc ctcctcaagt 50

cgcagacact atgctgcctc ccatggccct gcccagtgta tcttggatgc 100

tgctttcctg cctcatgctg ctgtctcagg ttcaaggtga agaaccccag 150

agggaactgc cctctgcacg gatccgctgt cccaaaggct ccaaggccta 200

tggctcccac tgctatgcct tgtttttgtc accaaaatcc tggacagatg 250

cagatctggc ctgccagaag cggccctctg gaaacctggt gtctgtgctc 300

agtggggctg agggatcctt cgtgtcctcc ctggtgaaga gcattggtaa 350

cagctactca tacgtctgga ttgggctcca tgaccccaca cagggcaccg 400

agcccaatgg agaaggttgg gagtggagta gcagtgatgt gatgaattac 450

tttgcatggg agagaaatcc ctccaccatc tcaagccccg gccactgtgc 500

gagcctgtcg agaagcacag catttctgag gtggaaagat tataactgta 550

atgtgaggtt accctatgtc tgcaagttca ctgactagtg caggagggaa 600

gtcagcagcc tgtgtttggt gtgcaactca tcatgggcat gagaccagtg 650

tgaggactca ccctggaaga gaatattcgc ttaattcccc caacctgacc 700

acctcattct tatctttctt ctgtttcttc ctccccgctg tcatttcagt 750

ctcttcattt tgtcatacgg cctaaggctt taaagagcaa taaaattttt 800

agtctgc 807

<210> 16
<211> 175
<212> PRT
<213> Homo sapiens

<400> 16

```
Met Leu Pro Pro Met Ala Leu Pro Ser Val Ser Trp Met Leu Leu
1               5                   10                  15

Ser Cys Leu Met Leu Leu Ser Gln Val Gln Gly Glu Glu Pro Gln
                20                  25                  30

Arg Glu Leu Pro Ser Ala Arg Ile Arg Cys Pro Lys Gly Ser Lys
                35                  40                  45

Ala Tyr Gly Ser His Cys Tyr Ala Leu Phe Leu Ser Pro Lys Ser
                50                  55                  60

Trp Thr Asp Ala Asp Leu Ala Cys Gln Lys Arg Pro Ser Gly Asn
                65                  70                  75

Leu Val Ser Val Leu Ser Gly Ala Glu Gly Ser Phe Val Ser Ser
                80                  85                  90

Leu Val Lys Ser Ile Gly Asn Ser Tyr Ser Tyr Val Trp Ile Gly
                95                  100                 105

Leu His Asp Pro Thr Gln Gly Thr Glu Pro Asn Gly Glu Gly Trp
                110                 115                 120

Glu Trp Ser Ser Ser Asp Val Met Asn Tyr Phe Ala Trp Glu Arg
                125                 130                 135

Asn Pro Ser Thr Ile Ser Ser Pro Gly His Cys Ala Ser Leu Ser
                140                 145                 150

Arg Ser Thr Ala Phe Leu Arg Trp Lys Asp Tyr Asn Cys Asn Val
                155                 160                 165

Arg Leu Pro Tyr Val Cys Lys Phe Thr Asp
                170                 175
```

<210> 17
<211> 784
<212> DNA
<213> Homo sapiens

<400> 17

```
aaaccatacc atatcccacc agagagtcgc agacactatg ctgcctccca 50
tggccctgcc cagtgtatct tggatgctgc tttcctgcct catgctgctg 100
tctcaggttc aaggtgaaga accccagagg gaactgccct ctgcacggat 150
ccgctgtccc aaaggctcca aggcctatgg ctcccactgc tatgccttgt 200
```

ttttgtcacc aaaatcctgg acagatgcag atctggcctg ccagaagcgg 250

ccctctggaa acctggtgtc tgtgctcagt ggggctgagg gatccttcgt 300

gtcctccctg gtgaagagca ttggtaacag ctactcatac gtctggattg 350

ggctccatga ccccacacag ggcaccgagc ccaatggaga aggttgggag 400

tggagtagca gtgatgtgat gaattacttt gcatgggaga gaaatccctc 450

caccatctca agccccggcc actgtgcgag cctgtcgaga agcacagcat 500

ttctgaggtg gaaagattat aactgtaatg tgaggttacc ctatgtctgc 550

aagttcactg actagtgcag gagggaagtc agcagcctgt gtttggtgtg 600

caactcatca tgggcatgag accagtgtga ggactcaccc tggaagagaa 650

tattcgctta attcccccaa cctgaccacc tcattcttat ctttcttctg 700

tttcttcctc cccgctgtca tttcagtctc ttcattttgt catacggcct 750

aaggctttaa agagcaataa aatttttagt ctgc 784

<210> 18
<211> 175
<212> PRT
<213> Homo sapiens

<400> 18

```
Met Leu Pro Pro Met Ala Leu Pro Ser Val Ser Trp Met Leu Leu
1               5                   10                  15

Ser Cys Leu Met Leu Leu Ser Gln Val Gln Gly Glu Glu Pro Gln
                20                  25                  30

Arg Glu Leu Pro Ser Ala Arg Ile Arg Cys Pro Lys Gly Ser Lys
                35                  40                  45

Ala Tyr Gly Ser His Cys Tyr Ala Leu Phe Leu Ser Pro Lys Ser
                50                  55                  60

Trp Thr Asp Ala Asp Leu Ala Cys Gln Lys Arg Pro Ser Gly Asn
                65                  70                  75

Leu Val Ser Val Leu Ser Gly Ala Glu Gly Ser Phe Val Ser Ser
                80                  85                  90

Leu Val Lys Ser Ile Gly Asn Ser Tyr Ser Tyr Val Trp Ile Gly
                95                  100                 105

Leu His Asp Pro Thr Gln Gly Thr Glu Pro Asn Gly Glu Gly Trp
                110                 115                 120

Glu Trp Ser Ser Ser Asp Val Met Asn Tyr Phe Ala Trp Glu Arg
                125                 130                 135

Asn Pro Ser Thr Ile Ser Ser Pro Gly His Cys Ala Ser Leu Ser
                140                 145                 150

Arg Ser Thr Ala Phe Leu Arg Trp Lys Asp Tyr Asn Cys Asn Val
                155                 160                 165

Arg Leu Pro Tyr Val Cys Lys Phe Thr Asp
                170                 175
```

<210> 19
<211> 1002
<212> DNA
<213> Homo sapiens

<400> 19

gggagggtcc cttcctcagg gagcacagga actctgagac tcagcaaggg 50

tgtcctggga gggctcgggg atgggagagt acacagattc acaactcatt 100

cagaactgta gaagatgatg gatgtgacca agatcacttt agtcctaggg 150

gactagagaa ggaaaatgac atgaggcagt ggggtatctg tgtgttctcc 200

cactgaccac gctttcttta gtgactcctg attgcctcct caagtcgcag 250

acactatgct gcctcccatg gccctgccca gtgtatcttg gatgctgctt 300

tcctgcctca tgctgctgtc tcaggttcaa ggtgaagaac cccagaggga 350

actgccctct gcacggatcc gctgtcccaa aggctccaag gcctatggct 400

cccactgcta tgccttgttt ttgtcaccaa aatcctggac agatgcagat 450

ctggcctgcc agaagcggcc ctctggaaac ctggtgtctg tgctcagtgg 500

ggctgaggga tccttcgtgt cctccctggt gaagagcatt ggtaacagct 550

actcatacgt ctggattggg ctccatgacc ccacacaggg caccgagccc 600

aatggagaag gttgggagtg gagtagcagt gatgtgatga attactttgc 650

atgggagaga aatccctcca ccatctcaag ccccggccac tgtgcgagcc 700

tgtcgagaag cacagcattt ctgaggtgga aagattataa ctgtaatgtg 750

aggttaccct atgtctgcaa gttcactgac tagtgcagga gggaagtcag 800

cagcctgtgt ttggtgtgca actcatcatg ggcatgagac cagtgtgagg 850

actcaccctg gaagagaata ttcgcttaat tcccccaacc tgaccacctc 900

attcttatct ttcttctgtt tcttcctccc cgctgtcatt tcagtctctt 950

cattttgtca tacggcctaa ggctttaaag agcaataaaa tttttagtct 1000

gc 1002

<210> 20
<211> 175
<212> PRT
<213> Homo sapiens

<400> 20

Met Leu Pro Pro Met Ala Leu Pro Ser Val Ser Trp Met Leu Leu
1 5 10 15

Ser Cys Leu Met Leu Leu Ser Gln Val Gln Gly Glu Glu Pro Gln
20 25 30

Arg Glu Leu Pro Ser Ala Arg Ile Arg Cys Pro Lys Gly Ser Lys
35 40 45

Ala Tyr Gly Ser His Cys Tyr Ala Leu Phe Leu Ser Pro Lys Ser

```
                        50                  55                  60

Trp Thr Asp Ala Asp Leu Ala Cys Gln Lys Arg Pro Ser Gly Asn
                65                  70                  75

Leu Val Ser Val Leu Ser Gly Ala Glu Gly Ser Phe Val Ser Ser
                80                  85                  90

Leu Val Lys Ser Ile Gly Asn Ser Tyr Ser Tyr Val Trp Ile Gly
                95                  100                 105

Leu His Asp Pro Thr Gln Gly Thr Glu Pro Asn Gly Glu Gly Trp
                110                 115                 120

Glu Trp Ser Ser Ser Asp Val Met Asn Tyr Phe Ala Trp Glu Arg
                125                 130                 135

Asn Pro Ser Thr Ile Ser Ser Pro Gly His Cys Ala Ser Leu Ser
                140                 145                 150

Arg Ser Thr Ala Phe Leu Arg Trp Lys Asp Tyr Asn Cys Asn Val
                155                 160                 165

Arg Leu Pro Tyr Val Cys Lys Phe Thr Asp
                170                 175
```

<210> 21
<211> 847
<212> DNA
<213> Homo sapiens

<400> 21

```
ccatccctga gatcttttta taaaaaaccc agtctttgct gaccagacaa 50
agcataccag atctcaccag agagtcgcag acactatgct gcctcccatg 100
gccctgccca gtgtgtcctg gatgctgctt tcctgcctca ttctcctgtg 150
tcaggttcaa ggtgaagaaa cccagaagga actgccctct ccacggatca 200
gctgtcccaa aggctccaag gcctatggct ccccctgcta tgccttgttt 250
ttgtcaccaa aatcctggat ggatgcagat ctggcttgcc agaagcggcc 300
ctctggaaaa ctggtgtctg tgctcagtgg ggctgaggga tccttcgtgt 350
cctccctggt gaggagcatt agtaacagct attcatacat ctggattggg 400
ctccatgacc ccacacaggg ctctgagcct gatggagatg gatgggagtg 450
gagtagcact gatgtgatga attactttgc atgggagaaa aatccctcca 500
ccatcttaaa ccctggccac tgtgggagcc tgtcaagaag cacaggattt 550
ctgaagtgga aagattataa ctgtgatgca aagttaccct atgtctgcaa 600
gttcaaggac tagggcaggt gggaagtcag cagcctgagc ttggcgtgca 650
gctcatcatg gacatgagac cagtgtgaag actcaccctg gaagagaata 700
ttctccccaa actgccctac ctgactacct tgtcatgatc ctccttcttt 750
ttcctttttc ttcaccttca tttcaggctt ttctctgtct tccatgtctt 800
gagatctcag agaataataa taaaaatgtt actttatact  taaaaaa 847
```

<210> 22
<211> 175
<212> PRT
<213> Homo sapiens

<400> 22

```
Met Leu Pro Pro Met Ala Leu Pro Ser Val Ser Trp Met Leu Leu
1               5                   10                  15

Ser Cys Leu Ile Leu Leu Cys Gln Val Gln Gly Glu Glu Thr Gln
                20                  25                  30

Lys Glu Leu Pro Ser Pro Arg Ile Ser Cys Pro Lys Gly Ser Lys
                35                  40                  45

Ala Tyr Gly Ser Pro Cys Tyr Ala Leu Phe Leu Ser Pro Lys Ser
                50                  55                  60

Trp Met Asp Ala Asp Leu Ala Cys Gln Lys Arg Pro Ser Gly Lys
                65                  70                  75

Leu Val Ser Val Leu Ser Gly Ala Glu Gly Ser Phe Val Ser Ser
                80                  85                  90

Leu Val Arg Ser Ile Ser Asn Ser Tyr Ser Tyr Ile Trp Ile Gly
                95                  100                 105

Leu His Asp Pro Thr Gln Gly Ser Glu Pro Asp Gly Asp Gly Trp
                110                 115                 120

Glu Trp Ser Ser Thr Asp Val Met Asn Tyr Phe Ala Trp Glu Lys
                125                 130                 135

Asn Pro Ser Thr Ile Leu Asn Pro Gly His Cys Gly Ser Leu Ser
                140                 145                 150

Arg Ser Thr Gly Phe Leu Lys Trp Lys Asp Tyr Asn Cys Asp Ala
                155                 160                 165

Lys Leu Pro Tyr Val Cys Lys Phe Lys Asp
                170                 175
```

<210> 23
<211> 947
<212> DNA
<213> Homo sapiens

<400> 23

```
ccatccctga gatcttttta taaaaaaccc agtctttgct gaccagacaa 50

agcataccag atctcaccag agagtcctag gggactacag aaggaaaaag 100

acaagaggca gtaggatatc tgtgtgtcct cccgctgacc acacttcctt 150

tagtgacccg attgcctcct caagtcgcag acactatgct gcctcccatg 200

gccctgccca gtgtgtcctg gatgctgctt tcctgcctca ttctcctgtg 250

tcaggttcaa ggtgaagaaa cccagaagga actgccctct ccacggatca 300

gctgtcccaa aggctccaag gcctatggct ccccctgcta tgccttgttt 350

ttgtcaccaa aatcctggat ggatgcagat ctggcttgcc agaagcggcc 400
```

ctctggaaaa ctggtgtctg tgctcagtgg ggctgaggga tccttcgtgt 450
cctccctggt gaggagcatt agtaacagct attcatacat ctggattggg 500
ctccatgacc ccacacaggg ctctgagcct gatggagatg gatgggagtg 550
gagtagcact gatgtgatga attactttgc atgggagaaa aatccctcca 600
ccatcttaaa ccctggccac tgtgggagcc tgtcaagaag cacaggattt 650
ctgaagtgga aagattataa ctgtgatgca aagttaccct atgtctgcaa 700
gttcaaggac tagggcaggt gggaagtcag cagcctgagc ttggcgtgca 750
gctcatcatg gacatgagac cagtgtgaag actcaccctg gaagagaata 800
ttctccccaa actgccctac ctgactacct tgtcatgatc ctccttcttt 850
ttcctttttc ttcaccttca tttcaggctt ttctctgtct tccatgtctt 900
gagatctcag agaataataa taaaaatgtt actttatacg taaaaaa 947

<210> 24
<211> 175
<212> PRT
<213> Homo sapiens

<400> 24

```
Met Leu Pro Pro Met Ala Leu Pro Ser Val Ser Trp Met Leu Leu
1               5                   10                  15

Ser Cys Leu Ile Leu Leu Cys Gln Val Gln Gly Glu Glu Thr Gln
                20                  25                  30

Lys Glu Leu Pro Ser Pro Arg Ile Ser Cys Pro Lys Gly Ser Lys
                35                  40                  45

Ala Tyr Gly Ser Pro Cys Tyr Ala Leu Phe Leu Ser Pro Lys Ser
                50                  55                  60

Trp Met Asp Ala Asp Leu Ala Cys Gln Lys Arg Pro Ser Gly Lys
                65                  70                  75

Leu Val Ser Val Leu Ser Gly Ala Glu Gly Ser Phe Val Ser Ser
                80                  85                  90

Leu Val Arg Ser Ile Ser Asn Ser Tyr Ser Tyr Ile Trp Ile Gly
                95                  100                 105

Leu His Asp Pro Thr Gln Gly Ser Glu Pro Asp Gly Asp Gly Trp
                110                 115                 120

Glu Trp Ser Ser Thr Asp Val Met Asn Tyr Phe Ala Trp Glu Lys
                125                 130                 135

Asn Pro Ser Thr Ile Leu Asn Pro Gly His Cys Gly Ser Leu Ser
                140                 145                 150

Arg Ser Thr Gly Phe Leu Lys Trp Lys Asp Tyr Asn Cys Asp Ala
                155                 160                 165

Lys Leu Pro Tyr Val Cys Lys Phe Lys Asp
                170                 175
```

<210> 25
<211> 1285
<212> DNA
<213> Homo sapiens

<400> 25

ataagacttt tatggatgga ttgtttttct caaataatat tatcgctttg 50

tgactaaagt aaagattatt aattcctgag gcaagaagat ataaaagctc 100

cagaaacgtt gactgggacc actggagaca ctgaagaagg caggggccct 150

tagagtcttg gttgccaaac agatttgcag atcaaggaga acccaggagt 200

ttcaaagaag cgctagtaag gtctctgaga tccttgcact agctacatcc 250

tcagggtagg aggaagatgg cttccagaag catgcggctg ctcctattgc 300

tgagctgcct ggccaaaaca ggagtcctgg gtgatatcat catgagaccc 350

agctgtgctc ctggatggtt ttaccacaag tccaattgct atggttactt 400

caggaagctg aggaactggt ctgatgccga gctcgagtgt cagtcttacg 450

gaaacggagc ccacctggca tctatcctga gtttaaagga agccagcacc 500

atagcagagt acataagtgg ctatcagaga agccagccga tatggattgg 550

cctgcacgac ccacagaaga ggcagcagtg gcagtggatt gatggggcca 600

tgtatctgta cagatcctgg tctggcaagt ccatgggtgg gaacaagcac 650

tgtgctgaga tgagctccaa taacaacttt ttaacttgga gcagcaacga 700

atgcaacaag cgccaacact tcctgtgcaa gtaccgacca tagagcaaga 750

atcaagattc tgctaactcc tgcacagccc cgtcctcttc ctttctgcta 800

gcctggctaa atctgctcat tatttcagag gggaaaccta gcaaactaag 850

agtgataagg gccctactac actggctttt ttaggcttag agacagaaac 900

tttagcattg gcccagtagt ggcttctagc tctaaatgtt tgccccgcca 950

tccctttcca cagtatcctt cttccctcct cccctgtctc tggctgtctc 1000

gagcagtcta gaagagtgca tctccagcct atgaaacagc tgggtctttg 1050

gccataagaa gtaaagattt gaagacagaa ggaagaaact caggagtaag 1100

cttctagacc ccttcagctt ctacaccctt ctgccctctc tccattgcct 1150

gcaccccacc ccagccactc aactcctgct tgtttttcct ttggccatag 1200

gaaggtttac cagtagaatc cttgctaggt tgatgtgggc catacattcc 1250

tttaataaac cattgtgtac ataagaaaaa aaaaa 1285

<210> 26
<211> 158
<212> PRT
<213> Homo sapiens

<400> 26

Met Ala Ser Arg Ser Met Arg Leu Leu Leu Leu Leu Ser Cys Leu
1 5 10 15

Ala Lys Thr Gly Val Leu Gly Asp Ile Ile Met Arg Pro Ser Cys
20 25 30

Ala Pro Gly Trp Phe Tyr His Lys Ser Asn Cys Tyr Gly Tyr Phe
35 40 45

Arg Lys Leu Arg Asn Trp Ser Asp Ala Glu Leu Glu Cys Gln Ser
50 55 60

Tyr Gly Asn Gly Ala His Leu Ala Ser Ile Leu Ser Leu Lys Glu
65 70 75

Ala Ser Thr Ile Ala Glu Tyr Ile Ser Gly Tyr Gln Arg Ser Gln
80 85 90

Pro Ile Trp Ile Gly Leu His Asp Pro Gln Lys Arg Gln Gln Trp
95 100 105

Gln Trp Ile Asp Gly Ala Met Tyr Leu Tyr Arg Ser Trp Ser Gly
110 115 120

Lys Ser Met Gly Gly Asn Lys His Cys Ala Glu Met Ser Ser Asn
125 130 135

Asn Asn Phe Leu Thr Trp Ser Ser Asn Glu Cys Asn Lys Arg Gln
140 145 150

His Phe Leu Cys Lys Tyr Arg Pro
155

<210> 27
<211> 1087
<212> DNA
<213> Mus musculus

<400> 27

```
ccaagaacga tagtcaattc cagaaaccgc tatgaagttc ctctctgcaa 50
gagacttcca tccagttgcc ttcttgggac tgatgctggt gacaaccacg 100
gccttcccta cttcacaagt ccggagagga gacttcacag aggataccac 150
tcccaacaga cctgtctata ccacttcaca agtcggaggc ttaattacac 200
atgttctctg ggaaatcgtg gaaatgagaa aagagttgtg caatggcaat 250
tctgattgta tgaacaacga tgatgcactt gcagaaaaca atctgaaact 300
tccagagata caaagaaatg atggatgcta ccaaactgga tataatcagg 350
aaatttgcct attgaaaatt tcctctggtc ttctggagta ccatagctac 400
ctggagtaca tgaagaacaa cttaaaagat aacaagaaag acaaagccag 450
agtccttcag agagatacag aaactctaat tcatatcttc aaccaagagg 500
taaaagattt acataaaata gtccttccta ccccaatttc caatgctctc 550
ctaacagata agctggagtc acagaaggag tggctaagga ccaagaccat 600
ccaattcatc ttgaaatcac ttgaagaatt tctaaaagtc actttgagat 650
ctactcggca aacctagtgc gttatgccta agcatatcag tttgtggaca 700
ttcctcactg tggtcagaaa atatatcctg ttgtcaggta tctgacttat 750
gttgttctct acgaagaact gacaatatga atgttgggac actattttaa 800
ttatttttaa tttattgata atttaaataa gtaaacttta agttaattta 850
tgattgatat ttattatttt tatgaagtgt cacttgaaat gttatatgtt 900
atagttttga aatgataacc taaaaatcta tttgatataa atattctgtt 950
acctagccag atggtttctt ggaatgtata agtttacctc aatgaattgc 1000
taatttaaat atgtttttaa agaaatcttt gtgatgtatt tttataatgt 1050
ttagactgtc ttcaaacaaa taaattatat  tatattt 1087
```

<210> 28
<211> 211
<212> PRT
<213> Mus musculus

<400> 28

```
Met Lys Phe Leu Ser Ala Arg Asp Phe His Pro Val Ala Phe Leu
1               5                   10                  15

Gly Leu Met Leu Val Thr Thr Thr Ala Phe Pro Thr Ser Gln Val
                20                  25                  30

Arg Arg Gly Asp Phe Thr Glu Asp Thr Thr Pro Asn Arg Pro Val
                35                  40                  45

Tyr Thr Thr Ser Gln Val Gly Gly Leu Ile Thr His Val Leu Trp
                50                  55                  60

Glu Ile Val Glu Met Arg Lys Glu Leu Cys Asn Gly Asn Ser Asp
                65                  70                  75

Cys Met Asn Asn Asp Asp Ala Leu Ala Glu Asn Asn Leu Lys Leu
                80                  85                  90

Pro Glu Ile Gln Arg Asn Asp Gly Cys Tyr Gln Thr Gly Tyr Asn
                95                  100                 105

Gln Glu Ile Cys Leu Leu Lys Ile Ser Ser Gly Leu Leu Glu Tyr
                110                 115                 120

His Ser Tyr Leu Glu Tyr Met Lys Asn Asn Leu Lys Asp Asn Lys
                125                 130                 135

Lys Asp Lys Ala Arg Val Leu Gln Arg Asp Thr Glu Thr Leu Ile
                140                 145                 150

His Ile Phe Asn Gln Glu Val Lys Asp Leu His Lys Ile Val Leu
                155                 160                 165

Pro Thr Pro Ile Ser Asn Ala Leu Leu Thr Asp Lys Leu Glu Ser
                170                 175                 180

Gln Lys Glu Trp Leu Arg Thr Lys Thr Ile Gln Phe Ile Leu Lys
                185                 190                 195

Ser Leu Glu Glu Phe Leu Lys Val Thr Leu Arg Ser Thr Arg Gln
                200                 205                 210

Thr
```

<210> 29
<211> 1951
<212> DNA
<213> Mus musculus

<400> 29

agaaggaaca gtgggtgtcc aggcacatca gaccaggcag ctcgcagcaa 50
agcaaggtaa gttctctcct cttccctgtc gctaactccc tgcatctaga 100
ggctgtccag attcagactc caggggacag gctacccctg aaccaggcag 150
cgtgggagtg ggatgtgtcc tcagaagcta accatctcct ggtttgccat 200
cgttttgctg gtgtctccac tcatggccat gtgggagctg gagaaagacg 250
tttatgttgt agaggtggac tggactcccg atgcccctgg agaaacagtg 300
aacctcacct gtgacacgcc tgaagaagat gacatcacct ggacctcaga 350
ccagagacat ggagtcatag gctctggaaa gaccctgacc atcactgtca 400
aagagtttct agatgctggc cagtacacct gccacaaagg aggcgagact 450
ctgagccact cacatctgct gctccacaag aaggaaaatg gaatttggtc 500
cactgaaatt ttaaaaaatt tcaaaaacaa gactttcctg aagtgtgaag 550
caccaaatta ctccggacgg ttcacgtgct catggctggt gcaaagaaac 600
atggacttga agttcaacat caagagcagt agcagttccc ctgactctcg 650
ggcagtgaca tgtggaatgg cgtctctgtc tgcagagaag gtcacactgg 700
accaaaggga ctatgagaag tattcagtgt cctgccagga ggatgtcacc 750
tgcccaactg ccgaggagac cctgcccatt gaactggcgt tggaagcacg 800
gcagcagaat aaatatgaga actacagcac cagcttcttc atcagggaca 850
tcatcaaacc agacccgccc aagaacttgc agatgaagcc tttgaagaac 900
tcacaggtgg aggtcagctg ggagtaccct gactcctgga gcactcccca 950
ttcctacttc tccctcaagt tctttgttcg aatccagcgc aagaaagaaa 1000
agatgaagga gacagaggag gggtgtaacc agaaaggtgc gttcctcgta 1050
gagaagacat ctaccgaagt ccaatgcaaa ggcgggaatg tctgcgtgca 1100
agctcaggat cgctattaca attcctcatg cagcaagtgg gcatgtgttc 1150
cctgcagggt ccgatcctag gatgcaacgt tggaaaggaa agaaaagtgg 1200
aagacattaa ggaagaaaaa tttaaactca ggatggaaga gtcccccaaa 1250
agctgtcttc tgcttggttg gctttttcca gttttcctaa gttcatcatg 1300
acacctttgc tgatttctac atgtaaatgt taaatgcccg cagagccagg 1350
gagctaatgt atgcatagat attctagcat tccacttggc cttatgctgt 1400
tgaaatattt aagtaattta tgtatttatt aatttatttc tgcatttcac 1450

atttgtatac caagatgtat tgaatatttc atgtgctcgt ggcctgatcc 1500

actgggacca ggccctatta tgcaaattgt gagcttgtta tcttcttcaa 1550

cagctcttca atcagggctg cgtaggtaca ttagcttttg tgacaaccaa 1600

taagaacata atattctgac acaagcagtg ttacatattt gtgaccagta 1650

aagacatagg tggtatttgg agacatgaag aagctgtaaa gttgactctg 1700

aagagtttag cactagtttc aacaccaaga aagacttttt agaagtgata 1750

ttgataagaa accagggcct tctttagaag ggtacctaaa tttaaaagaa 1800

ttttgaaagg ctgggtatcg gtggtatatg cttttaattc cagcactcag 1850

gagaccaagg caggcagatc tctgtgagtt tgaggacagc ctggtgtaca 1900

gagggagttc cagcacagcc agtgccacac agaaattctg tctcaaaaac 1950

a 1951

<210> 30
<211> 335
<212> PRT
<213> Mus musculus

<400> 30

```
Met Cys Pro Gln Lys Leu Thr Ile Ser Trp Phe Ala Ile Val Leu
1               5                   10                  15

Leu Val Ser Pro Leu Met Ala Met Trp Glu Leu Glu Lys Asp Val
                20                  25                  30

Tyr Val Val Glu Val Asp Trp Thr Pro Asp Ala Pro Gly Glu Thr
                35                  40                  45

Val Asn Leu Thr Cys Asp Thr Pro Glu Glu Asp Asp Ile Thr Trp
                50                  55                  60

Thr Ser Asp Gln Arg His Gly Val Ile Gly Ser Gly Lys Thr Leu
                65                  70                  75

Thr Ile Thr Val Lys Glu Phe Leu Asp Ala Gly Gln Tyr Thr Cys
                80                  85                  90

His Lys Gly Gly Glu Thr Leu Ser His Ser His Leu Leu Leu His
                95                  100                 105

Lys Lys Glu Asn Gly Ile Trp Ser Thr Glu Ile Leu Lys Asn Phe
                110                 115                 120

Lys Asn Lys Thr Phe Leu Lys Cys Glu Ala Pro Asn Tyr Ser Gly
                125                 130                 135

Arg Phe Thr Cys Ser Trp Leu Val Gln Arg Asn Met Asp Leu Lys
                140                 145                 150

Phe Asn Ile Lys Ser Ser Ser Ser Ser Pro Asp Ser Arg Ala Val
                155                 160                 165

Thr Cys Gly Met Ala Ser Leu Ser Ala Glu Lys Val Thr Leu Asp
                170                 175                 180
```

Gln Arg Asp Tyr Glu Lys Tyr Ser Val Ser Cys Gln Glu Asp Val
185 190 195

Thr Cys Pro Thr Ala Glu Glu Thr Leu Pro Ile Glu Leu Ala Leu
200 205 210

Glu Ala Arg Gln Gln Asn Lys Tyr Glu Asn Tyr Ser Thr Ser Phe
215 220 225

Phe Ile Arg Asp Ile Ile Lys Pro Asp Pro Pro Lys Asn Leu Gln
230 235 240

Met Lys Pro Leu Lys Asn Ser Gln Val Glu Val Ser Trp Glu Tyr
245 250 255

Pro Asp Ser Trp Ser Thr Pro His Ser Tyr Phe Ser Leu Lys Phe
260 265 270

Phe Val Arg Ile Gln Arg Lys Lys Glu Lys Met Lys Glu Thr Glu
275 280 285

Glu Gly Cys Asn Gln Lys Gly Ala Phe Leu Val Glu Lys Thr Ser
290 295 300

Thr Glu Val Gln Cys Lys Gly Gly Asn Val Cys Val Gln Ala Gln
305 310 315

Asp Arg Tyr Tyr Asn Ser Ser Cys Ser Lys Trp Ala Cys Val Pro
320 325 330

Cys Arg Val Arg Ser
335

<210> 31
<211> 866
<212> DNA
<213> Mus musculus

<400> 31

ggcacagctg gacctggtgg gggttctctg tggttccatg ctttctggac 50
tcctgcctgc tggctggagc tgctgacagg cctgacatct tctgcaacct 100
ccagcatcag gacaaagaaa gccgcctcaa accttccaaa tcacttcctc 150
ttggcccagg aacaatggct gccatgtcag aagactcttg cgtcaacttc 200
aaggaaatga tgtttattga caacacgctt tactttatac ctgaagaaaa 250
tggagacctg gaatcagaca actttggccg acttcactgt acaaccgcag 300
taatacggaa tataaatgac caagttctct tcgttgacaa aagacagcct 350
gtgttcgagg atatgactga tattgatcaa agtgccagtg aaccccagac 400
cagactgata atatacatgt acaaagacag tgaagtaaga ggactggctg 450
tgaccctctc tgtgaaggat agtaaaatgt ctaccctctc ctgtaagaac 500
aagatcattt cctttgagga aatggatcca cctgaaaata ttgatgatat 550
acaaagtgat ctcatattct ttcagaaacg tgttccagga cacaacaaga 600
tggagtttga atcttcactg tatgaaggac actttcttgc ttgccaaaag 650
gaagatgatg ctttcaaact cattctgaaa aaaaaggatg aaaatgggga 700
taaatctgta atgttcactc tcactaactt acatcaaagt taggtgggga 750
gggtttgtgt tccagaaaga tgattagcac acatgcgcct tgtgatgacc 800
tcgcctgtat ttccataaca gaatacccga ggctgcatga tttatagagt 850
aaacacgttt atttgt 866

<210> 32
<211> 192
<212> PRT
<213> Mus musculus

<400> 32

```
Met Ala Ala Met Ser Glu Asp Ser Cys Val Asn Phe Lys Glu Met
1               5                   10                  15

Met Phe Ile Asp Asn Thr Leu Tyr Phe Ile Pro Glu Glu Asn Gly
                20                  25                  30

Asp Leu Glu Ser Asp Asn Phe Gly Arg Leu His Cys Thr Thr Ala
                35                  40                  45

Val Ile Arg Asn Ile Asn Asp Gln Val Leu Phe Val Asp Lys Arg
                50                  55                  60

Gln Pro Val Phe Glu Asp Met Thr Asp Ile Asp Gln Ser Ala Ser
                65                  70                  75

Glu Pro Gln Thr Arg Leu Ile Ile Tyr Met Tyr Lys Asp Ser Glu
                80                  85                  90

Val Arg Gly Leu Ala Val Thr Leu Ser Val Lys Asp Ser Lys Met
                95                  100                 105

Ser Thr Leu Ser Cys Lys Asn Lys Ile Ile Ser Phe Glu Glu Met
                110                 115                 120

Asp Pro Pro Glu Asn Ile Asp Asp Ile Gln Ser Asp Leu Ile Phe
                125                 130                 135

Phe Gln Lys Arg Val Pro Gly His Asn Lys Met Glu Phe Glu Ser
                140                 145                 150

Ser Leu Tyr Glu Gly His Phe Leu Ala Cys Gln Lys Glu Asp Asp
                155                 160                 165

Ala Phe Lys Leu Ile Leu Lys Lys Lys Asp Glu Asn Gly Asp Lys
                170                 175                 180

Ser Val Met Phe Thr Leu Thr Asn Leu His Gln Ser
                185                 190
```

<210> 33
<211> 1121
<212> DNA
<213> Mus musculus

<400> 33

```
cctaaacagg ctctcctctc acttatcaac tgttgacact tgtgcgatct 50

ctgatggctg tcctgcagaa atctatgagt ttttccctta tggggacttt 100
```

ggccgccagc tgcctgcttc tcattgccct gtgggcccag gaggcaaatg 150
cgctgcccgt caacacccgg tgcaagcttg aggtgtccaa cttccagcag 200
ccgtacatcg tcaaccgcac ctttatgctg gccaaggagg ccagccttgc 250
agataacaac acagacgtcc ggctcatcgg ggagaaactg ttccgaggag 300
tcagtgctaa agatcagtgc tacctgatga agcaggtgct caacttcacc 350
ctggaagacg ttctgctccc ccagtcagac aggttccagc cctacatgca 400
ggaggtggta cctttcctga ccaaactcag caatcagctc agctcctgtc 450
acatcagcgg tgacgaccag aacatccaga agaatgtcag aaggctgaag 500
gagacagtga aaaagcttgg agagagtgga gagatcaagg cgattgggga 550
actggacctg ctgtttatgt ctctgagaaa tgcttgcgtc tgagcgagaa 600
gaagctagaa aacgaagaac tgctccttcc tgccttctaa aaagaacaat 650
aagatccctg aatggacttt tttactaaag gaaagtgaga agctaacgtc 700
catcatcatt agaagatttc acatgaaacc tggctcagtt gaaaaagaaa 750
atagtgtcaa gttgtccatg agaccagagg tagacttgat aaccacaaag 800
attcattgac aatattttat tgtcactgat gatacaacag aaaaataatg 850
tactttaaaa aattgtttga aaggaggtta cctctcattc ctttagaaaa 900
aaagcttatg taacttcatt tccatatcca atattttata tatgtaagtt 950
tatttattat aagtatacat tttatttatg tcagtttatt aatatggatt 1000
tatttataga aacattatct gctattgata tttagtataa ggcaaataat 1050
atttatgaca ataactatgg aaacaagata tcttaggctt taataaacac 1100
atggatatca taaaaaaaaa a 1121

<210> 34
<211> 179
<212> PRT
<213> Mus musculus

<400> 34

```
Met Ala Val Leu Gln Lys Ser Met Ser Phe Ser Leu Met Gly Thr
1               5                   10                  15

Leu Ala Ala Ser Cys Leu Leu Leu Ile Ala Leu Trp Ala Gln Glu
                20                  25                  30

Ala Asn Ala Leu Pro Val Asn Thr Arg Cys Lys Leu Glu Val Ser
                35                  40                  45

Asn Phe Gln Gln Pro Tyr Ile Val Asn Arg Thr Phe Met Leu Ala
                50                  55                  60

Lys Glu Ala Ser Leu Ala Asp Asn Asn Thr Asp Val Arg Leu Ile
                65                  70                  75

Gly Glu Lys Leu Phe Arg Gly Val Ser Ala Lys Asp Gln Cys Tyr
                80                  85                  90

Leu Met Lys Gln Val Leu Asn Phe Thr Leu Glu Asp Val Leu Leu
                95                  100                 105

Pro Gln Ser Asp Arg Phe Gln Pro Tyr Met Gln Glu Val Val Pro
                110                 115                 120

Phe Leu Thr Lys Leu Ser Asn Gln Leu Ser Ser Cys His Ile Ser
                125                 130                 135

Gly Asp Asp Gln Asn Ile Gln Lys Asn Val Arg Arg Leu Lys Glu
                140                 145                 150

Thr Val Lys Lys Leu Gly Glu Ser Gly Glu Ile Lys Ala Ile Gly
                155                 160                 165

Glu Leu Asp Leu Leu Phe Met Ser Leu Arg Asn Ala Cys Val
                170                 175
```

<210> 35
<211> 1359
<212> DNA
<213> Mus musculus

<400> 35

cgcttagaag tcggactaca gagttagact cagaaccaaa ggaggtggat 50
aggggtcca caggcctggt gcagatcaca gagccagcca gatctgagaa 100
gcagggaaca agatgctgga ttgcagagca gtaataatgc tatggctgtt 150
gccctgggtc actcagggcc tggctgtgcc taggagtagc agtcctgact 200
gggctcagtg ccagcagctc tctcggaatc tctgcatgct agcctggaac 250
gcacatgcac cagcgggaca tatgaatcta ctaagagaag aagaggatga 300
agagactaaa aataatgtgc cccgtatcca gtgtgaagat ggttgtgacc 350
cacaaggact caaggacaac agccagttct gcttgcaaag gatccgccaa 400
ggtctggctt tttataagca cctgcttgac tctgacatct tcaaagggga 450
gcctgctcta ctccctgata gccccatgga gcaacttcac acctccctac 500
taggactcag ccaactcctc cagccagagg atcacccccg ggagacccaa 550
cagatgccca gcctgagttc tagtcagcag tggcagcgcc cccttctccg 600
ttccaagatc cttcgaagcc tccaggcctt tttggccata gctgcccggg 650
tctttgccca cggagcagca actctgactg agcccttagt gccaacagct 700
taaggatgcc caggttccca tggctaccat gataagacta atctatcagc 750
ccagacatct accagttaat taacccatta ggacttgtgc tgttcttgtt 800
ttgtttgttt tgcgtgaagg gcaaggacac cattattaaa gagaaaagaa 850
acaaacccca gagcaggcag ctggctagag aaaggagctg gagaagaaga 900
ataaagtctc gagcccttgg ccttggaagc gggcaagcag ctgcgtggcc 950
tgaggggaag gggcggtgg catcgagaaa ctgtgagaaa acccagagca 1000
tcagaaaaag tgagcccagg ctttggccat tatctgtaag aaaaacaaga 1050
aaaggggaac attatacttt cctgggtggc tcagggaaat gtgcagatgc 1100
acagtactcc agacagcagc tctgtacctg cctgctctgt ccctcagttc 1150
taacagaatc tagtcactaa gaactaacag gactaccaat acgaactgac 1200
aaatactacc actatgacct gtgacaaagc tgcatattta ttaagtggga 1250
agggaacttt tgatattatt tatccttgta acagtataga tgatggttat 1300
ttattctatt tataaggaat tatgtatttt ttttttcaat aaagatttat 1350
ttatgtggc 1359

<210> 36
<211> 196
<212> PRT
<213> Mus musculus

<400> 36

```
Met Leu Asp Cys Arg Ala Val Ile Met Leu Trp Leu Leu Pro Trp
1               5                   10                  15

Val Thr Gln Gly Leu Ala Val Pro Arg Ser Ser Ser Pro Asp Trp
                20                  25                  30

Ala Gln Cys Gln Gln Leu Ser Arg Asn Leu Cys Met Leu Ala Trp
                35                  40                  45

Asn Ala His Ala Pro Ala Gly His Met Asn Leu Leu Arg Glu Glu
                50                  55                  60

Glu Asp Glu Glu Thr Lys Asn Asn Val Pro Arg Ile Gln Cys Glu
                65                  70                  75

Asp Gly Cys Asp Pro Gln Gly Leu Lys Asp Asn Ser Gln Phe Cys
                80                  85                  90

Leu Gln Arg Ile Arg Gln Gly Leu Ala Phe Tyr Lys His Leu Leu
                95                  100                 105

Asp Ser Asp Ile Phe Lys Gly Glu Pro Ala Leu Leu Pro Asp Ser
                110                 115                 120

Pro Met Glu Gln Leu His Thr Ser Leu Leu Gly Leu Ser Gln Leu
                125                 130                 135

Leu Gln Pro Glu Asp His Pro Arg Glu Thr Gln Gln Met Pro Ser
                140                 145                 150

Leu Ser Ser Ser Gln Gln Trp Gln Arg Pro Leu Leu Arg Ser Lys
                155                 160                 165

Ile Leu Arg Ser Leu Gln Ala Phe Leu Ala Ile Ala Ala Arg Val
                170                 175                 180

Phe Ala His Gly Ala Ala Thr Leu Thr Glu Pro Leu Val Pro Thr
                185                 190                 195

Ala
```

<210> 37
<211> 759
<212> DNA
<213> Mus musculus

<400> 37

acaccatcca gatctctgga agacagacaa gatgctgcct ccaacagcct 50

gctccgtcat gtcctggatg ctgctctcct gcctgatgct cttatctcag 100

gttcaaggtg aagactccct gaagaatata ccctccgcac gcattagttg 150

ccccaagggc tcccaggctt atggctccta ctgctatgcc ttgtttcaga 200

taccacagac ctggtttgat gcagaactgg cctgccaaaa gaggcctgga 250

ggacacctcg tatctgtgct caatagcgct gaggcttcat tcttgtcctc 300

catggtgaag agaacaggaa acagctacca atacacttgg attgggctcc 350

atgaccccac tctgggtgca gaacccaatg gaggtggatg ggaatggagt 400

aacaatgacg tgatgaatta ctttaactgg gagaggaacc catctactgc 450

cttagaccgt gctttctgtg gcagcttgtc aagagcttct ggatttctaa 500

aatggagaga tatgacatgt gaggtgaagt tgccctatgt ctgcaaattt 550

actggttaaa cttatcagac agcaaacatc ccgaatttgt cttgaagagc 600

atcatggaca agggacaaaa tgtgaagact cacctagaaa aagcattttc 650

tatctacagt ccacattaga gccttaatct gctctttcca tatctgtctt 700

tagtcctttt ggtataagtt tgggctcaat tctaaaataa aaataagctt 750

tctgtcaca 759

<210> 38
<211> 175
<212> PRT
<213> Mus musculus

<400> 38

Met Leu Pro Pro Thr Ala Cys Ser Val Met Ser Trp Met Leu Leu
1               5                   10                  15

Ser Cys Leu Met Leu Leu Ser Gln Val Gln Gly Glu Asp Ser Leu
                20                  25                  30

Lys Asn Ile Pro Ser Ala Arg Ile Ser Cys Pro Lys Gly Ser Gln
                35                  40                  45

Ala Tyr Gly Ser Tyr Cys Tyr Ala Leu Phe Gln Ile Pro Gln Thr
                50                  55                  60

Trp Phe Asp Ala Glu Leu Ala Cys Gln Lys Arg Pro Gly Gly His
                65                  70                  75

Leu Val Ser Val Leu Asn Ser Ala Glu Ala Ser Phe Leu Ser Ser
                80                  85                  90

Met Val Lys Arg Thr Gly Asn Ser Tyr Gln Tyr Thr Trp Ile Gly
                95                  100                 105

Leu His Asp Pro Thr Leu Gly Ala Glu Pro Asn Gly Gly Gly Trp

```
                    110                 115                 120

Glu Trp Ser Asn Asn Asp Val Met Asn Tyr Phe Asn Trp Glu Arg
                125                 130                 135

Asn Pro Ser Thr Ala Leu Asp Arg Ala Phe Cys Gly Ser Leu Ser
                140                 145                 150

Arg Ala Ser Gly Phe Leu Lys Trp Arg Asp Met Thr Cys Glu Val
                155                 160                 165

Lys Leu Pro Tyr Val Cys Lys Phe Thr Gly
                170                 175
```

<210> 39
<211> 769
<212> DNA
<213> Mus musculus

<400> 39

```
aagacacctt ggtctcagcc tgcagagatc gttgagttgc atcctaagca 50
aaagactgtc tgctgctcag catggctagg aacgcctact tcatcctgct 100
ctcatgcctg atcgtcctgt ctccaagcca aggccaggaa gctgaagaag 150
acctgccatc tgccaggatc agttgcccag aaggttccaa tgcctacagc 200
tcctattgtt actacttcac tgaagaccgt ttaacttggg ctgatgcaga 250
tctttttgc cagaacatga attcaggcta cctggtgtca gttctcagtc 300
aggctgaggg caactttgtg gcctctctga ttaaggagag tggcactaca 350
gacgccaatg tctggactgg actccatgat cccaaaagga atcgtcgctg 400
gcactggagc agtgggtctc tgtttctcta caaatcctgg gcaactgggt 450
ctcctaacag ttccaatcgt ggctactgtg tatctctgac ttcaaacaca 500
ggatacaaga aatggaagga tgacaactgt gatgcccaat actcatttgt 550
ctgcaagttc aaaggctgaa gtcacctgaa aaaaatagt catataaagc 600
aaaattgaaa ttactatagt gtcagaaatt aaattggacc atctatcaaa 650
agcaaattag atcctgtctt cctggagaga cattcttgct tcactgtcct 700
atggtacctg tatctccatt attttctgga aatttgcaca actgaaataa 750
aaacaccttt  acaatgttg 769
```

<210> 40
<211> 165
<212> PRT
<213> Mus musculus

<400> 40

```
Met Ala Arg Asn Ala Tyr Phe Ile Leu Leu Ser Cys Leu Ile Val
1               5                   10                  15

Leu Ser Pro Ser Gln Gly Gln Glu Ala Glu Glu Asp Leu Pro Ser
                20                  25                  30

Ala Arg Ile Ser Cys Pro Glu Gly Ser Asn Ala Tyr Ser Ser Tyr


                35                  40                  45

Cys Tyr Tyr Phe Thr Glu Asp Arg Leu Thr Trp Ala Asp Ala Asp
                50                  55                  60

Leu Phe Cys Gln Asn Met Asn Ser Gly Tyr Leu Val Ser Val Leu
                65                  70                  75

Ser Gln Ala Glu Gly Asn Phe Val Ala Ser Leu Ile Lys Glu Ser
                80                  85                  90

Gly Thr Thr Asp Ala Asn Val Trp Thr Gly Leu His Asp Pro Lys
                95                  100                 105

Arg Asn Arg Arg Trp His Trp Ser Ser Gly Ser Leu Phe Leu Tyr
                110                 115                 120

Lys Ser Trp Ala Thr Gly Ser Pro Asn Ser Ser Asn Arg Gly Tyr
                125                 130                 135

Cys Val Ser Leu Thr Ser Asn Thr Gly Tyr Lys Lys Trp Lys Asp
                140                 145                 150

Asp Asn Cys Asp Ala Gln Tyr Ser Phe Val Cys Lys Phe Lys Gly
                155                 160                 165
```

<210> 41
<211> 692
<212> DNA
<213> Mus musculus

<400> 41

agtattcatt attcctagct gacagaaatt attgatttag aatttaaatc 50

gaagactgtc tgctgatcag catggctcag aacaatgtat accttatcct 100

gttcttatgc ctgatgttcc tgtcatacag ccaaggccag gtagctgaag 150

aagacttccc cttggctgaa aaagaccttc cttctgccaa aatcaactgc 200

ccagagggtg ccaacgccta tggttcctac tgttattatc taattgaaga 250

ccgtttgacc tgggggaggg ctgatctctt ttgccagaac atgaatgcag 300

gtcacctggt gtcaatactc agccaggctg agagcaactt tgtggcctcg 350

ctggttaagg agagtggtac tacagcttcc aatgtctgga ctggacttca 400

tgaccctaaa agtaaccgtc gttggcactg gagcagtggc tccctatttc 450

tcttcaagtc atgggccact ggagctccaa gcactgccaa ccgtggttat 500

tgtgtatcgc tgacttcaaa cacagcatac aaaaaatgga aggacgaaaa 550

ctgtgaggca cagtactcct ttgtctgcaa gttcagagcc taaagtcacc 600

tgaagaacag atgtgcagaa ctctgttact atatattata aatgaaagaa 650

gaccatctat ctgcaaaata aatactcttt cctgtaataa ct 692

<210> 42
<211> 173
<212> PRT
<213> Mus musculus

<400> 42

```
Met Ala Gln Asn Asn Val Tyr Leu Ile Leu Phe Leu Cys Leu Met
1               5                   10                  15

Phe Leu Ser Tyr Ser Gln Gly Gln Val Ala Glu Glu Asp Phe Pro
                20                  25                  30

Leu Ala Glu Lys Asp Leu Pro Ser Ala Lys Ile Asn Cys Pro Glu
                35                  40                  45

Gly Ala Asn Ala Tyr Gly Ser Tyr Cys Tyr Tyr Leu Ile Glu Asp
                50                  55                  60

Arg Leu Thr Trp Gly Glu Ala Asp Leu Phe Cys Gln Asn Met Asn
                65                  70                  75

Ala Gly His Leu Val Ser Ile Leu Ser Gln Ala Glu Ser Asn Phe
                80                  85                  90

Val Ala Ser Leu Val Lys Glu Ser Gly Thr Thr Ala Ser Asn Val
                95                  100                 105

Trp Thr Gly Leu His Asp Pro Lys Ser Asn Arg Arg Trp His Trp
                110                 115                 120

Ser Ser Gly Ser Leu Phe Leu Phe Lys Ser Trp Ala Thr Gly Ala
                125                 130                 135

Pro Ser Thr Ala Asn Arg Gly Tyr Cys Val Ser Leu Thr Ser Asn
                140                 145                 150

Thr Ala Tyr Lys Lys Trp Lys Asp Glu Asn Cys Glu Ala Gln Tyr
                155                 160                 165

Ser Phe Val Cys Lys Phe Arg Ala
                170
```

<210> 43
<211> 837
<212> DNA
<213> Mus musculus

<400> 43

gcccttcctt caaatcctat cataaagcag tcacctttgt cctgacaaac 50

catctcagat ctctacaaga gagacaagat gctgcctcac ctggtcctca 100

acagtatttc ctggatgctg ctctcctgcc tgttgtttgt atttcaggta 150

caaggtgaag acttccagaa ggaagtgccc tctccacgta ccagctgccc 200

catgggttac aaggcttatc gctcccactg ctatgcctta gttatgacac 250

ctaaatcctg gtttcaagca gatctggtct gccagaagag accctcagga 300

catctcgtgt ctattcttag tggaggtgag gcttcctttg tgtcctcctt 350

ggtgaacggc agagtggaca actaccaaga catctggatt gggctccatg 400

atccaacaat gggtcaacaa cccaatggag gtggatggga gtggagtaac 450

tccgatgtgc tgaattatct taactgggat ggggatcctt cctctactgt 500

caaccgtggt cactgtggga gtctgacagc aagttcaggg tttctgaagt 550

ggggagacta ttactgcgat gggacattac catttgtctg caagttcaag 600

cagtagacaa gcagcatcca gcatttatca tgaagctccc catgacaagg 650

gatgaaatac aagaattcac ccggcaaggc tgtacttgct ttacagttgt 700

gcatcagact tattctggtt ttctgtcctc tttcatccat ctccttcccc 750

ttacttcagg cttttcaata tagttcctgc tttgcaatct tgcagataaa 800

taataaatac aacattttgg ttttactttt gtgtttt 837

<210> 44
<211> 175
<212> PRT
<213> Mus musculus

<400> 44

Met Leu Pro His Leu Val Leu Asn Ser Ile Ser Trp Met Leu Leu
1 5 10 15

Ser Cys Leu Leu Phe Val Phe Gln Val Gln Gly Glu Asp Phe Gln
20 25 30

Lys Glu Val Pro Ser Pro Arg Thr Ser Cys Pro Met Gly Tyr Lys
35 40 45

Ala Tyr Arg Ser His Cys Tyr Ala Leu Val Met Thr Pro Lys Ser
50 55 60

Trp Phe Gln Ala Asp Leu Val Cys Gln Lys Arg Pro Ser Gly His
65 70 75

Leu Val Ser Ile Leu Ser Gly Gly Glu Ala Ser Phe Val Ser Ser
80 85 90

Leu Val Asn Gly Arg Val Asp Asn Tyr Gln Asp Ile Trp Ile Gly
95 100 105

Leu His Asp Pro Thr Met Gly Gln Gln Pro Asn Gly Gly Gly Trp
110 115 120

Glu Trp Ser Asn Ser Asp Val Leu Asn Tyr Leu Asn Trp Asp Gly
125 130 135

Asp Pro Ser Ser Thr Val Asn Arg Gly His Cys Gly Ser Leu Thr
140 145 150

Ala Ser Ser Gly Phe Leu Lys Trp Gly Asp Tyr Tyr Cys Asp Gly
155 160 165

Thr Leu Pro Phe Val Cys Lys Phe Lys Gln
170 175

<210> 45
<211> 788
<212> DNA
<213> Mus musculus

<400> 45

cccggggcga aatcacctct gagctgtcaa agcattgcag acctctgtat 50

agacagatat accatggtgt ctcacaagac ccttcatagc atgtcctgga 100

tgctactgtg ttgcctgatg tccctttctt gggtacaagg ggaacaatcc 150

cagaaaaaac tgtcttctcc acgcatcagc tgtccccaag aagcccaagc 200

ttatggctcc tattgctatt tactgattct ggaaccacag acctgggcta 250

atgcagagat ccactgccag aagcatttct caggacacct ggcatttctg 300

ctcacttatg gggaaattat ctttgtgtcc tctctggtga aaaacagttt 350

gaccacattc ccatacatct ggattggact ccatgatctg tcacttggga 400

gtttgcccaa tgaaaatgga tggaagtgga gcagctctga ccctctgacc 450

ttctataact gggagatccc accctccatg tctgcacacc acggttactg 500

cgcagctttg tctcaggcct caggttatca gaagtggaga gattattatt 550

gtgacccaac atttccctat gtctgcaaat tcaagggtta ggccagttct 600

gatttcaact gcctgaaagt atcctgaaga tcacatagac aaaggagcga 650

gcatgatggc tcaccaagaa agtccttctc acaccccgac accgaattcc 700

tcatctcatc tctgctgttc ttccataagt gtattctctg gggactctgg 750

cctaaggatt cggagaacta taataaaatt tagtcaat 788

<210> 46
<211> 175
<212> PRT
<213> Mus musculus

<400> 46

```
Met Val Ser His Lys Thr Leu His Ser Met Ser Trp Met Leu Leu
1               5                   10                  15

Cys Cys Leu Met Ser Leu Ser Trp Val Gln Gly Glu Gln Ser Gln
                20                  25                  30

Lys Lys Leu Ser Ser Pro Arg Ile Ser Cys Pro Gln Glu Ala Gln
                35                  40                  45

Ala Tyr Gly Ser Tyr Cys Tyr Leu Leu Ile Leu Glu Pro Gln Thr
                50                  55                  60

Trp Ala Asn Ala Glu Ile His Cys Gln Lys His Phe Ser Gly His
                65                  70                  75

Leu Ala Phe Leu Leu Thr Tyr Gly Glu Ile Ile Phe Val Ser Ser
                80                  85                  90

Leu Val Lys Asn Ser Leu Thr Thr Phe Pro Tyr Ile Trp Ile Gly
                95                  100                 105

Leu His Asp Leu Ser Leu Gly Ser Leu Pro Asn Glu Asn Gly Trp
                110                 115                 120

Lys Trp Ser Ser Ser Asp Pro Leu Thr Phe Tyr Asn Trp Glu Ile
                125                 130                 135

Pro Pro Ser Met Ser Ala His His Gly Tyr Cys Ala Ala Leu Ser
                140                 145                 150

Gln Ala Ser Gly Tyr Gln Lys Trp Arg Asp Tyr Tyr Cys Asp Pro
                155                 160                 165

Thr Phe Pro Tyr Val Cys Lys Phe Lys Gly
            170                 175
```

<210> 47
<211> 1024
<212> DNA
<213> Mus musculus

<400> 47

ccgactcaag ctgaaaaagg cagggttctc ggagtgttgc ttccaatcag 50
attgcaggtc taggagaatc cacaaaaaag agaagaaaag aaaagaaaaa 100
aactggaacg ggctctgagg gccttgaaat caaagcattg aagtatcatc 150
tccatcgaaa gaggaagatg gcttacaaag gcgtgcggct actcttactg 200
ctgagctggg tagctggccc cgaagtcctg agcgatatct tgagacccag 250
ctgtgcccca ggatggtttt actataggtc ccactgctat ggatacttcc 300
ggaagctaag aaactggtct catgctgagc tggagtgtca gtcatatgga 350
aatggatccc atctggcatc tgtcttgaat caaaaggaag ccagtgtcat 400
atcaaagtac ataactggct atcagagaaa cctgcctgtg tggattggcc 450
tgcatgaccc acaaaagaag caattatggc agtggactga tgggtctaca 500
aacctgtaca gacgctggaa tcccaggaca aagagtgaag ccaggcattg 550
cgctgagatg aaccccaagg ataaattctt aacttggaac aaaaatggat 600
gtgccaaccg ccaacacttc ctgtgcaagt ataagacata gagcaaaaat 650
caagcgtcta ccagccttgc acaaactctt cccacttccc tctcacctgg 700
tggctgatct aatcattatc ccagagtaaa cactgtagca aacattgagg 750
aggcctccag ggcactggct atcaagccct gcttagcatg gtgggacagt 800
ggcttccggt ctcagagttt agcatggtgg gacggtgact tccggtctca 850
gagattagca tggtgggaca agggcttctg gtctccgtgt tcactctaca 900
atcctttctg gtactcccct tccctctcat tgtcttaaac agcaatgctt 950
aacaagctag aaatgtgctt tcttgactac tgcgtctctg tcaaaccagt 1000
aaagttttgg agccaagaaa cagc 1024

<210> 48
<211> 157
<212> PRT
<213> Mus musculus

<400> 48

Met Ala Tyr Lys Gly Val Arg Leu Leu Leu Leu Leu Ser Trp Val
1               5                   10                  15

Ala Gly Pro Glu Val Leu Ser Asp Ile Leu Arg Pro Ser Cys Ala
            20                  25                  30

Pro Gly Trp Phe Tyr Tyr Arg Ser His Cys Tyr Gly Tyr Phe Arg

```
                            35                  40                  45

Lys Leu Arg Asn Trp Ser His Ala Glu Leu Glu Cys Gln Ser Tyr
                50                  55                  60

Gly Asn Gly Ser His Leu Ala Ser Val Leu Asn Gln Lys Glu Ala
                65                  70                  75

Ser Val Ile Ser Lys Tyr Ile Thr Gly Tyr Gln Arg Asn Leu Pro
                80                  85                  90

Val Trp Ile Gly Leu His Asp Pro Gln Lys Lys Gln Leu Trp Gln
                95                  100                 105

Trp Thr Asp Gly Ser Thr Asn Leu Tyr Arg Arg Trp Asn Pro Arg
                110                 115                 120

Thr Lys Ser Glu Ala Arg His Cys Ala Glu Met Asn Pro Lys Asp
                125                 130                 135

Lys Phe Leu Thr Trp Asn Lys Asn Gly Cys Ala Asn Arg Gln His
                140                 145                 150

Phe Leu Cys Lys Tyr Lys Thr
                155
```

<210> 49
<211>
<212> DNA
<213> Homo sapiens

<400> 49

```
1   atgacaccac ctgaacgtct cttcctccca agggtgtgtg gcaccaccct acacctcctc
61  cttctggggc tgctgctggt tctgctgcct ggggcccagg ggctccctgg tgttggcctc
121 acaccttcag ctgcccagac tgcccgtcag caccccaaga tgcatcttgc ccacagcacc
181 ctcaaacctg ctgctcacct cattggagac cccagcaagc agaactcact gctctggaga
241 gcaaacacgg accgtgcctt cctccaggat ggtttctcct tgagcaacaa ttctctcctg
301 gtccccacca gtggcatcta cttcgtctac tcccaggtgg tcttctctgg gaaagcctac
361 tctcccaagg ccacctcctc cccactctac ctggcccatg aggtccagct cttctcctcc
421 cagtacccct tccatgtgcc tctcctcagc tcccagaaga tggtgtatcc agggctgcag
481 gaaccctggc tgcactcgat gtaccacggg gctgcgttcc agctcaccca gggagaccag
541 ctatccaccc acacagatgg catcccccac ctagtcctca gccctagtac tgtcttcttt
601 ggagccttcg ctctgtag
```

<210> 50
<211>
<212> PRT
<213> Homo sapiens

<400> 50

```
MTPPERLFLPRVCGTTLHLLLLGLLLVLLPGAQGLPGVGLTPSAAQTARQHPKMHLAHSTLKPAAHLIGDPSK
QNSLLWRANTDRAFLQDGFSLSNNSLLVPTSGIYFVYSQVVFSGKAYSPKATSSPLYLAHEVQLFSSQYPFHV
PLLSSQKMVYPGLQEPWLHSMYHGAAFQLTQGDQLSTHTDGIPHLVLSPSTVFFGAFAL
```

<210> 51
<211>
<212> DNA

<213> Homo sapiens

<400> 51

1 atgggggcac tggggctgga gggcaggggt gggaggctcc aggggagggg ttccctcctg
61 ctagctgtgg caggagccac ttctctggtg accttgttgc tggcggtgcc tatcactgtc
121 ctggctgtgc tggccttagt gccccaggat cagggaggac tggtaacgga gacggccgac
181 cccggggcac aggcccagca aggactgggg tttcagaagc tgccagagga ggagccagaa

241 acagatctca gccccgggct cccagctgcc cacctcatag gcgctccgct gaaggggcag
301 gggctaggct gggagacgac gaaggaacag gcgtttctga cgagcgggac gcagttctcg
361 gacgccgagg ggctggcgct cccgcaggac ggcctctatt acctctactg tctcgtcggc
421 taccggggcc gggcgccccc tggcggcggg gacccccagg gccgctcggt cacgctgcgc
481 agctctctgt accgggcggg gggcgcctac gggccgggca ctcccgagct gctgctcgag
541 ggcgccgaga cggtgactcc agtgctggac ccggccagga gacaagggta cgggcctctc
601 tggtacacga gcgtggggtt cggcggcctg gtgcagctcc ggagggggcga gagggtgtac
661 gtcaacatca gtcaccccga tatggtggac ttcgcgagag ggaagacctt ctttggggcc
721 gtgatggtgg ggtga

<210> 52
<211>
<212> PRT
<213> Homo sapiens

<400> 52

MGALGLEGRGGRLQGRGSLLLAVAGATSLVTLLLAVPITVLAVLALVPQDQGGLVTETADPGAQAQQGLGFQKLPEE
EPETDL
SPGLPAAHLIGAPLKGQGLGWETTKEQAFLTSGTQFSDAEGLALPQDGLYYLYCLVGYRGRAPPGGGDPQGRSVTLR
SSLYRA
GGAYGPGTPELLLEGAETVTPVLDPARRQGYGPLWYTSVGFGGLVQLRRGERVYVNISHPDMVDFARGKTFFGAVMV
G

<210> 53
<211>
<212> DNA
<213> Mus musculus

<400> 53

1 atgacactgc tcggccgtct ccacctcttg agggtgcttg gcacccctcc tgtcttcctc
61 ctggggctgc tgctggccct gcctctaggg gcccagggac tctctggtgt ccgcttctcc
121 gctgccagga cagcccatcc actccctcag aagcacttga cccatggcat cctgaaacct
181 gctgctcacc ttgttgggta ccccagcaag cagaactcac tgctctggag agcaagcacg
241 gatcgtgcct ttctccgaca tggcttctct ttgagcaaca actccctcct gatccccacc
301 agtggcctct actttgtcta ctcccaggtg gttttctctg gagaaagctg ctcccccagg
361 gccattccca ctcccatcta cctggcacac gaggtccagc tcttttcctc ccaatacccc
421 ttccatgtgc ctctcctcag tgcgcagaag tctgtgtatc cgggacttca aggaccgtgg
481 gtgcgctcaa tgtaccaggg ggctgtgttc ctgctcagta agggagacca gctgtccacc
541 cacaccgacg gcatctccca tctacacttc agccccagca gtgtattctt tggagccttt
601 gcactgtag

<210> 54
<211>
<212> PRT
<213> Mus musculus

<400> 54

MTLLGRLHLL RVLGTPPVFL LGLLLALPLG AQGLSGVRFS AARTAHPLPQ KHLTHGILKPAAHLVGYPSK
QNSLLWRAST DRAFLRHGFS LSNNSLLIPT SGLYFVYSQV VFSGESCSPRAIPTPIYLAH EVQLFSSQYP
FHVPLLSAQK SVYPGLQGPW VRSMYQGAVF LLSKGDQLSTHTDGISHLHF SPSSVFFGAFAL

<210> 55
<211>
<212> DNA
<213> Mus musculus

<400> 55

1 atgacactgc tcggccgtct ccacctcttg agggtgcttg gcaccccctcc tgtcttcctc
61 ctggggctgc tgctggccct gcctctaggg gcccagggac tctctggtgt ccgcttctcc
121 gctgccagga cagcccatcc actccctcag aagcacttga cccatggcat cctgaaacct

181 gctgctcacc ttgttgggta ccccagcaag cagaactcac tgctctggag agcaagcacg
241 gatcgtgcct ttctccgaca tggcttctct ttgagcaaca actccctcct gatcccccacc
301 agtggcctct actttgtcta ctcccaggtg gtttctctg gagaaagctg ctccccccagg
361 gccattccca ctcccatcta cctggcacac gaggtccagc tcttttcctc ccaatacccc
421 ttccatgtgc ctctcctcag tgcgcagaag tctgtgtatc cgggacttca aggaccgtgg
481 gtgcgctcaa tgtaccaggg ggctgtgttc ctgctcagta agggagacca gctgtccacc
541 cacaccgacg gcatctccca tctacacttc agccccagca gtgtattctt tggagccttt
601 gcactgtag

<210> 56
<211>
<212> PRT
<213> Mus musculus

<400> 56

MGTRGLQGLG GRPQGRGCLL LAVAGATSLV TLLLAVPITV LAVLALVPQD QGRRVEKIIGSGAQAQKRLD
DSKPSCILPS PSSLSETPDP RLHPQRSNAS RNLASTSQGP VAQSSREASAWMTILSPAAD STPDPGVQQL
PKGEPETDLN PELPAAHLIG AWMSGQGLSW EASQEEAFLRSGAQFSPTHG LALPQDGVYY LYCHVGYRGR
TPPAGRSRAR SLTLRSALYR AGGAYGRGSPELLLEGAETV TPVVDPIGYG SLWYTSVGFG GLAQLRSGER
VYVNISHPDM VDYRRGKTFF GAVMVG

<210> 57
<211>
<212> DNA
<213> Homo sapiens

<400> 57

ATGCTCCTGCCTTGGGCCACCTCTGCCCCCGGCCTGGCCTGGGGGCCTCTGGTGCTGGGCCTCTTCGGGCTCCTGGC
AGC
ATCGCAGCCCCAGGCGGTGCCTCCATATGCGTCGGAGAACCAGACCTGCAGGGACCAGGAAAAGGAATACTATGAGC
CCC
AGCACCGCATCTGCTGCTCCCGCTGCCCGCCAGGCACCTATGTCTCAGCTAAATGTAGCCGCATCCGGGACACAGTT
TGT
GCCACATGTGCCGAGAATTCCTACAACGAGCACTGGAACTACCTGACCATCTGCCAGCTGTGCCGCCCCTGTGACCC
AGT
GATGGGCCTCGAGGAGATTGCCCCCTGCACAAGCAAACGGAAGACCCAGTGCCGCTGCCAGCCGGGAATGTTCTGTG
CTG
CCTGGGCCCTCGAGTGTACACACTGCGAGCTACTTTCTGACTGCCCGCCTGGCACTGAAGCCGAGCTCAAAGATGAA
GTT
GGGAAGGGTAACAACCACTGCGTCCCCTGCAAGGCAGGGCACTTCCAGAATACCTCCTCCCCCAGCGCCCGCTGCCA
GCC
CCACACCAGGTGTGAGAACCAAGGTCTGGTGGAGGCAGCTCCAGGCACTGCCCAGTCCGACACAACCTGCAAAAATC
CAT
TAGAGCCACTGCCCCCAGAGATGTCAGGAGGG

<210> 58
<211>
<212> DNA
<213> Homo sapiens

<400> 58

CGCGCCCAGGTCACCGACAAAGCTGCGCACTATACTCTGTGCCCACCGTGCCCAGCACCTGAACTCCTGGGGGGACC
GTC
AGTCTTCCTCTTCCCCCCAAAACCCAAGGACACCCTCATGATCTCCCGGACCCCTGAGGTCACATGCGTGGTGGTGG
ACG
TGAGCCACGAAGACCCTGAGGTCAAGTTCAACTGGTACGTGGACGGCGTGGAGGTGCATAATGCCAAGACAAAGCCG
CGG
GAGGAGCAGTACAACAGCACGTACCGTGTGGTCAGCGTCCTCACCGTCCTGCACCAGGACTGGCTGAATGGCAAGGA
GTA
CAAGTGCAAGGTCTCCAACAAAGCCCTCCCAGCCCCCATCGAGAAAACCATCTCCAAAGCCAAAGGGCAGCCCCGAG
AAC
CACAGGTGTACACCCTGCCCCCATCCCGGGAAGAGATGACCAAGAACCAGGTCAGCCTGACCTGCCTGGTCAAAGGC

TTC
TATCCCAGCGACATCGCCGTGGAGTGGGAGAGCAATGGGCAGCCGGAGAACAACTACAAGACCACGCCTCCCGTGCT
GGA
CTCCGACGGCTCCTTCTTCCTCTACAGCAAGCTCACCGTGGACAAGAGCAGGTGGCAGCAGGGGAACGTCTTCTCAT
GCT
CCGTGATGCATGAGGCTCTGCACAACCACTACACGCAGAAGAGCCTCTCCCTGTCTCCGGGTAAAT

<210> 59
<211>
<212> DNA
<213> Mus musculus

<400> 59

ATGCGCCTGCCCCGGGCCTCCTCTCCCTGCGGCCTGGCCTGGGGGCCACTCCTGCTGGGACTCAGCGGGCTTCTGGT
GGC
CTCTCAGCCCCAGCTGGTGCCCCCTTATCGCATAGAAAACCAGACTTGCTGGGACCAGGACAAGGAATACTACGAGC
CCA
TGCACGACGTCTGCTGCTCCCGCTGTCCCCCAGGCGAGTTTGTCTTTGCGGTATGCAGCCGCAGCCAAGACACGGTT
TGC
AAGACTTGCCCCCATAATTCCTATAATGAACACTGGAACCATCTCTCCACCTGCCAGCTGTGCCGCCCCTGTGACAT
TGT
GCTGGGCTTTGAGGAGGTTGCCCCTTGCACCAGCGATCGGAAAGCCGAGTGCCGCTGTCAGCCGGGGATGTCCTGTG
TGT
ATCTGGACAATGAGTGTGTGCACTGTGAGGAGGAGCGGCTTGTACTCTGCCAGCCTGGCACAGAAGCCGAGGTCACA
GAT
GAAATTATGGATACTGACGTCAACTGTGTCCCCTGTAAGCCGGGACACTTCCAGAACACTTCCTCCCCTCGAGCCCG
CTG
TCAACCCCATACCAGATGTGAGATCCAGGGCCTGGTGGAGGCAGCTCCAGGTACCTCCTACTCGGATACCATCTGTA
AAA

<210> 60
<211>
<212> DNA
<213> Mus musculus

<400> 60

CTCGAGGACCCACAATCAAGCCCTGTCCTCCATGCAAATGCCCAGCACCTAACCTCTTGGGTGGACCATCCGTCTTC
ATC
TTCCCTCCAAAGATCAAGGATGTACTCATGATCTCCCTGAGCCCCATAGTCACATGTGTGGTGGATGTGAGCGA
GGA
TGACCCAGATGTCCAGATCAGCTGGTTTGTGAACAACGTGGAAGTACACACAGCTCAGACACAAACCCATAGAGAGG
ATT
ACGACAGTACTCTACGCGTGGTCAGTGCCCTCCCCATCCAGCACCAGGACTGGATGAGTGGCAAGGAGTTCAAATGC
AAG
GTCAACAACAAAGACCTCCCAGCGCCCATCGAGAGAACCATCTCAAAACCCAAAGGGTCAGTAAGAGCTCCACAGGT
ATA
TGTCTTGCCTCCACCAGAAGAAGAGATGACTAAGAAACAGGTCACTCTGACCTGCATGGTCACAGACTTCATGCCTG
AAG
ACATTTACGTGGAGTGGACCAACAACGGGAAAACAGAGCTAAACTACAAGAACACTGAACCAGTCCTGGACTCTGAT
GGT
TCTTACTTCATGTACAGCAAGCTGAGAGTGGAAAAGAAGAACTGGGTGGAAAGAAATAGCTACTCCTGTTCAGTGGT
CCA
CGAGGGTCTGCACAATCACCACACGACTAAGAGCTTCTCCCGGACTCCGGGTAAATGAAAGCTTGGCCGCCATGGCC
C

<210> 61
<211>
<212> PRT
<213> Homo sapiens

<400> 61

MKHQHQHQHQHQHQMHQAQTARQHPK-

MHLAHSTLKPAAHLIGDPSKQNSLLWRANTDRAFLQDGFSLSNNSLLVPTSGIYFVYSQVVFSGKAYSPKATSSPLY
LAHE
VQLFSSQYPFHVPLLSSQKMVYPGLQEPWLHSMYHGAAFQLTQGDQLSTHTDGIPHLVLSPSTVFFGAFAL

## Claims

1. A polypeptide comprising IL-22 for use in a method of treating a microbial disorder in a subject, comprising administering to said subject an effective amount of the polypeptide, wherein the microbial disorder is Inflammatory Bowel Disease (IBD).

**2.** An IL-22 agonist which increases IL-22 activity for use in a method of treating a microbial disorder in a subject, comprising administering to said subject an effective amount of the IL-22 agonist, wherein the microbial disorder is IBD and wherein the IL-22 agonist is selected from the group:

(a) an IL-22-Fc fusion polypeptide
(b) an IL-22 nucleic acid;
(c) an IL-22 fusion polypeptide;
(d) an antibody or biologically active fragment thereof, wherein the antibody or fragment binds to IL-22, IL-22R or IL-22BP;
(e) a monoclonal antibody that binds to IL-22, IL-22R or IL-22BP; and
(f) a humanized antibody that binds to IL-22, IL-22R or IL-22BP.

**3.** The polypeptide or IL-22 agonist for use according to claim 1 or 2 wherein the administering is intravenous as a bolus or by continuous infusion over a period of time, intramuscular, intraperitoneal, intracerobrospinal, subcutaneous, intraarticular, intrasynovial, intrathecal, oral, topical, or inhalation.

**4.** The polypeptide or IL-22 agonist for use according to claim 3, wherein the administering is intravenous as a bolus or by continuous infusion over a period of time.

**5.** The polypeptide or IL-22 agonist for use according to any one of the preceding claims, wherein said IBD is Crohn's disease.

**6.** The polypeptide or IL-22 agonist for use according any one of the preceding claims, wherein said IBD is ulcerative colitis.

**7.** A kit for use in a method for treatment of a microbial disorder in a subject, wherein the kit comprises a pharmaceutical composition comprising IL-22, and wherein the microbial disoder is IBD.

**Patentansprüche**

**1.** Polypeptid, das IL-22 umfasst, zur Verwendung in einem Verfahren zur Behandlung einer mikrobiellen Erkrankung bei einem Individuum, wobei das Verfahren das Verabreichen einer wirksamen Menge des Polypeptids an das Individuum umfasst und wobei die mikrobielle Erkrankung entzündliche Darmerkrankung (ED) ist.

**2.** IL-22-Agonist, der die IL-22-Aktivität steigert zur Verwendung in einem Verfahren zur Behandlung einer mikrobiellen Erkrankung bei einem Individuum, wobei das Verfahren das Verabreichen einer wirksamen Menge des IL-22-Agonisten an das Individuum umfasst und wobei die mikrobielle Erkrankung ED ist und wobei der IL-22-Agonist ausgewählt ist aus folgender Gruppe:

(a) einem IL-22-Fc-Fusionspolypeptid;
(b) einer IL-22-Nucleinsäure;
(c) einem IL-22-Fusionspolypeptid;
(d) einem Antikörper oder biologisch aktiven Fragmente davon, wobei der Antikörper oder das Fragment an IL-22, IL-22R oder IL-22BP bindet;
(e) einem monoklonalen Antikörper, der an IL-22, IL-22R oder IL-22BP bindet und
(f) einem humanisierten Antikörper, der an IL-22, IL-22R oder IL-22BP bindet.

**3.** Polypeptid oder IL-Agonist zur Verwendung nach Anspruch 1 oder 2, wobei die Verabreichung intravenös als Bolus oder durch kontinuierliche Infusion über einen Zeitraum hinweg, intramuskulär, intraperitoneal, intrazerebrospinal, subkutan, intraartikulär, intrasynovial, intrathekal, oral, topisch oder mittels Inhalation erfolgt.

**4.** Polypeptid oder IL-Agonist zur Verwendung nach Anspruch 3, wobei die Verabreichung intravenös als Bolus oder durch kontinuierliche Infusion über einen Zeitraum hinweg erfolgt.

5. Polypeptid oder IL-Agonist zur Verwendung nach einem der vorangegangenen Ansprüche, wobei die ED Morbus Crohn ist.

6. Polypeptid oder IL-Agonist zur Verwendung nach einem der vorangegangenen Ansprüche, wobei die ED Colitis ulcerosa ist.

7. Set zur Verwendung in einem Verfahren zur Behandlung einer mikrobiellen Erkrankung bei einem Individuum, wobei das Set eine pharmazeutische Zusammensetzung umfasst, die IL-22 umfasst, und wobei die mikrobielle Erkrankung eine ED ist.

**Revendications**

1. Polypeptide comprenant l'IL-22 pour une utilisation dans une méthode de traitement d'un trouble microbien chez un sujet, comprenant l'administration audit sujet d'une quantité efficace du polypeptide, le trouble microbien étant une maladie inflammatoire de l'intestin (MII).

2. Agoniste d'IL-22 qui augmente d'IL-22 pour une utilisation dans une méthode de traitement d'un trouble microbien chez un sujet, comprenant l'administration audit sujet d'une quantité efficace de l'agoniste d'IL-22, le trouble microbien étant une MII et l'agoniste d'IL-22 étant choisi dans le groupe constitué par :

   (a) un polypeptide de fusion d'IL-22-Fc ;
   (b) un acide nucléique d'IL-22 ;
   (c) un polypeptide de fusion d'IL-22 ;
   (d) un anticorps ou un fragment biologiquement actif de celui-ci, l'anticorps ou le fragment se liant à IL-22, IL-22R ou IL-22BP ;
   (e) un anticorps monoclonal qui se lie à IL-22, IL-22R ou IL-22BP ; et
   (f) un anticorps humanisé qui se lie à IL-22, IL-22R ou IL-22BP.

3. Polypeptide ou agoniste d'IL-22 pour une utilisation selon la revendication 1 ou 2, dans lequel l'administration est une administration intraveineuse sous forme de bolus ou par perfusion continue sur une certaine période de temps, intramusculaire, intrapéritonéale, intracérobrospinale, sous-cutanée, intra-articulaire, intrasynoviale, intrathécale, orale, topique ou par inhalation.

4. Polypeptide ou agoniste d'IL-22 pour une utilisation selon la revendication 3, dans lequel l'administration est une administration intraveineuse sous forme de bolus ou par perfusion continue sur une certaine période de temps.

5. Polypeptide ou agoniste d'IL-22 pour une utilisation selon l'une quelconque des revendications précédentes, dans lequel ladite MII est la maladie de Crohn.

6. Polypeptide ou agoniste d'IL-22 pour une utilisation selon l'une quelconque des revendications précédentes, dans lequel ladite MII est la rectocolite hémorragique.

7. Kit pour une utilisation dans une méthode de traitement d'un trouble microbien chez un sujet, dans lequel le kit comprend une composition pharmaceutique comprenant l'IL-22 et dans lequel le trouble microbien est une MII.

Fig.1

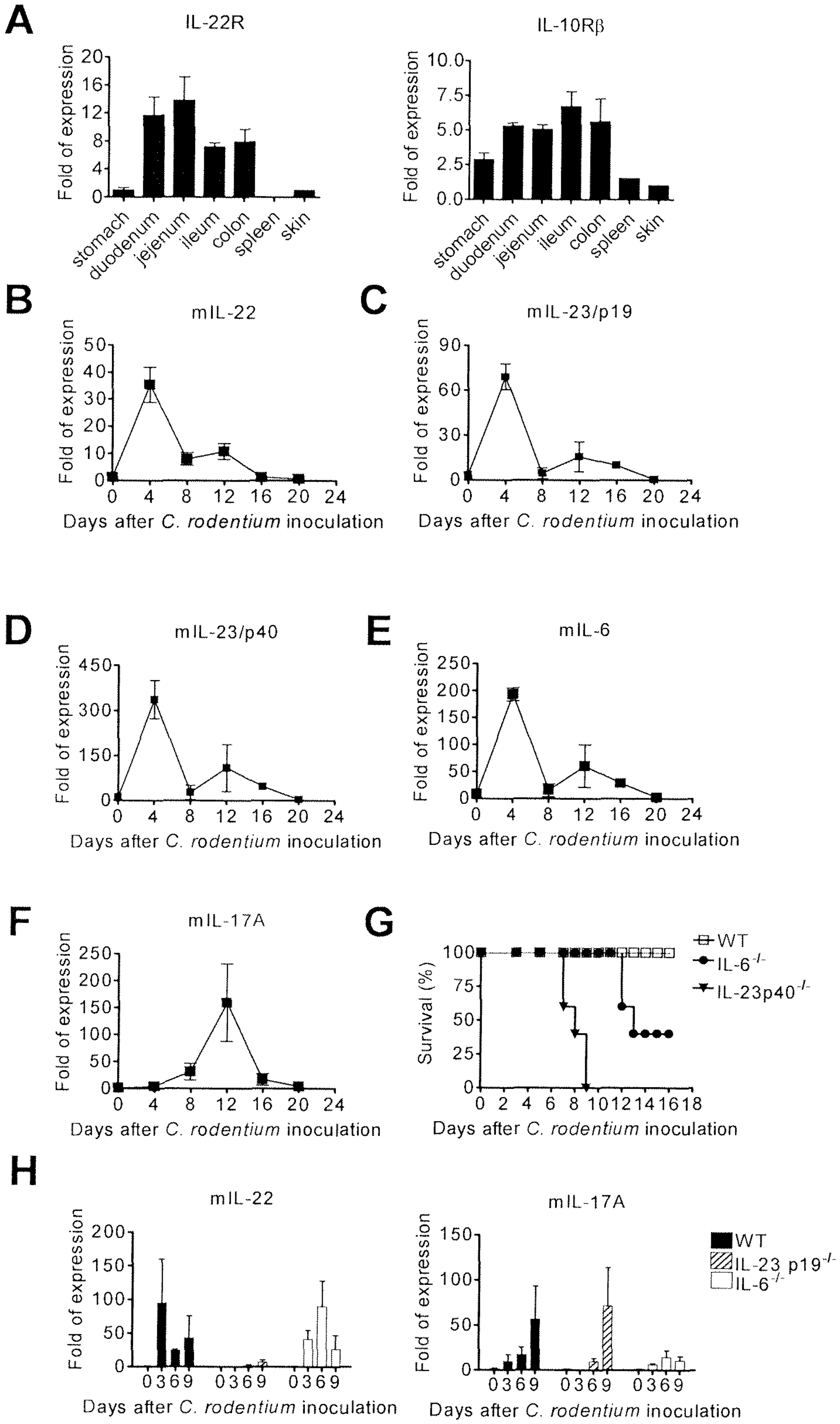
A
IL-22R
IL-10Rβ
Fold of expression
stomach
duodenum
jejenum
ileum
colon
spleen
skin
B
mIL-22
C
mIL-23/p19
D
mIL-23/p40
E
mIL-6
F
mIL-17A
G
Survival (%)
WT
IL-6-/-
IL-23p40-/-
H
mIL-22
mIL-17A
WT
IL-23 p19-/-
IL-6-/-
Days after C. rodentium inoculation

Fig.2

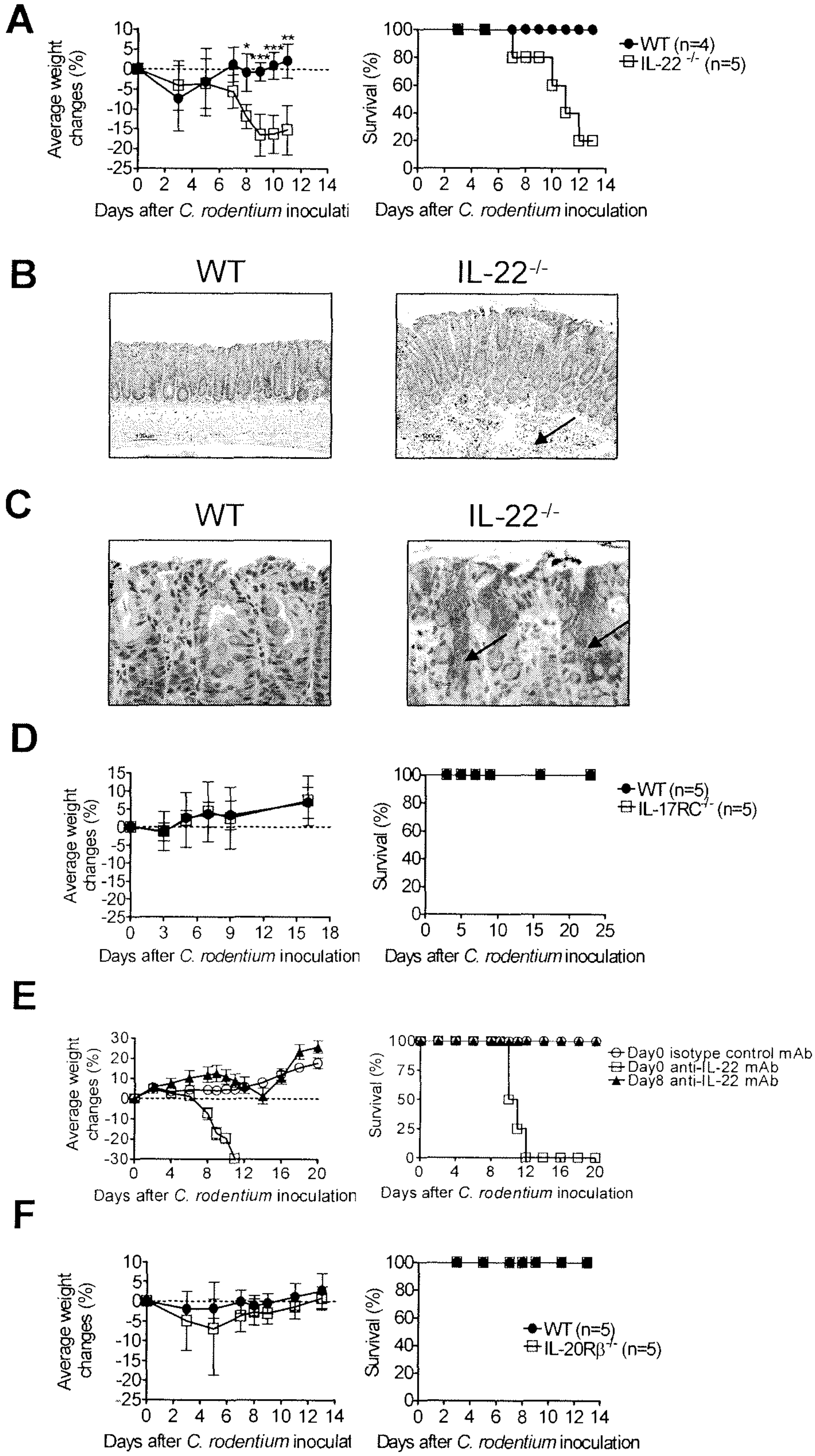
A
Average weight changes (%)
Days after C. rodentium inoculati
Survival (%)
Days after C. rodentium inoculation
WT (n=4)
IL-22 -/- (n=5)
B
WT
IL-22-/-
C
WT
IL-22-/-
D
Average weight changes (%)
Days after C. rodentium inoculation
Survival (%)
Days after C. rodentium inoculation
WT (n=5)
IL-17RC-/- (n=5)
E
Average weight changes (%)
Days after C. rodentium inoculation
Survival (%)
Days after C. rodentium inoculation
Day0 isotype control mAb
Day0 anti-IL-22 mAb
Day8 anti-IL-22 mAb
F
Average weight changes (%)
Days after C. rodentium inoculat
Survival (%)
Days after C. rodentium inoculation
WT (n=5)
IL-20Rβ-/- (n=5)

Fig.3

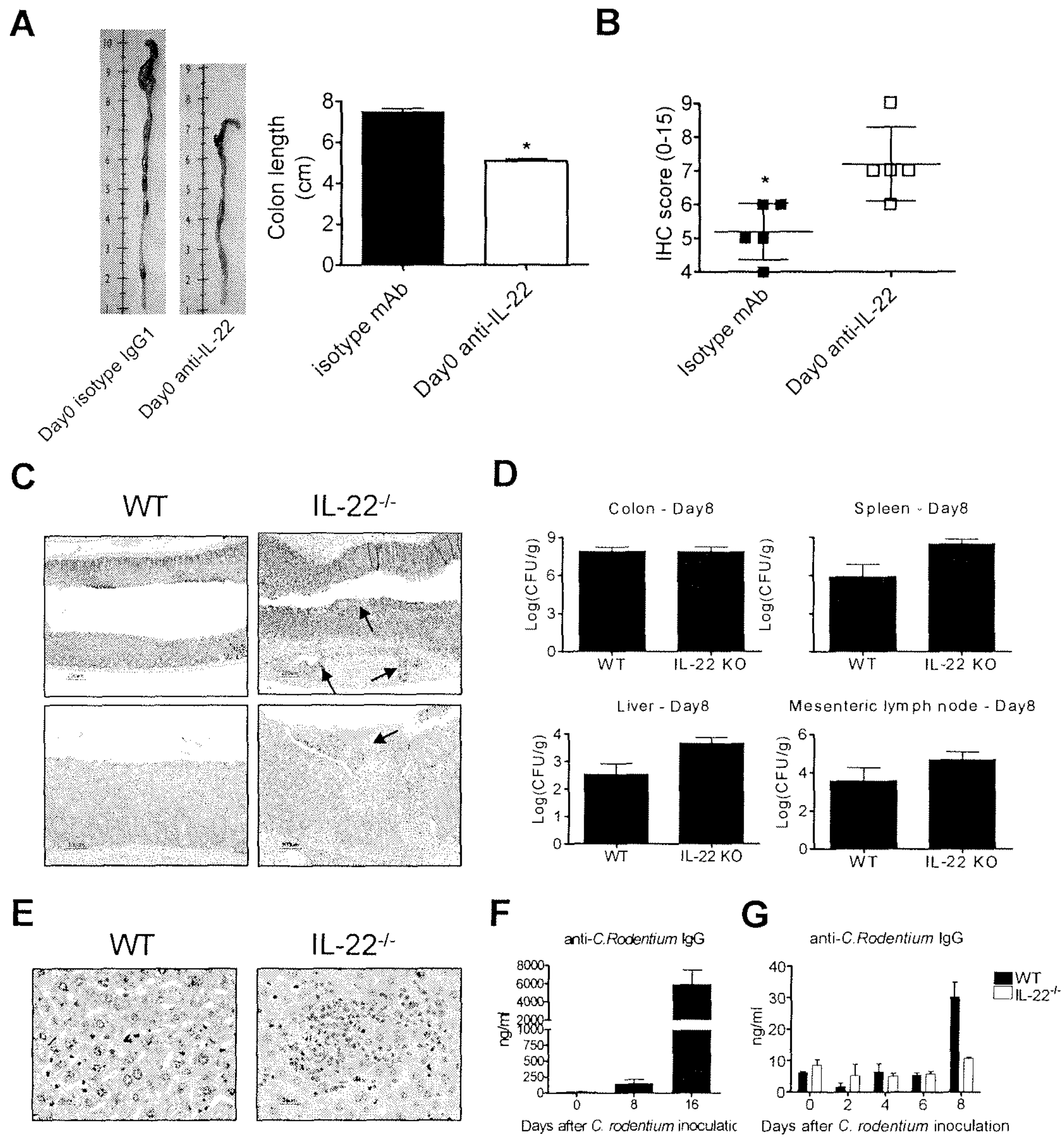
A
Day0 isotype IgG1
Day0 anti-IL-22
Colon length (cm)
isotype mAb
Day0 anti-IL-22
B
IHC score (0-15)
Isotype mAb
Day0 anti-IL-22
C
WT
IL-22-/-
D
Colon - Day8
Spleen - Day8
Liver - Day8
Mesenteric lymph node - Day8
Log(CFU/g)
WT
IL-22 KO
E
WT
IL-22-/-
F
anti-C.Rodentium IgG
ng/ml
Days after C. rodentium inoculatic
G
anti-C.Rodentium IgG
WT
IL-22-/-
ng/ml
Days after C. rodentium inoculation

Fig.4

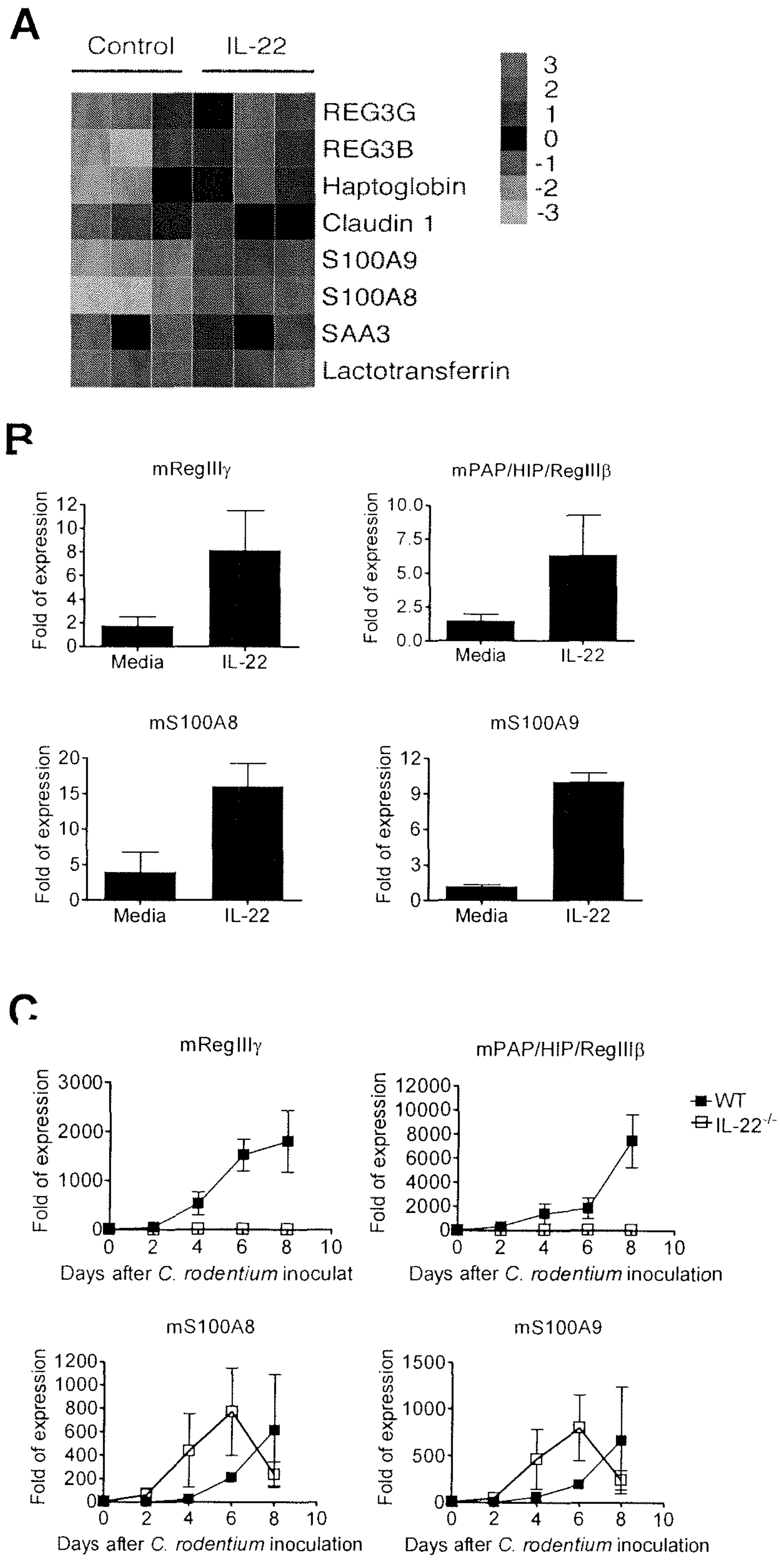
A
Control
IL-22
REG3G
REG3B
Haptoglobin
Claudin 1
S100A9
S100A8
SAA3
Lactotransferrin
3
2
1
0
-1
-2
-3
B
mRegIIIγ
mPAP/HIP/RegIIIβ
mS100A8
mS100A9
Fold of expression
Media
IL-22
C
mRegIIIγ
mPAP/HIP/RegIIIβ
mS100A8
mS100A9
WT
IL-22-/-
Fold of expression
Days after C. rodentium inoculat
Days after C. rodentium inoculation

Fig. 5

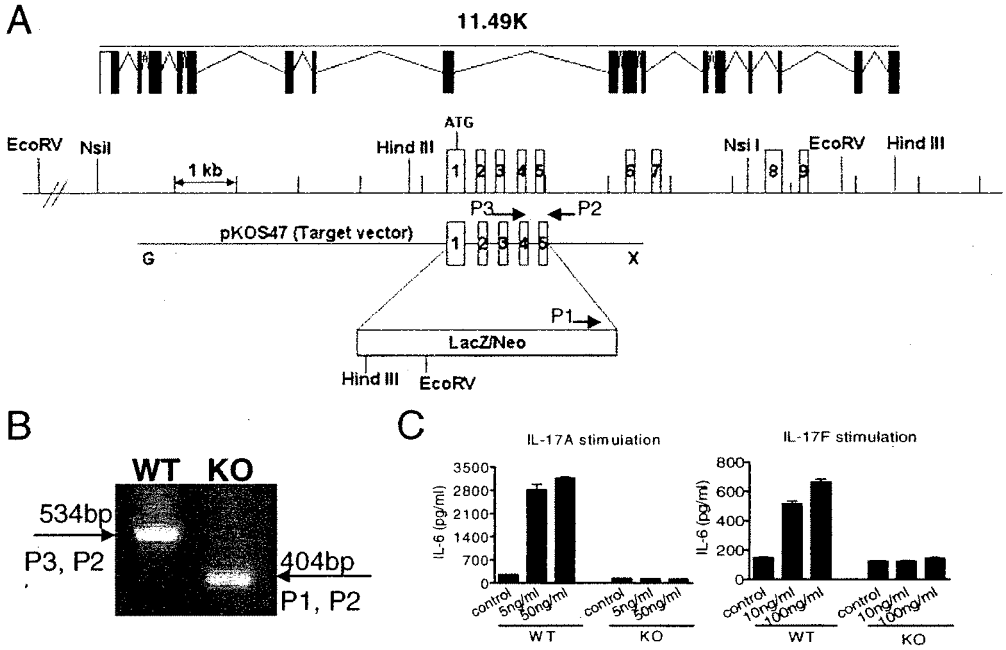
A
11.49K
ATG
EcoRV
NsiI
1 kb
Hind III
Nsi I
EcoRV
Hind III
P3
P2
pKOS47 (Target vector)
G
X
P1
LacZ/Neo
Hind III
EcoRV
B
WT
KO
534bp
P3, P2
404bp
P1, P2
C
IL-17A stimulation
IL-17F stimulation
IL-6 (pg/ml)
control
5ng/ml
50ng/ml
10ng/ml
100ng/ml
WT
KO

Fig. 6

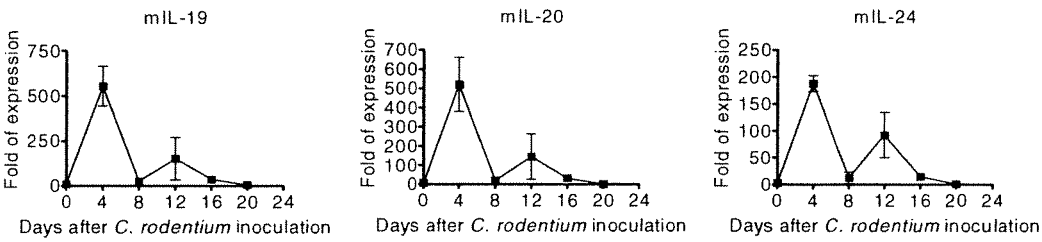
mIL-19
Fold of expression
750
500
250
0
0 4 8 12 16 20 24
Days after *C. rodentium* inoculation
mIL-20
Fold of expression
700
600
500
400
300
200
100
0
0 4 8 12 16 20 24
Days after *C. rodentium* inoculation
mIL-24
Fold of expression
250
200
150
100
50
0
0 4 8 12 16 20 24
Days after *C. rodentium* inoculation

Fig. 7

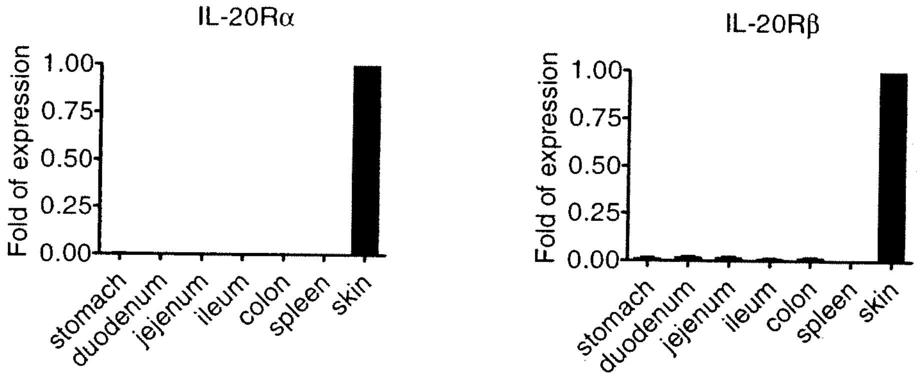
IL-20Rα
Fold of expression
1.00
0.75
0.50
0.25
0.00
stomach
duodenum
jejenum
ileum
colon
spleen
skin
IL-20Rβ
Fold of expression
1.00
0.75
0.50
0.25
0.00
stomach
duodenum
jejenum
ileum
colon
spleen
skin

Fig. 8

A

B

C

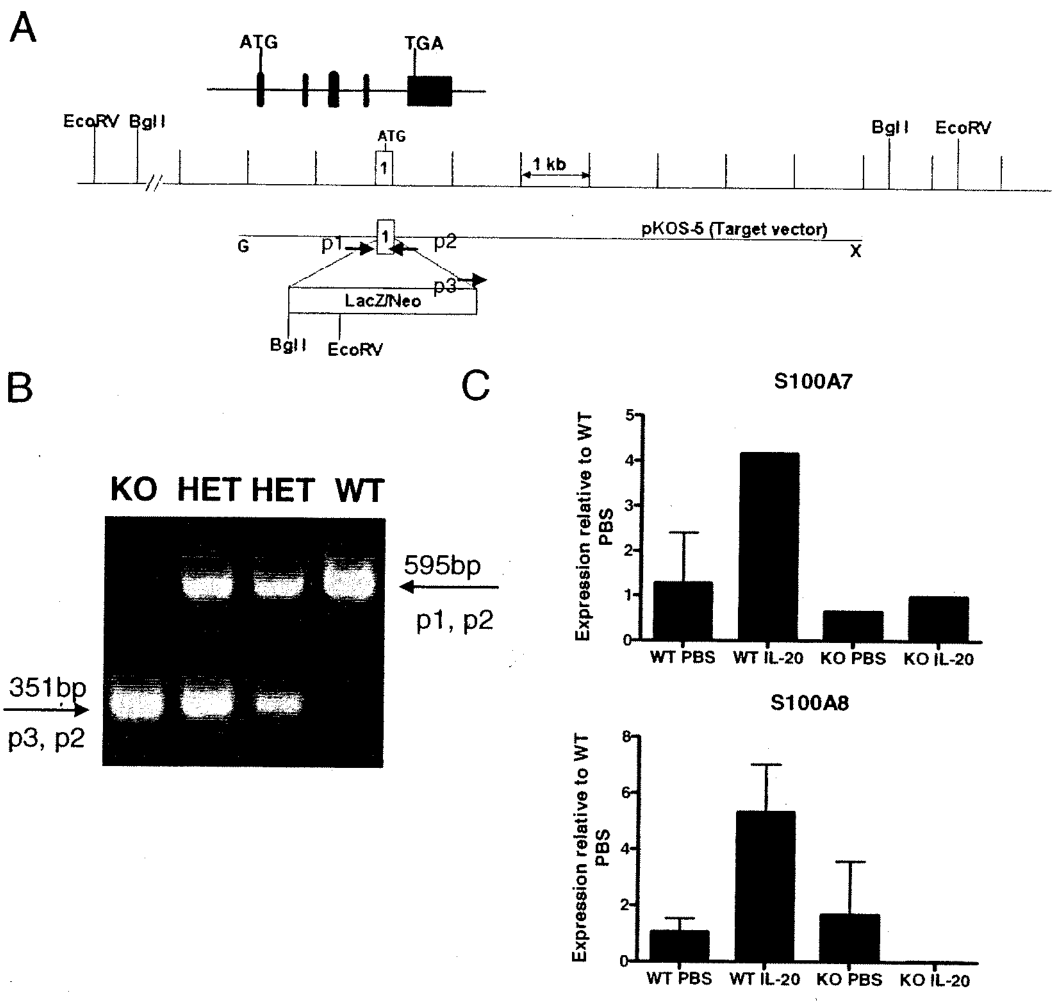
ATG
TGA
EcoRV
BglI
ATG
1
1 kb
BglI
EcoRV
G
p1
1
p2
pKOS-5 (Target vector)
X
p3
LacZ/Neo
BglI
EcoRV
KO
HET
HET
WT
595bp
p1, p2
351bp
p3, p2
S100A7
Expression relative to WT PBS
WT PBS
WT IL-20
KO PBS
KO IL-20
S100A8
Expression relative to WT PBS
WT PBS
WT IL-20
KO PBS
KO IL-20

Fig. 9

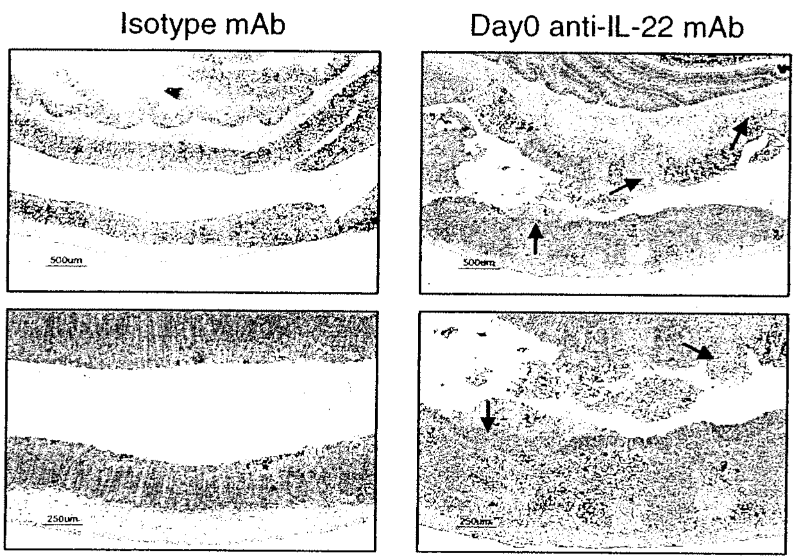

Fig. 10

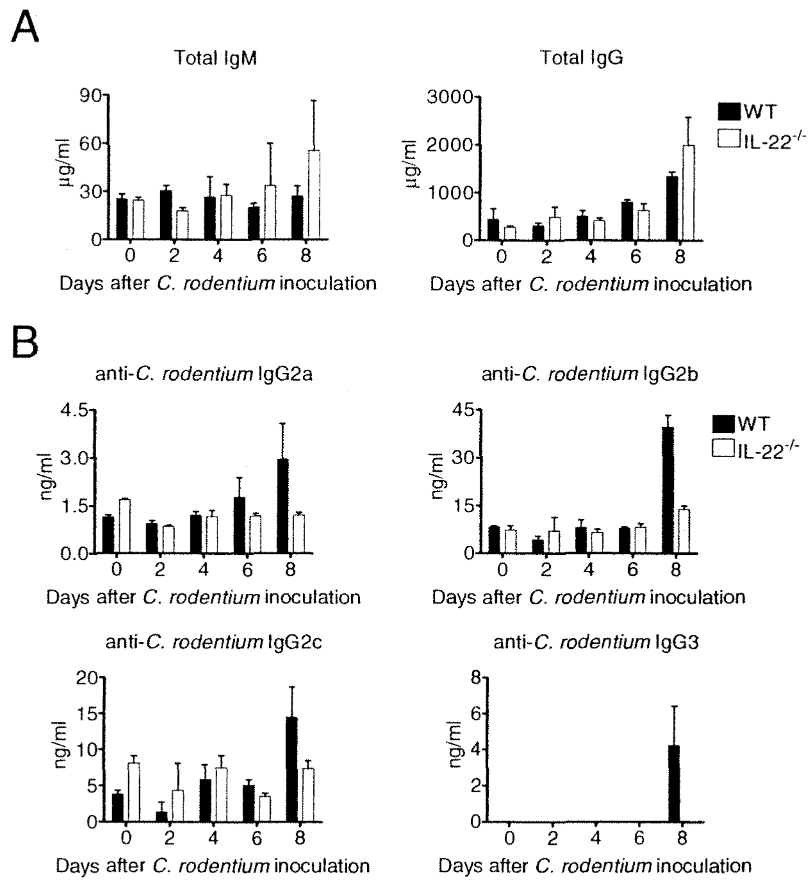
A
Total IgM
μg/ml
Total IgG
WT
IL-22-/-
Days after C. rodentium inoculation
B
anti-C. rodentium IgG2a
anti-C. rodentium IgG2b
anti-C. rodentium IgG2c
anti-C. rodentium IgG3
ng/ml
Days after C. rodentium inoculation

Fig. 11

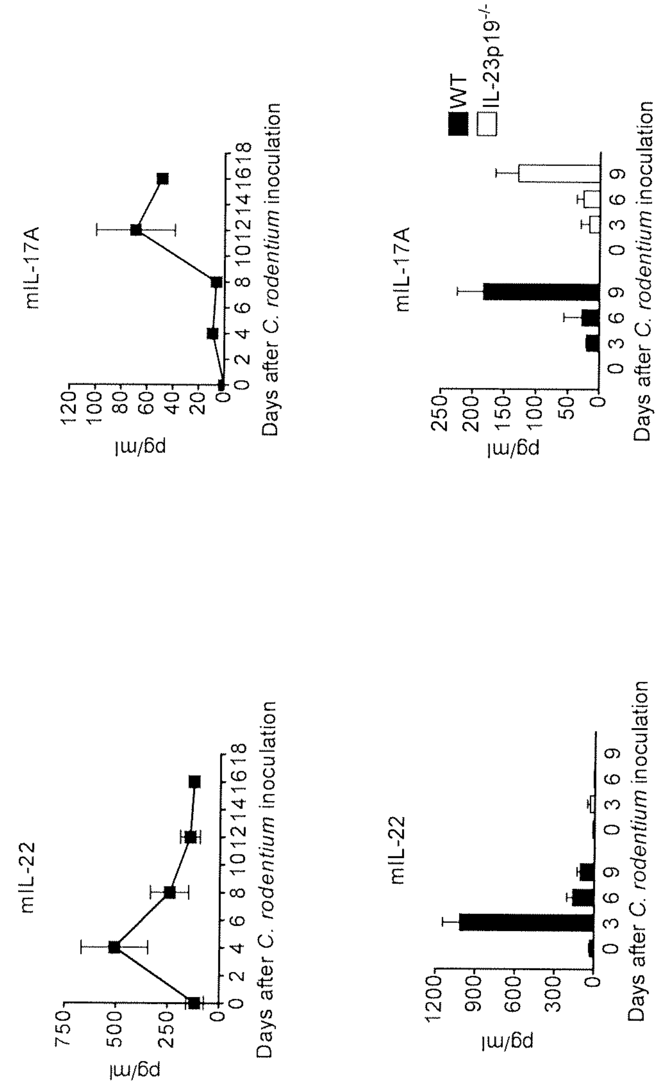
mIL-22
pg/ml
750
500
250
0
0 2 4 6 8 10 12 14 16 18
Days after C. rodentium inoculation
mIL-17A
pg/ml
120
100
80
60
40
20
0
0 2 4 6 8 10 12 14 16 18
Days after C. rodentium inoculation
mIL-22
pg/ml
1200
900
600
300
0
0 3 6 9 0 3 6 9
Days after C. rodentium inoculation
mIL-17A
pg/ml
250
200
150
100
50
0
0 3 6 9 0 3 6 9
Days after C. rodentium inoculation
WT
IL-23p19-/-

Fig. 12

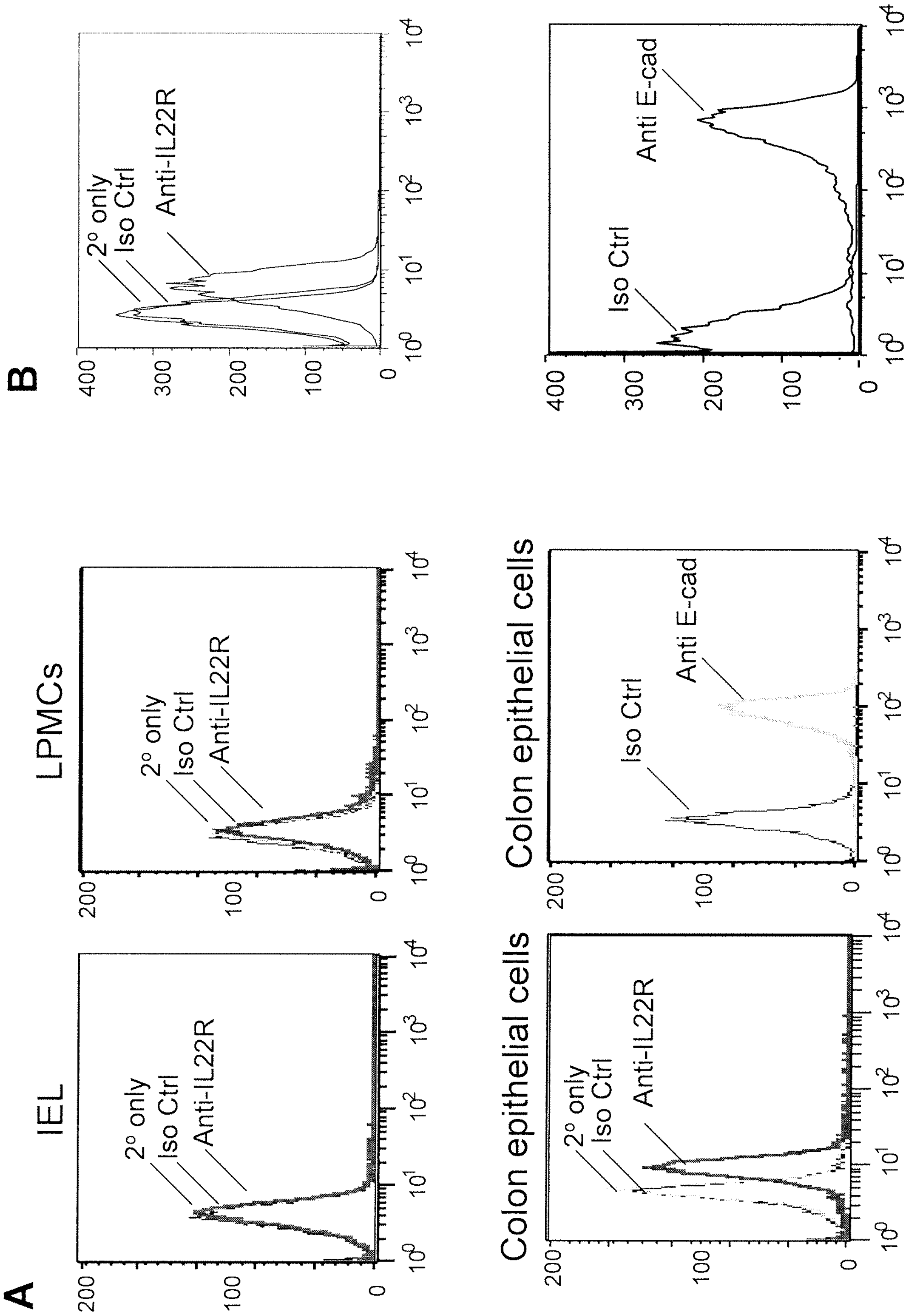
A
B
IEL
LPMCs
Colon epithelial cells
Colon epithelial cells
2° only
Iso Ctrl
Anti-IL22R
Anti E-cad
0
100
200
300
400
$10^0$
$10^1$
$10^2$
$10^3$
$10^4$

Fig. 13

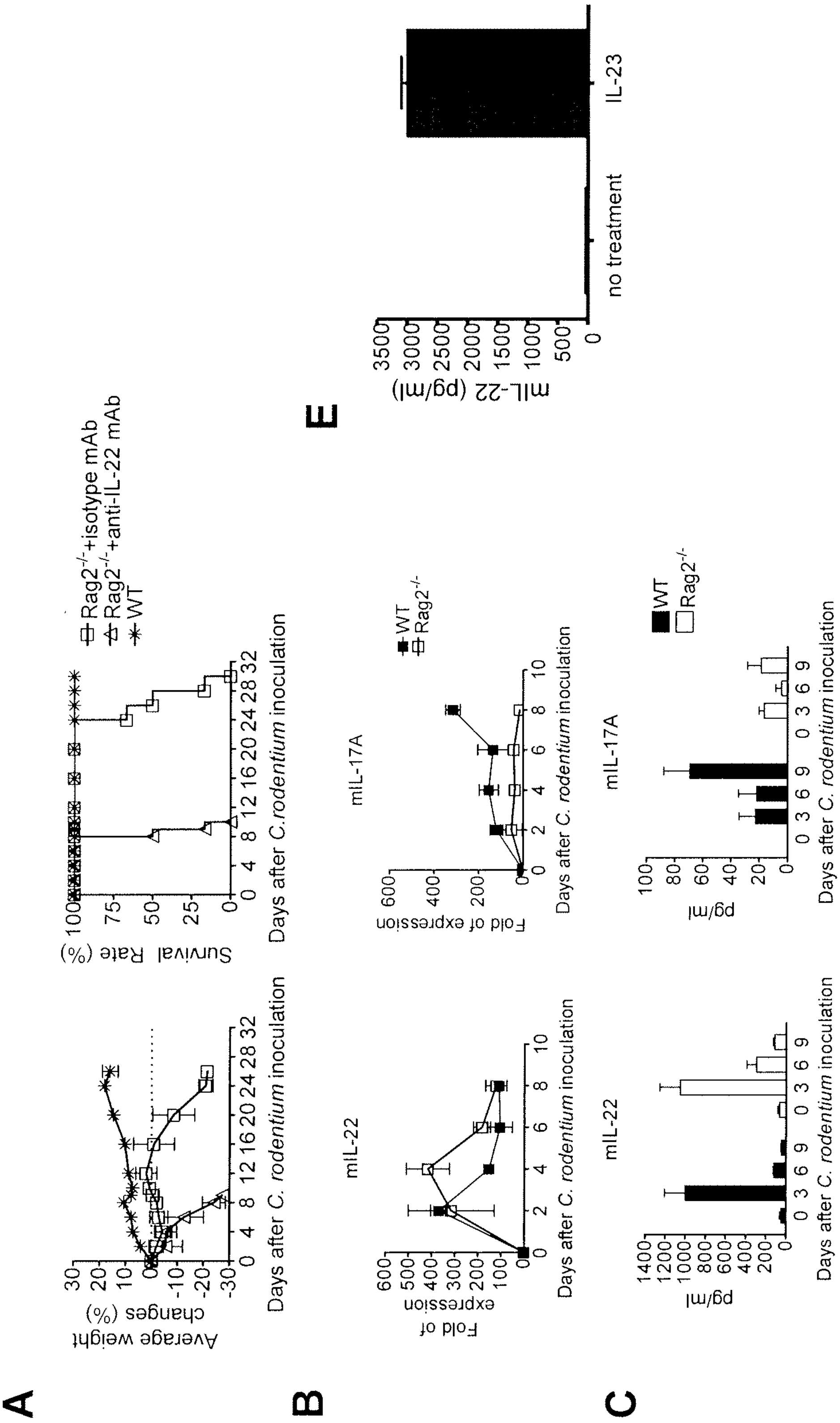
A
Average weight changes (%)
Days after C. rodentium inoculation
Survival Rate (%)
Days after C.rodentium inoculation
Rag2-/-+isotype mAb
Rag2-/-+anti-IL-22 mAb
WT
B
mIL-22
Fold of expression
Days after C. rodentium inoculation
mIL-17A
WT
Rag2-/-
C
mIL-22
pg/ml
Days after C. rodentium inoculation
mIL-17A
WT
Rag2-/-
E
mIL-22 (pg/ml)
no treatment
IL-23

Fig. 13D

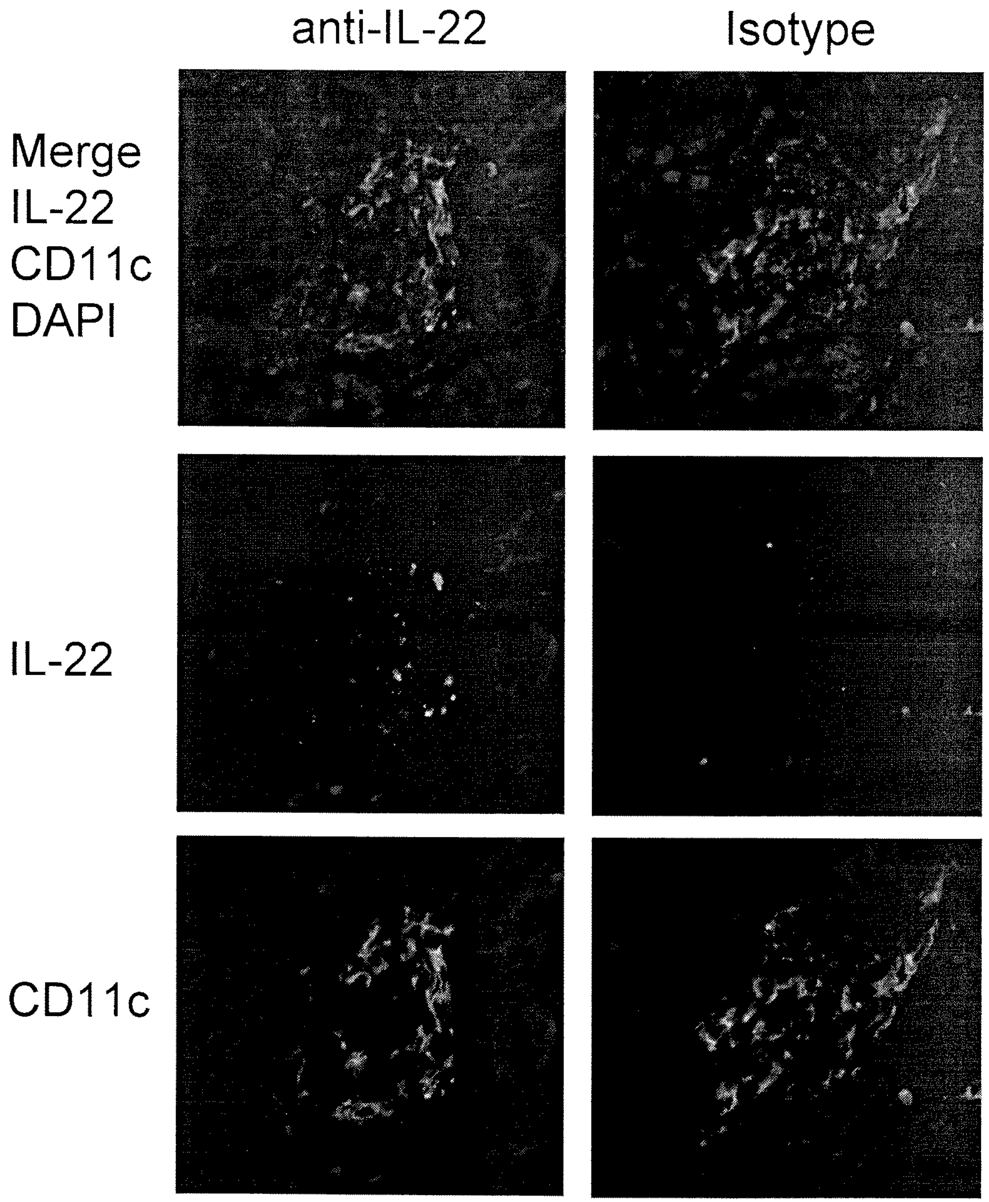

Fig. 14

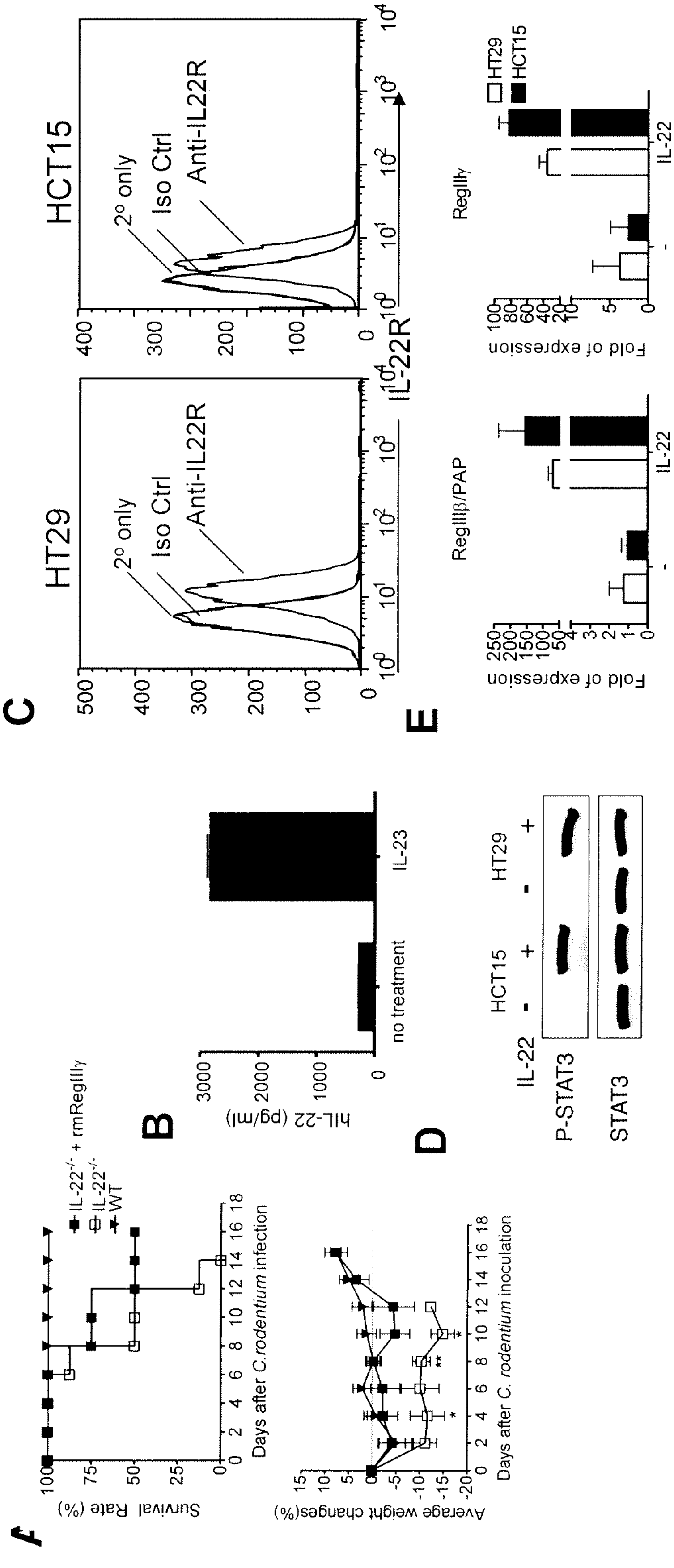
A
Survival Rate (%)
Days after C. rodentium infection
IL-22-/- + rmRegIIIγ
IL-22-/-
WT
Average weight changes(%)
Days after C. rodentium inoculation
B
hIL-22 (pg/ml)
no treatment
IL-23
C
HT29
HCT15
2° only
Iso Ctrl
Anti-IL22R
IL-22R
D
IL-22
HCT15
HT29
P-STAT3
STAT3
E
Fold of expression
RegIIIβ/PAP
RegIIIγ
HT29
HCT15
IL-22

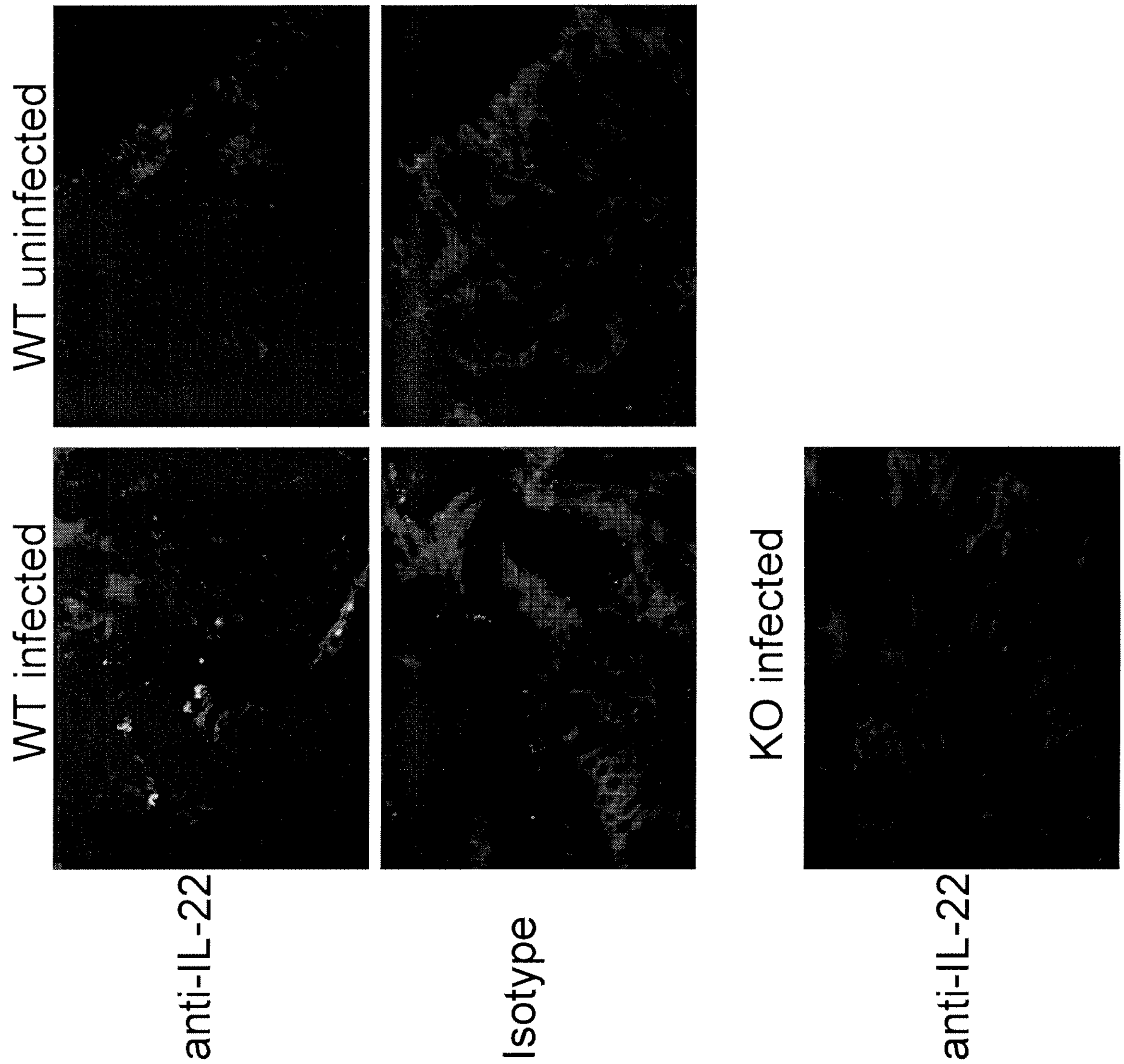
WT uninfected
WT infected
KO infected
anti-IL-22
Isotype
anti-IL-22

Fig. 15A

Fig. 15B

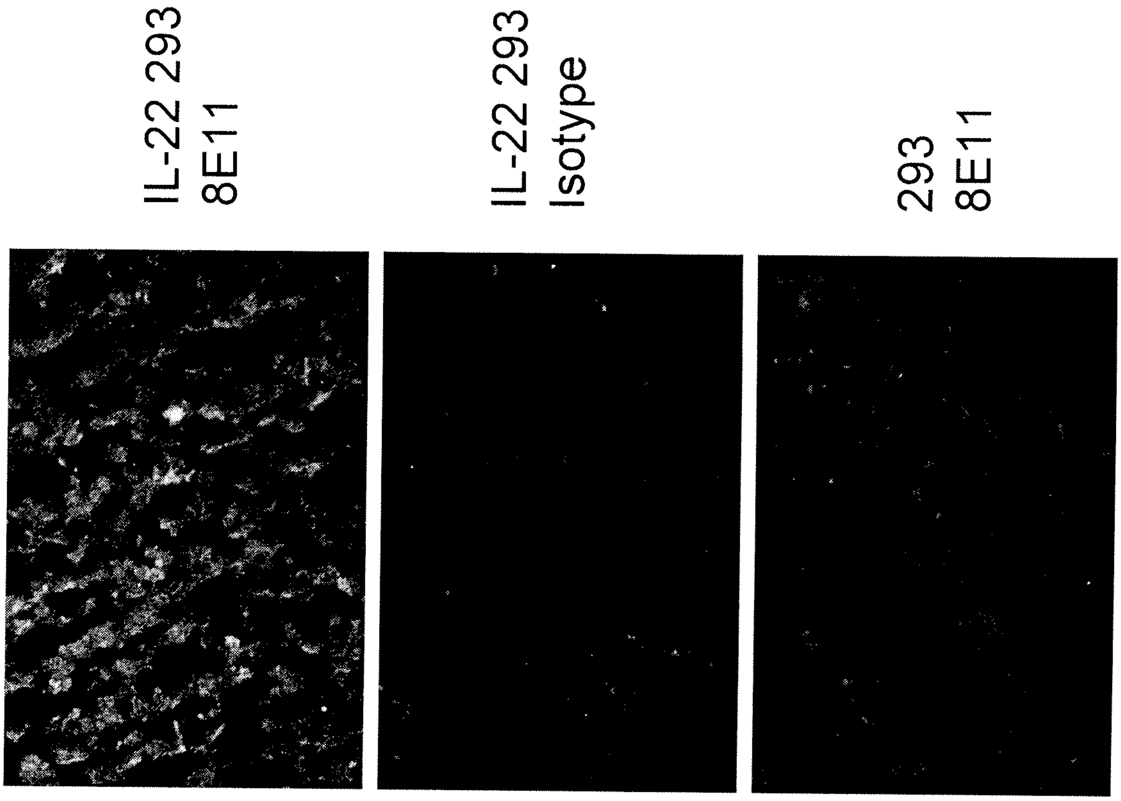

Fig. 16

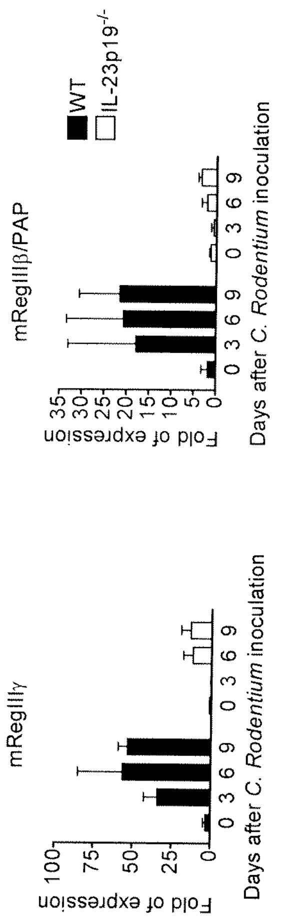
mRegIIIγ
Fold of expression
100
75
50
25
0
0 3 6 9 0 3 6 9
Days after C. Rodentium inoculation
mRegIIIβ/PAP
Fold of expression
35
30
25
20
15
10
5
0
0 3 6 9 0 3 6 9
Days after C. Rodentium inoculation
WT
IL-23p19-/-

Fig. 17

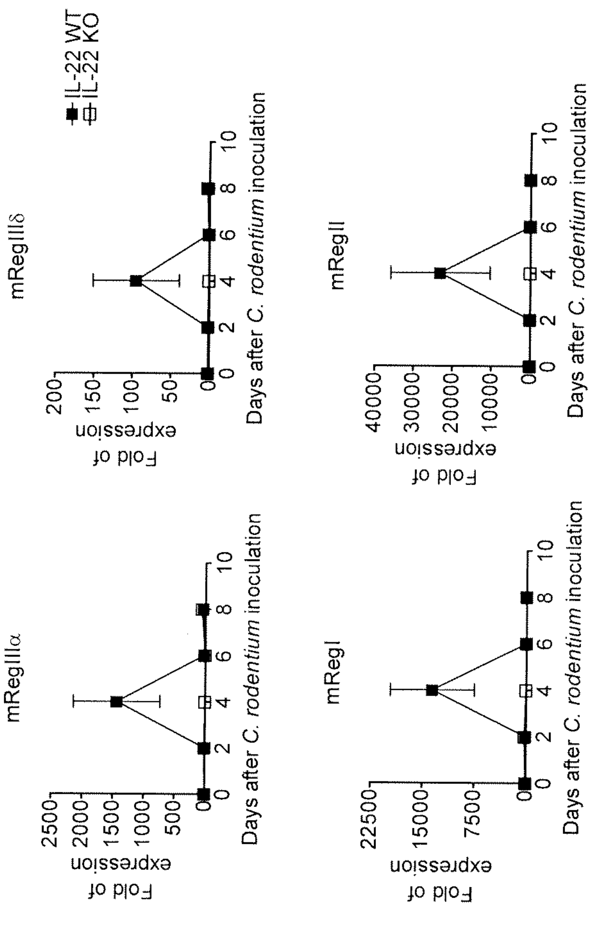
mRegIIIα
mRegIIIδ
mRegI
mRegII
IL-22 WT
IL-22 KO
Fold of expression
Days after *C. rodentium* inoculation
0
2
4
6
8
10
2500
2000
1500
1000
500
200
150
100
50
22500
15000
7500
40000
30000
20000
10000

Fig. 18

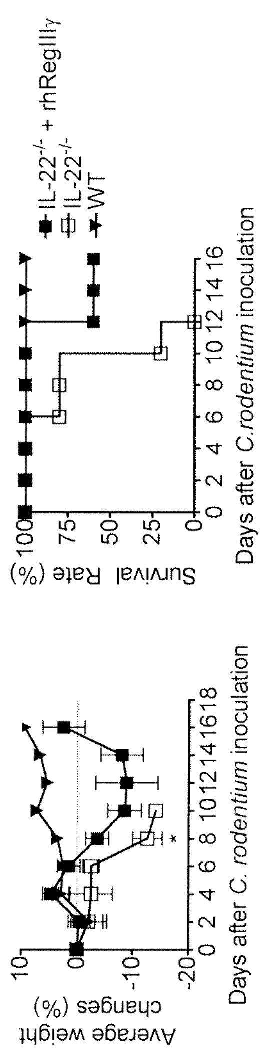
Average weight changes (%)
10
0
-10
-20
0 2 4 6 8 10121411618
Days after C. rodentium inoculation
Survival Rate (%)
100
75
50
25
0
0 2 4 6 8 10 12 14 16
Days after C.rodentium inoculation
IL-22-/- + rhRegIIIγ
IL-22-/-
WT

# Fig. 19A

| Probeset | P-value | Log2 FC | ID | Symbol |
|---|---|---|---|---|
| A_51_P256827 | 0 | 5.674 | NM_013650 | S100a8 |
| A_52_P700056 | 0.001 | 3.722 | BC089618 | |
| A_51_P402943 | 0.001 | 3.637 | NM_009114 | S100a9 |
| A_51_P235945 | 0.007 | 2.668 | NM_017370 | Hp |
| A_51_P169671 | 0.042 | 2.523 | NM_011036 | Pap |
| A_52_P472324 | 0.012 | 2.351 | NM_011414 | Slpi |
| A_52_P329207 | 0.006 | 2.262 | NM_007969 | Expi |
| A_52_P295432 | 0.015 | 2.111 | NM_009141 | Cxcl5 |
| A_52_P321831 | 0.007 | 2.062 | XM_894811 | Krtap3-1 |
| A_51_P279437 | 0.013 | 2.032 | NM_029662 | Mfsd2 |
| A_51_P246166 | 0.01 | 1.983 | NM_007969 | Expi |
| A_52_P230938 | 0.008 | 1.966 | NM_010741 | Ly6c |
| A_51_P384894 | 0.019 | 1.837 | NM_001001332 | Stfa1 |
| A_51_P156955 | 0.045 | 1.837 | NM_013459 | Cfd |
| A_51_P303160 | 0.03 | 1.821 | NM_007482 | Arg1 |
| A_51_P461703 | 0.034 | 1.804 | NM_031188 | Mup1 |
| A_51_P374476 | 0.019 | 1.742 | NM_008220 | Hbb-b1 |
| A_51_P481958 | 0.012 | 1.735 | NM_016674 | Cldn1 |
| A_51_P464703 | 0.019 | 1.727 | NM_021443 | Ccl8 |
| A_51_P132185 | 0.031 | 1.724 | NM_001001332 | Stfa1 |
| A_51_P496795 | 0.019 | 1.669 | NM_145555 | A330049M08Rik |
| A_51_P296109 | 0.01 | 1.63 | NM_028618 | Dmkn |
| A_51_P228768 | 0.043 | 1.591 | NM_011409 | Slfn3 |
| A_51_P466535 | 0.014 | 1.571 | AK172918 | Ttc9 |
| A_51_P387239 | 0.04 | 1.569 | NM_021792 | Iigp1 |
| A_51_P337308 | 0.03 | 1.547 | NM_011315 | Saa3 |
| A_51_P346938 | 0.031 | 1.541 | NM_029796 | Lrg1 |
| A_51_P504546 | 0.047 | 1.536 | NM_027406 | Aldh1l1 |
| A_51_P386870 | 0.026 | 1.53 | NM_011472 | Sprr2f |
| A_51_P216456 | 0.024 | 1.522 | NM_009311 | Tac1 |
| A_51_P212491 | 0.025 | 1.517 | NM_133232 | Pfkfb3 |
| A_52_P48681 | 0.018 | 1.514 | NM_016674 | Cldn1 |
| A_52_P424959 | 0.038 | 1.498 | NM_153457 | Rtn1 |
| A_52_P232813 | 0.047 | 1.484 | NM_203320 | Gm1960 |
| A_51_P181891 | 0.011 | 1.458 | NM_028113 | 2600011E07Rik |
| A_51_P249989 | 0.014 | 1.455 | NM_145133 | T2bp |
| A_52_P487686 | 0.042 | 1.405 | NM_001001332 | Stfa1 |
| A_52_P168962 | 0.023 | 1.387 | NM_007646 | Cd38 |
| A_51_P485312 | 0.026 | 1.372 | NM_013653 | Ccl5 |
| A_52_P99353 | 0.019 | 1.37 | AK009488 | |
| A_52_P252931 | 0.037 | 1.358 | NM_013505 | Dsc2 |
| A_52_P278538 | 0.039 | 1.349 | NM_008218 | Hba-a1 |
| A_52_P85132 | 0.034 | 1.331 | NM_172440 | Stxbp5l |
| A_52_P407198 | 0.023 | 1.322 | NM_183153 | A430060F13Rik |
| A_52_P638459 | 0.03 | 1.317 | BC033508 | Ccl5 |
| A_51_P335559 | 0.019 | 1.311 | AK034993 | |
| A_51_P240801 | 0.018 | 1.308 | BC046640 | 2610307O08Rik |
| A_52_P627816 | 0.032 | 1.292 | NM_019984 | Tgm1 |
| A_52_P656860 | 0.01 | 1.273 | NM_026232 | Slc25a30 |
| A_51_P180314 | 0.045 | 1.258 | AK011413 | Mup1 |
| A_51_P328870 | 0.011 | 1.249 | D86599 | Oxtr |
| A_51_P482908 | 0.041 | 1.237 | NM_025549 | Arrdc4 |
| A_51_P257951 | 0.041 | 1.235 | NM_020509 | Retnla |
| A_51_P441426 | 0.029 | 1.234 | NM_019932 | Cxcl4 |

Fig. 19B

| Probeset | P-value | Log2 FC | ID | Symbol |
|---|---|---|---|---|
| A_51_P128463 | 0.049 | 1.232 | XM_485455 | Grrp1 |
| A_51_P239750 | 0.008 | 1.196 | NM_008380 | Inhba |
| A_51_P413866 | 0.044 | 1.188 | NM_008198 | Cfb |
| A_51_P462192 | 0.037 | 1.18 | NM_138648 | Olr1 |
| A_51_P291417 | 0.026 | 1.172 | NM_009378 | Thbd |
| A_51_P459176 | 0.013 | 1.162 | XM_899837 | 1700095J03Rik |
| A_51_P122321 | 0.012 | 1.161 | NM_133775 | 9230117N10Rik |
| A_51_P110301 | 0.023 | 1.158 | NM_009778 | C3 |
| A_51_P294807 | 0.039 | 1.154 | NM_013515 | Stom |
| A_51_P504991 | 0.027 | 1.151 | NM_181542 | Slfn10 |
| A_51_P440743 | 0.041 | 1.148 | NM_009886 | Celsr1 |
| A_51_P141012 | 0.019 | 1.137 | AK173296 | Slitrk1 |
| A_51_P445726 | 0.043 | 1.128 | NM_011095 | Lilrb3 |
| A_51_P397968 | 0.03 | 1.087 | AK087113 | |
| A_51_P464408 | 0.034 | 1.078 | NM_177314 | 5330439B14Rik |
| A_52_P597634 | 0.041 | 1.077 | NM_021457 | Fzd1 |
| A_51_P149714 | 0.038 | 1.066 | NM_026835 | Ms4a6d |
| A_52_P409833 | 0.027 | 1.04 | NM_008872 | Plat |
| A_51_P172853 | 0.03 | 1.04 | NM_009841 | Cd14 |
| A_52_P26161 | 0.026 | 1.039 | NM_008987 | Ptx3 |
| A_52_P164821 | 0.034 | 1.038 | XM_905096 | LOC631323 |
| A_52_P987411 | 0.036 | 1.038 | AK032717 | |
| A_51_P370552 | 0.037 | 1.037 | NM_030061 | 9230117E20Rik |
| A_52_P616392 | 0.017 | 1.032 | NM_183426 | Stno |
| A_51_P181899 | 0.027 | 1.021 | NM_028113 | 2600011E07Rik |
| A_52_P104075 | 0.037 | 1.021 | AK052629 | Frem1 |
| A_52_P621991 | 0.048 | 1.019 | NM_009684 | Apaf1 |
| A_52_P616949 | 0.036 | 1.014 | NM_027893 | Pvrl4 |
| A_51_P504988 | 0.022 | 1.01 | NM_172796 | Slfn9 |
| A_52_P87713 | 0.031 | 1.008 | NM_011593 | Timp1 |
| A_52_P1012710 | 0.034 | 1.005 | AK075639 | |
| A_52_P491718 | 0.049 | -1.002 | AK047841 | Mbtps2 |
| A_51_P191331 | 0.015 | -1.003 | NM_011379 | Sipa1 |
| A_52_P680941 | 0.028 | -1.003 | NM_010284 | Ghr |
| A_52_P1027837 | 0.038 | -1.003 | AK034309 | 9330175M20Rik |
| A_52_P787453 | 0.021 | -1.019 | AK045835 | |
| A_52_P484194 | 0.025 | -1.023 | NM_001025602 | Il1rl1 |
| A_52_P524426 | 0.03 | -1.023 | NM_001003815 | Epb4.1l1 |
| A_52_P828218 | 0.027 | -1.027 | NM_001030294 | Olfm4 |
| A_52_P674087 | 0.011 | -1.028 | AK036172 | Prtg |
| A_52_P826055 | 0.032 | -1.028 | AK134458 | 2310020J12Rik |
| A_52_P563505 | 0.022 | -1.033 | AK007074 | 1700095A21Rik |
| A_52_P44454 | 0.015 | -1.034 | P052591-1 | |
| A_51_P398037 | 0.018 | -1.039 | AK048407 | Scn3a |
| A_52_P1132297 | 0.017 | -1.058 | AK052006 | |
| A_52_P35598 | 0.034 | -1.064 | AK051891 | D230015P20Rik |
| A_51_P151983 | 0.009 | -1.066 | NM_019744 | Ncoa4 |
| A_51_P130719 | 0.014 | -1.066 | NM_023047 | Dpysl5 |
| A_52_P218833 | 0.018 | -1.066 | ENSMUST00000( | |
| A_52_P1084199 | 0.022 | -1.078 | AK036139 | 9630039A02Rik |
| A_51_P138496 | 0.021 | -1.08 | AK046037 | |
| A_52_P1124261 | 0.046 | -1.086 | AK081465 | |
| A_52_P158438 | 0.023 | -1.094 | AK006669 | 1700041C23Rik |
| A_52_P187046 | 0.022 | -1.098 | NM_022724 | Suv39h2 |

# Fig. 19C

| Probeset | P-value | Log2 FC | ID | Symbol |
|---|---|---|---|---|
| A_52_P344098 | 0.027 | -1.098 | AK040876 | Pde7b |
| A_51_P314669 | 0.034 | -1.1 | NM_011128 | Pnliprp2 |
| A_52_P475356 | 0.02 | -1.107 | NM_011445 | Sox6 |
| A_52_P177454 | 0.043 | -1.114 | AK045474 | Rab34 |
| A_52_P972196 | 0.015 | -1.12 | AK084612 | |
| A_52_P1140731 | 0.024 | -1.122 | AK089673 | |
| A_51_P249302 | 0.017 | -1.125 | NM_011994 | Abcd2 |
| A_52_P866925 | 0.031 | -1.133 | AK006067 | 1700017I07Rik |
| A_52_P956057 | 0.026 | -1.17 | AK081935 | |
| A_52_P939063 | 0.012 | -1.171 | AK032576 | |
| A_51_P243750 | 0.037 | -1.175 | NM_012025 | Racgap1 |
| A_52_P205710 | 0.047 | -1.189 | NM_021611 | Mylc2pl |
| A_52_P1188576 | 0.017 | -1.197 | AK082439 | |
| A_51_P282523 | 0.032 | -1.201 | NM_054044 | Gpr124 |
| A_52_P642801 | 0.009 | -1.202 | XM_618738 | Lats1 |
| A_51_P312615 | 0.038 | -1.207 | NM_010377 | Hist1h1t |
| A_52_P214828 | 0.022 | -1.209 | NM_011253 | Rbmy1a1 |
| A_52_P158660 | 0.024 | -1.231 | XM_001002308 | 4833417A11Rik |
| A_51_P104255 | 0.012 | -1.237 | NM_001039226 | LOC625026 |
| A_52_P811599 | 0.035 | -1.244 | AK084144 | |
| A_52_P668855 | 0.016 | -1.246 | AK042778 | 9330154J02Rik |
| A_51_P399974 | 0.035 | -1.246 | NM_146997 | Olfr178 |
| A_52_P355709 | 0.007 | -1.247 | XM_973329 | LOC664837 |
| A_52_P207356 | 0.048 | -1.249 | AK052515 | Aspm |
| A_52_P1188835 | 0.034 | -1.256 | NM_001002780 | F830005K03Rik |
| A_51_P407915 | 0.013 | -1.263 | AK040806 | A530026G17 |
| A_51_P352743 | 0.045 | -1.285 | NM_001013616 | Trim6 |
| A_51_P191139 | 0.012 | -1.293 | AK050699 | D030005H02Rik |
| A_52_P1139473 | 0.007 | -1.294 | AK018751 | 0610037L18Rik |
| A_52_P69506 | 0.042 | -1.294 | NM_177566 | Arhgef15 |
| A_52_P134003 | 0.042 | -1.327 | NM_001033289 | Slc9a2 |
| A_52_P519943 | 0.004 | -1.337 | BC044804 | Fads6 |
| A_51_P366413 | 0.009 | -1.394 | AK033346 | 8030498J20Rik |
| A_52_P988314 | 0.029 | -1.401 | AK083509 | |
| A_52_P778486 | 0.029 | -1.404 | AK016521 | 4932412D23Rik |
| A_52_P162486 | 0.009 | -1.409 | NM_007542 | Bgn |
| A_51_P242952 | 0.032 | -1.411 | AK079868 | |
| A_52_P267243 | 0.039 | -1.413 | NM_010200 | Fgf13 |
| A_52_P328714 | 0.018 | -1.422 | AK133758 | Fbxw2 |
| A_51_P238040 | 0.018 | -1.424 | AK030263 | |
| A_51_P327166 | 0.006 | -1.426 | AK015801 | |
| A_51_P201035 | 0.027 | -1.443 | NM_145924 | Cenpi |
| A_52_P169595 | 0.034 | -1.455 | NM_177669 | A630098G03Rik |
| A_52_P507877 | 0.04 | -1.463 | NM_007729 | Col11a1 |
| A_52_P224818 | 0.012 | -1.468 | NM_172483 | Zfp180 |
| A_51_P270050 | 0.041 | -1.485 | AK078370 | 6530421E24Rik |
| A_51_P309099 | 0.02 | -1.489 | AK050657 | |
| A_51_P103261 | 0.034 | -1.533 | NM_009198 | Slc17a1 |
| A_52_P535430 | 0.021 | -1.561 | NM_207130 | Abca16 |
| A_52_P1083387 | 0.015 | -1.585 | AK005856 | 1700011B04Rik |
| A_51_P152873 | 0.014 | -1.71 | NM_207670 | Gripap1 |
| A_52_P569873 | 0.012 | -1.778 | XM_127913 | 4930455B06Rik |
| A_51_P396119 | 0.008 | -2.489 | AK047973 | Ttll5 |

Fig.20

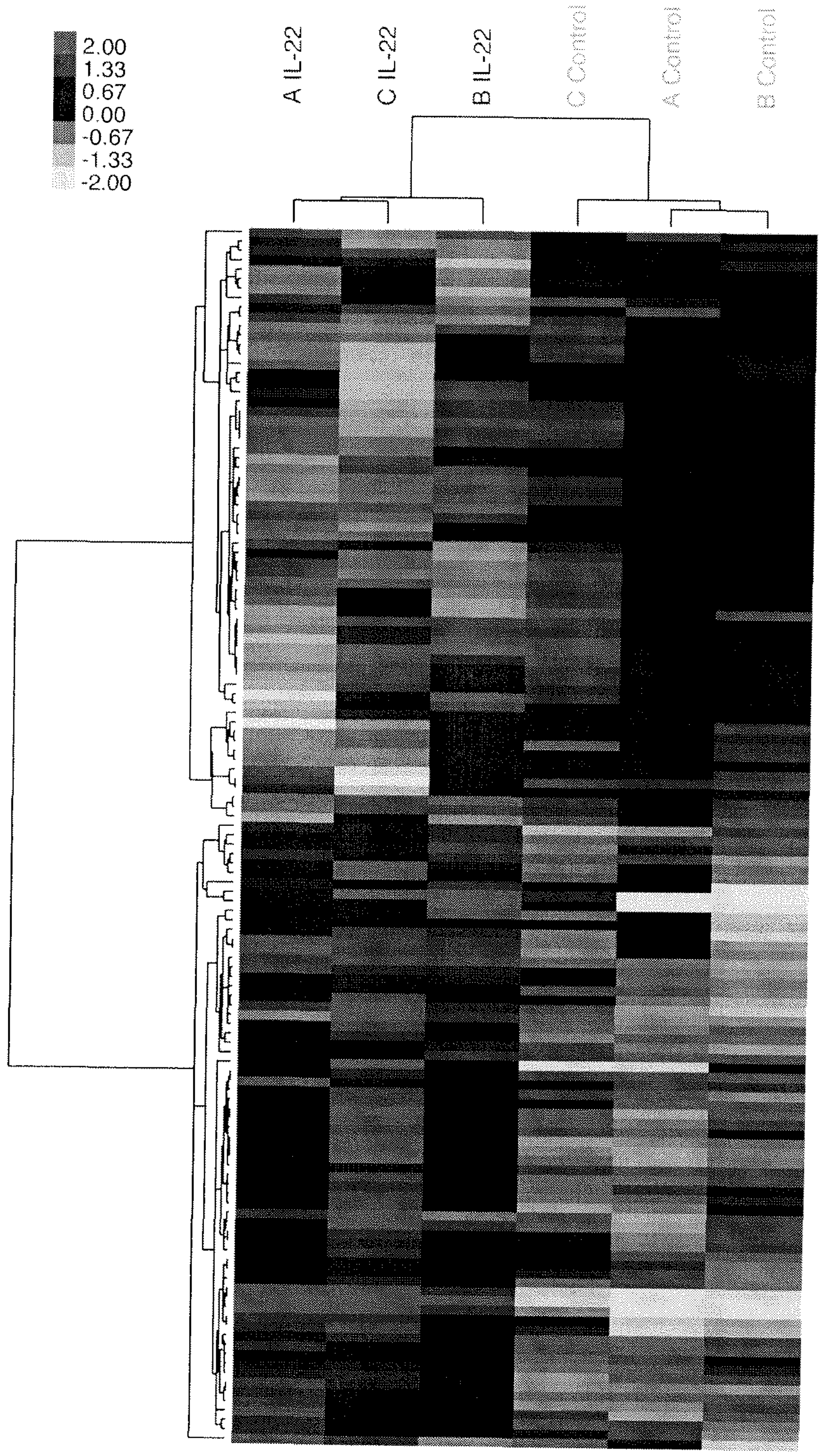
2.00
1.33
0.67
0.00
-0.67
-1.33
-2.00
A IL-22
C IL-22
B IL-22
C Control
A Control
B Control

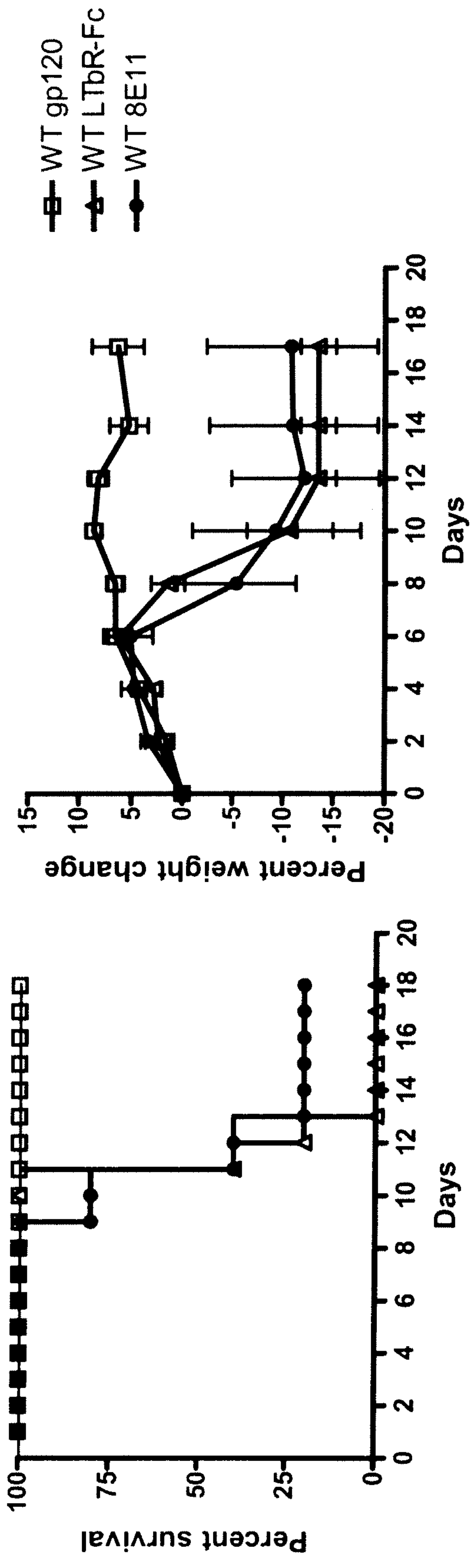
WT gp120
WT LTbR-Fc
WT 8E11
Percent weight change
Days
Percent survival
Days

Fig. 21

Fig. 22

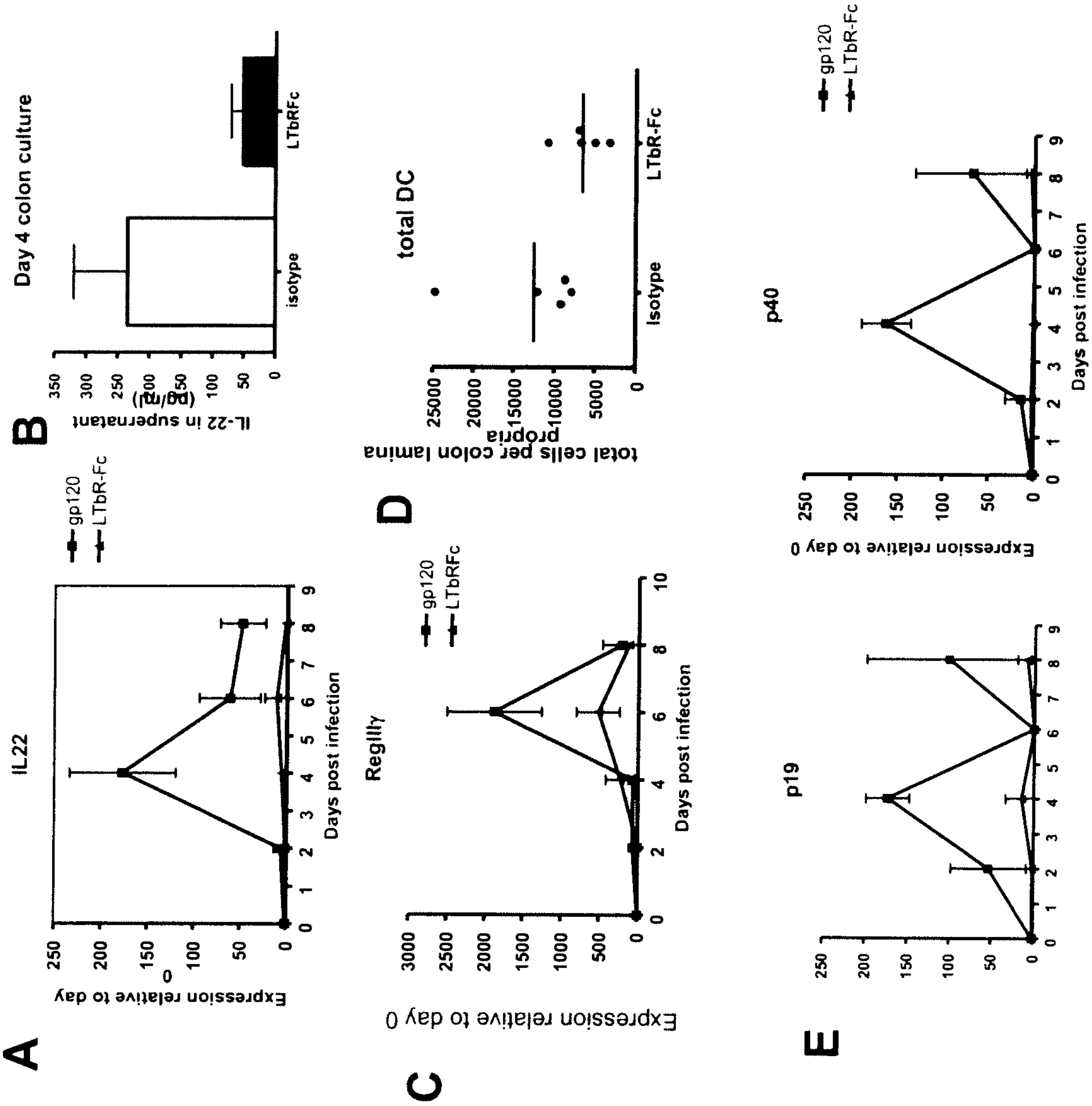
A
IL22
Expression relative to day 0
Days post infection
gp120
LTbR-Fc
B
Day 4 colon culture
IL-22 in supernatant (pg/ml)
isotype
LTbRFc
C
RegIIIγ
Expression relative to day 0
Days post infection
gp120
LTbRFc
D
total DC
total cells per colon lamina propria
Isotype
LTbR-Fc
E
p19
p40
Expression relative to day 0
Days post infection
gp120
LTbR-Fc

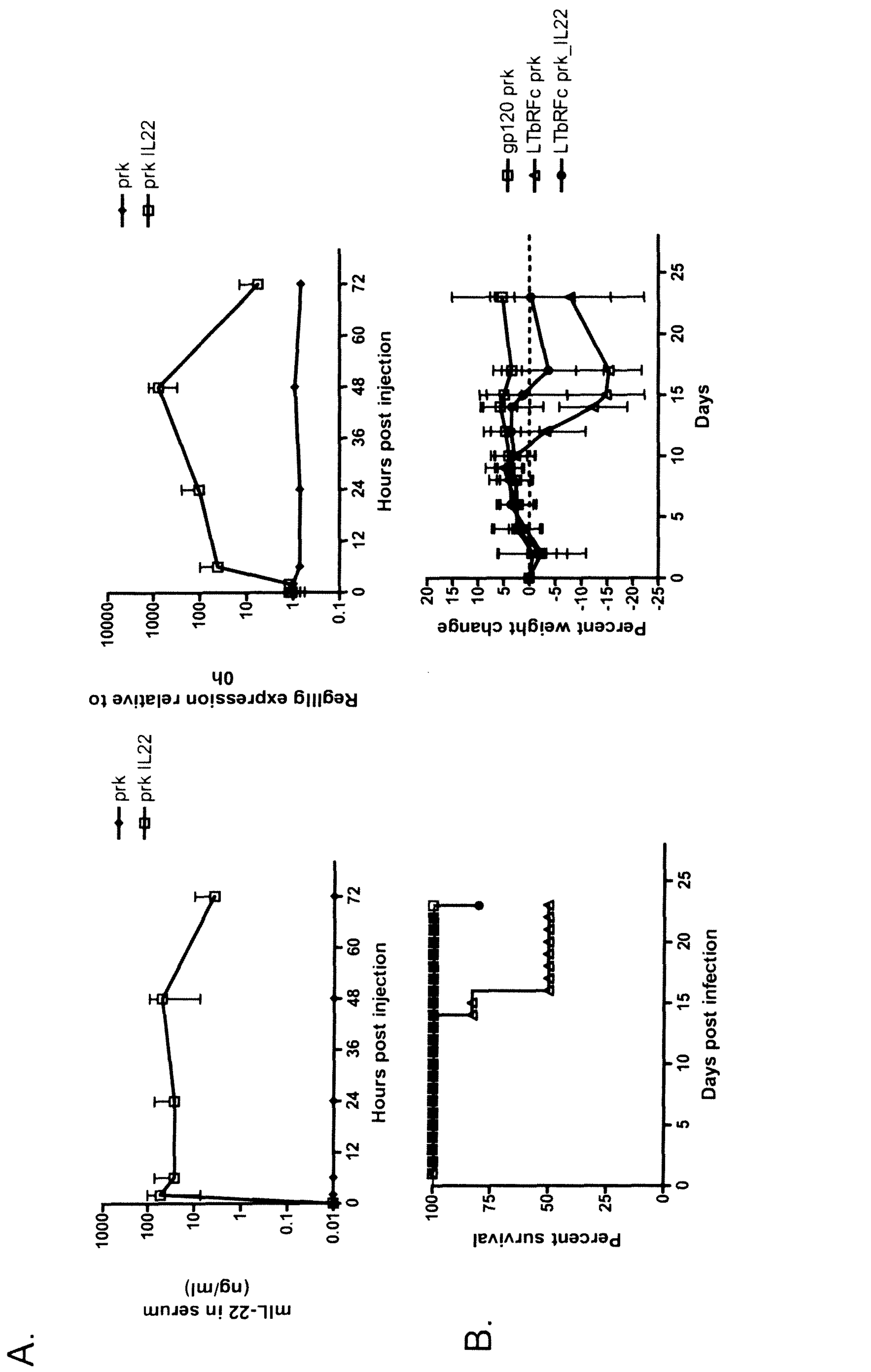
A.
mIL-22 in serum (ng/ml)
1000
100
10
1
0.1
0.01
0
12
24
36
48
60
72
Hours post injection
prk
prk IL22
RegIIIg expression relative to 0h
10000
1000
100
10
1
0.1
Hours post injection
prk
prk IL22
B.
Percent survival
100
75
50
25
0
5
10
15
20
25
Days post infection
Percent weight change
20
15
10
5
0
-5
-10
-15
-20
-25
Days
gp120 prk
LTbRFc prk
LTbRFc prk_IL22

Fig. 23

Fig. 24

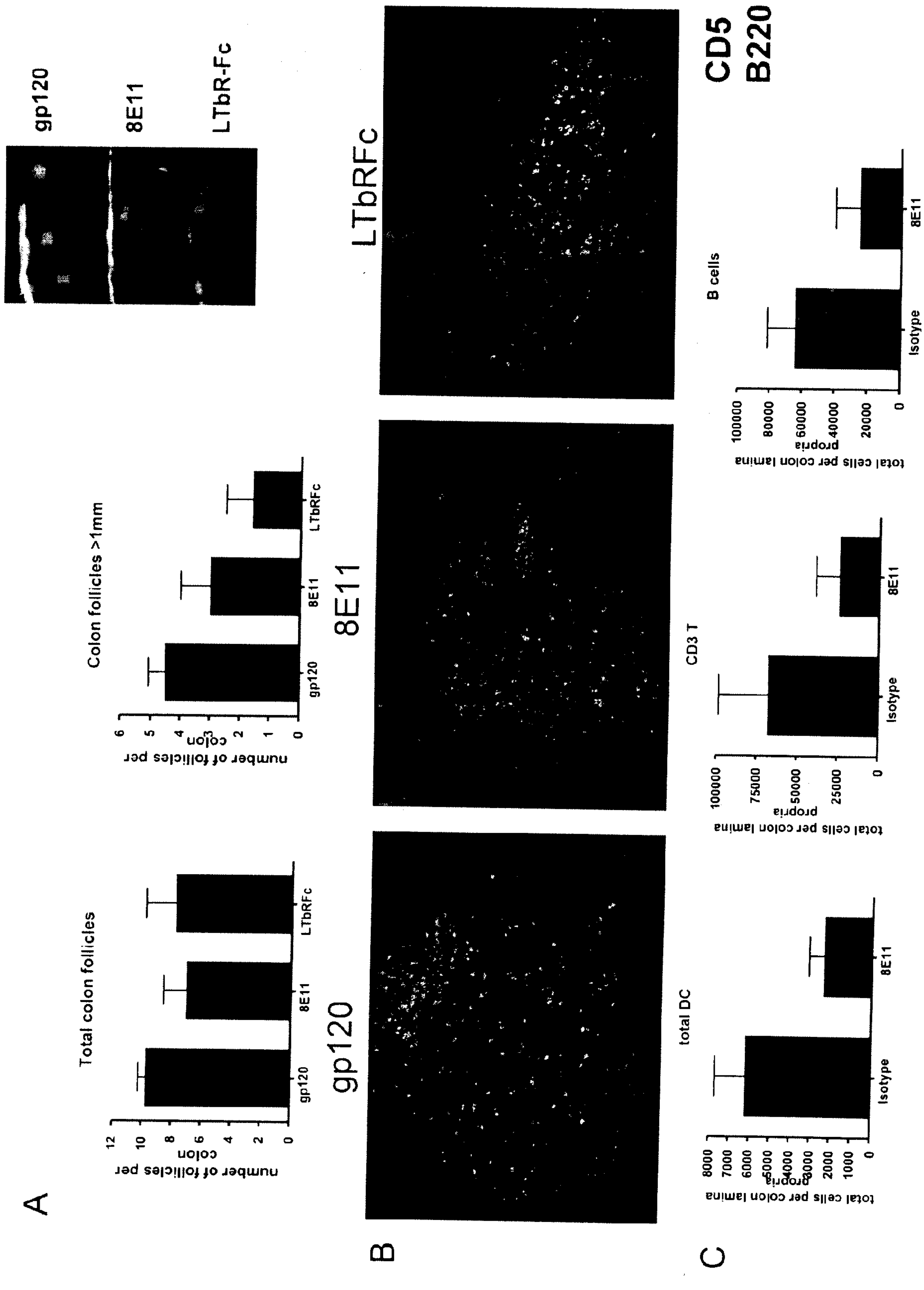
A
B
C
Total colon follicles
Colon follicles >1mm
number of follicles per colon
gp120
8E11
LTbRFc
LTbR-Fc
CD5
B220
total DC
CD3 T
B cells
total cells per colon lamina propria
Isotype

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050129614 A
- US 5824509 A
- US 5661004 A
- EP 404097 A
- WO 931161 A
- US 4816567 A
- WO 9824893 A
- WO 9634096 A
- WO 9633735 A
- WO 9110741 A
- US 5545807 A
- US 5545806 A
- US 5569825 A
- US 5625126 A
- US 5633425 A
- US 5661016 A
- US 4275149 A
- US 5364934 A
- US 5428130 A
- US 5869046 A
- WO 9316185 A
- US 5571894 A
- US 5587458 A
- US 5641870 A
- WO 9306213 A
- WO 9308829 A
- WO 9404690 A
- US 4676980 A
- WO 9100360 A
- WO 9200373 A
- EP 03089 A
- US 6248516 B1
- US 20030157108 A, Presta, L.
- US 20040093621 A
- WO 2003011878 A, Jean-Mairet
- US 6602684 B, Umana
- WO 199730087 A, Patel
- WO 199858964 A, Raju, S.
- WO 199922764 A, Raju, S.
- US 20050123546 A, Umana
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- US 20030157108 A1, Presta, L
- WO 2004056312 A1
- WO 9951642 A
- US 5648260 A
- US 5624821 A
- WO 9429351 A
- WO 0042072 A, Presta
- WO 2004056312 A
- US 20050014934 A1, Hinton
- US 6194551 B1
- US 5731168 A
- WO 92200373 A
- US 5648237 A, Carter
- US 5840523 A, Simmons
- US 5639635 A
- US 6083715 A, Georgiou
- US 6027888 A, Georgiou
- US 5264365 A, Georgiou
- US 5508192 A, Georgiou
- US 4965199 A
- US 4419446 A
- US 4601978 A
- WO 9411026 A
- US 4767704 A
- US 4657866 A
- US 4927762 A
- US 4560655 A
- US 5122469 A
- WO 9003430 A
- WO 8700195 A
- US RE30985 A
- US 5556762 A
- US 5750373 A
- US 4708871 A
- US 4833092 A
- US 5223409 A
- US 5403484 A
- US 5571689 A
- US 5663143 A
- WO 8403506 A
- WO 8403564 A
- WO 0000823 A
- WO 0039585 A
- US 4485045 A
- US 4544545 A

- US 5013556 A
- US 3773919 A
- US 4376110 A
- EP 08848478 A
- US 2008082890 W
- WO 60986170 A
- WO 61013620 A
- WO 61015620 A

**Non-patent literature cited in the description**


- *The World Health Report,* 2004
- *MMWR Morb Mortal Wkly Rep,* 29 September 2006, vol. 55, 1045
- **R. L. SIEGLER.** *Pediatr Clin North Am,* December 1995, vol. 42, 1505
- **T. S. WHITTAM et al.** *Infect Immun,* May 1993, vol. 61, 1619
- **L. ECKMANN.** *Ann NY Acad Sci,* 01 August 2006, vol. 1072, 28
- **D. B. SCHAUER ; S. FALKOW.** *Infect Immun,* June 1993, vol. 61, 2486
- **T. T. MACDONALD ; G. MONTELEONE.** *Science,* 25 March 2005, vol. 307, 1920
- **A. TAKAHASHI et al.** *FEBS Lett,* 23 November 2001, vol. 508, 484
- **L. BRY ; M. B. BRENNER.** *J Immunol,* 01 January 2004, vol. 172, 433
- **Y. ZHENG et al.** *Nature,* 08 February 2007, vol. 445, 648
- **K. I. HAPPEL et al.** *J. Exp. Med.,* 19 September 2005, vol. 202, 761
- **SAMBROOK et al.** Molecular Cloning: A Laboratory Manual. Cold Spring Harbor Laboratory Press, 2001
- Current Protocols in Molecular Biology. 2003
- Methods in Enzymology. Academic Press, Inc,
- PCR 2: A Practical Approach. 1995
- Antibodies, A Laboratory Manual. 1988
- Animal Cell Culture. 1987
- Oligonucleotide Synthesis. 1984
- Methods in Molecular Biology. Humana Press
- Cell Biology: A Laboratory Notebook. Academic Press, 1998
- **J. P. MATHER ; P. E. ROBERTS.** Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C. A. JANEWAY ; P. TRAVERS.** Immunobiology. 1997
- **P. FINCH.** Antibodies. 1997
- Antibodies: A Practical Approach. IRL Press, 1988
- Monoclonal Antibodies: A Practical Approach. Oxford University Press, 2000
- **E. HARLOW ; D. LANE.** Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **PENNICA et al.** *Nature,* 1984, vol. 312 (20/27), 724-729
- **AGGARWAL et al.** *J. Biol. Chem.,* 1985, vol. 260, 2345-2354
- **KABAT et al.** Sequences of Proteins of Immunological Interest. National Institute of Heath, 1991
- **ABBAS et al.** Cellular and Mol. Immunology. 2000
- The Pharmacology of Monoclonal Antibodies. Springer-Verlag, 1994, vol. 113, 269-315
- **HUDSON et al.** *Nat. Med.,* 2003, vol. 9, 129-134
- **HOLLINGER et al.** *Proc. Natl. Acad. Sci. USA,* 1993, vol. 90, 6444-6448
- **HUDSON et al.** *Nat. Med,* 2003, vol. 9, 129-134
- **KOHLER et al.** *Nature,* 1975, vol. 256, 495
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol,* 1992, vol. 222, 581-597
- **SIDHU et al.** *J Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *Bio. Technology,* 1992, vol. 10, 779-783

- **LONBERG et al.** *Nature,* 1994, vol. 368, 856-859
- **MORRISON.** *Nature,* 1994, vol. 368, 812-813
- **FISHWILD et al.** *Nature Biolechnol.,* 1996, vol. 14, 845-851
- **NEUBERGER.** *Nature Biotechnol.,* 1996, vol. 14, 826
- **LONBERG ; HUSZAR.** *Intern. Rev. Immunol.,* 1995, vol. 13, 65-93
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. transactions,* 1995, vol. 23, 1035-1038
- **HURLE ; GROSS.** *Curr. Op. Biotech.,* 1994, vol. 5, 428-433
- **MARKS et al.** *Bio/Technology,* 1992, vol. 10, 779-783
- **BARBAS et al.** *Proc Nat. Acad. Sci. USA,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **CHEN et al.** *J. Mol. Biol.,* 1999, vol. 293, 865-881
- **PRESTA et al.** *Cancer Res.,* 1997, vol. 57, 4593-4599
- **CHEN, Y. et al.** *J. Mol. Biol.,* 1999, vol. 293, 865-881
- Cell cycle regulation, oncogens, and antineoplastic drugs. **MURAKAMI et al.** The Molecular Basis of Cancer. WB Saunders: Philadelphia, 1995, 13
- **CARTER et al.** Nucl. Acids Res. 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **T.E. CREIGHTON.** Proteins: Structure and Molecular Properties. W.H. Freeman & Co., San Francisco, 1983, 79-86
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **DEUTSCHER.** *Methods in Enzymology,* 1990, vol. 182
- **SCOPES.** Protein Purification: Principles and Practice. Springer-Verlag, 1982
- **THOMAS.** *Proc. Natl. Acad. Sci. USA,* 1980, vol. 77, 5201-5205
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- Scatchard analysis of Munson and Pollard. *Anal. Biochem.,* 1980, vol. 107, 220
- **HOOGENBOOM et al.** Methods in Molecular Biology. Human Press, 2001, vol. 178, 1-37
- **LEE et al.** *J. Mol. Biol.,* 2004, vol. 340, 1073-1093
- **KABAT et al.** Sequences of Proteins of Immunological Interest. NIH Publication, 1991, vol. 1-3
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad. Sci. USA,* 1992, vol. 89, 4285
- **PRESTA et al.** *J. Immunol.,* 1993, vol. 151, 2623
- **BOERNER et al.** *J. Immunol.,* 1991, vol. 147, 86
- **JAKOBOVITS et al.** *Proc. Natl. Acad. Sci USA,* 1993, vol. 90, 2551

- **JAKOBOVITS et al.** *Nature,* 1993, vol. 362, 255
- **BRUGGERMANN et al.** *Year in Immunol.,* 1993, vol. 7, 33
- **MILSTEIN ; CUELLO.** *Nature,* 1983, vol. 305, 537
- **TRAUNECKER et al.** *EMBO J.,* 1991, vol. 10, 3655
- **SURESH et al.** *Methods in Enzymology,* 1986, vol. 121, 210
- **SHALABY et al.** *J. Exp. Med.,* 1992, vol. 175, 217-225
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CUNNINGHAM ; WELLS.** *Science,* 1989, vol. 244, 1081-1085
- **OKAZAKI et al.** *J. Mol. Biol.,* 2004, vol. 336, 1239-1249
- **YAMANE-OHNUKI.** *Biotech. Bioeng,* 2004, vol. 87, 614
- **RIPKA et al.** *Arch. Biochem. Biophys.,* 1986, vol. 249, 533-545
- **YAMANE-OHNUKI et al.** *Biotech. Bioeng.,* 2004, vol. 87, 614
- **DUNCAN ; WINTER.** *Nature,* 1988, vol. 322, 738-40
- **SHIELDS et al.** *J. Biol. Chem.,* 2001, vol. 9 (2), 6591-6604
- **GUYER et al.** *J. Immunol.,* 1976, vol. 117, 587
- **KIM et al.** *J. Immunol.,* 1994, vol. 24, 249
- **IDUSOGIE et al.** *J. Immunol.,* 2000, vol. 164, 4178-4184
- **KAM et al.** *Proc. Natl. Acad. Sci.,* 2005, vol. 102, 11600-11605
- **SICBENLIST et al.** *Cell,* 1980, vol. 20, 269
- **PROBA ; PLUCKTHUN.** *Gene,* 1995, vol. 159, 203
- **YANSURA et al.** *METHODS: A Companion to Methods in Enzymol.,* 1992, vol. 4, 15 1-158
- **BACHMANN.** Cellular and Molecular Biology. American Society for Microbiology, 1987, vol. 2, 1190-1219
- **BASS et al.** *Proteins,* 1990, vol. 8, 309-314
- **SIMMONS et al.** *J. Immunol. Methods,* 2002, vol. 263, 133-147
- **CHEN et al.** *J. Biol. Chem.,* 1999, vol. 274, 19601-19605
- **BOTHMANN ; PLUCKTHUN.** *J. Biol. Chem.,* 2000, vol. 275, 17100-17105
- **RAMM ; PLUCKTHUN.** *J. Biol. Chem.,* 2000, vol. 275, 17106-17113
- **ARIE et al.** *Mol. Microbiol.,* 2001, vol. 39, 199-210
- **HARA et al.** *Microbial Drug Resistance,* 1996, vol. 2, 63-72
- **LINDMARK et al.** *J. Immunol. Meth.,* 1983, vol. 62, 1-13
- **REYES.** *Nature,* 1982, vol. 297, 598-601
- **YANIV.** *Nature,* 1982, vol. 297, 17-18
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *Annals N.Y. Acad. Sci.,* 1982, vol. 383, 44-68
- **HAM et al.** *Meth. Enz.,* 1979, vol. 58, 44
- **BARNES et al.** *Anal. Biochem.,* 1980, vol. 102, 255
- **LINDMARK et al.** *J. Immunol. Methods,* 1983, vol. 62, 1-13
- **GUSS et al.** *EMBO J.,* 1986, vol. 5, 15671575
- **GEYSEN et al.** *Proc. Natl. Acad. Sci. U.S.A.,* 1984, vol. 81, 3998-4002
- **GEYSEN et al.** *Proc. Natl. Acad. Sci. USA,* 1985, vol. 82, 178-182
- **GEYSEN et al.** *Synthetic Peptides as Antigen,* 1986, 130-149
- **GEYSEN et al.** *J. Immunol. Meth.,* 1987, vol. 102, 259-274
- **SCHOOFS et al.** *J. Immunol.,* 1988, vol. 140, 611-616
- **CWIRLA, S. E. et al.** *Proc. Natl. Acad. Sci. USA,* 1990, vol. 87, 6378
- **LOWMAN, H.B. et al.** *Biochemistry,* 1991, vol. 30, 10832
- **CLACKSON, T. et al.** *Nature,* 1991, vol. 352, 624
- **MARKS, J. D. et al.** *J. Mol. Biol.,* 1991, vol. 222, 581
- **KANG, A.S. et al.** *Proc. Natl. Acad. Sci. USA,* 1991, vol. 88, 8363
- **SMITH, G. P.** *Current Opin. Biotechnol.,* 1991, vol. 2, 668
- Remington's Pharmaceutical Sciences. 1980
- **MARASCO et al.** *Proc. Natl. Acad. Sci. USA,* 1993, vol. 90, 7889-7893
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484
- **AUCHINCLOSS, H. JR. ; SACHS, D. H.** Fundamental Immunology. Raven Press, 1989, 889-992
- **TANABE, M. et al.** *Transplantation,* 1994, vol. 58, 23
- **TINUBU, S. A. et al.** *J. Immunol.,* 1994, 4330-4338

- Current Protocols in Immunology. John Wiley & Sons, Inc, 1994
- **GRABBE, S. ; SCHWARZ, T.** *Immun. Today,* 1998, vol. 19 (1), 37-44
- **SCHON, M. P. et al.** *Nat. Med.,* 1997, vol. 3, 183
- **NICKOLOFF, B. J. et al.** *Am. J. Path.,* 1995, vol. 146, 580
- **BOYMAN et al.** *J Exp Med.,* 2004, vol. 199 (5), 731-6
- **THOMAS ; CAPECCHI.** *Cell,* 1987, vol. 51, 503
- **LI et al.** *Cell,* 1992, vol. 69, 915
- Teratocarcirroma,s and Embryonic Stem Cells: A Practical Approach. IRL, 1987, 113-152
- **FIELDS ; SONG.** *Nature (London),* 1989, vol. 340, 245-246
- **CHIEN et al.** *Proc. Natl. Acad Sci. USA,* 1991, vol. 88, 9578-9582
- **CHEVRAY ; NATHANS.** *Proc. Natl, Acad. Sci. USA,* 1991, vol. 89, 5789-5793
- **ZOLA.** Monoclonal Antibodies: A Manual of Techniques. CRC Press, Inc, 1987, 147-158
- **D. TOY et al.** *J Immunol,* 01 July 2006, vol. 177, 36
- **J. WITOWSKI ; K. KSIAZEK ; A. JORRES.** *Cell Mol Life Sci,* March 2004, vol. 61, 567
- **S. M. SA et al.** *J Immunol,* 15 February 2007, vol. 178, 2229
- **J.-C. RENAULD.** *Nature Reviews Immunology,* 2003, vol. 3, 667
- **C. MAASER et al.** *Infect. Immun.,* 01 June 2004, vol. 72, 3315
- **S. LECART et al.** *Int. Immunol.,* 01 November 2002, vol. 14, 1351
- **T. GANZ.** *Science,* 15 October 1999, vol. 286, 420
- **H. L. CASH ; C. V. WHITHAM ; L. V. HOOPER.** *Protein Expression and Purification,* 2006, vol. 48, 151
- **S. A. KEILBAUGH et al.** *Gut,* 01 May 2005, vol. 54, 623
- **H. OGAWA et al.** *Inflammatory Bowel Diseases,* 2003, vol. 9, 162
- **H. OGAWA ; K. FUKUSHIMA ; I. SASAKI ; S. MATSUNO.** *Am JPhysiol Gastrointest Liver Physiol,* September 2000, vol. 279, G492
- **S. L. PULL ; J. M. DOHERTY ; J. C. MILLS ; J. I. GORDON ; T. S. STAPPENBECK.** *PNAS,* 04 January 2005, vol. 102, 99
- **V. MOUCADEL et al.** *Eur J Cell Biol,* February 2001, vol. 80, 156